# FASCICULE DE BREVET EUROPEEN

(11) **EP 2 456 754 B1**
(45) Date de publication et mention de la délivrance du brevet: **26.02.2014**
(21) Numéro de dépôt: 10747908.1
(22) Date de dépôt: 19.07.2010
(51) Int. Cl.: C07C 279/22, A61K 31/155, A61P 3/10, A61P 25/00, C07D 209/08, C07D 209/14, C07D 213/56, C07D 213/81, C07D 307/68, C07D 513/04, C07D 295/155

(54) **DERIVES D'ACYL-GUANIDINES MODULATEURS DE LA VOIE DE SIGNALISATION DES PROTEINES HEDGEHOG**
ACYL-GUANIDIN-DERIVATE ALS MODULATOREN DES HEDGEHOG-PROTEINSIGNALWEGES
ACYL GUANIDINE DERIVATIVES MODULATING THE HEDGEHOG PROTEIN SIGNALING PATHWAY

(30) Priorité: 24.07.2009 FR 0903654
(43) Date de publication de la demande: 30.05.2012
(73) Titulaire: Université de Strasbourg, 67081 Strasbourg Cedex (FR); Centre National de la Recherche Scientifique, 75016 Paris (FR)
(72) Inventeur: RUAT, Martial, F-91400 Orsay (FR); FAURE, Hélène, F-91190 Gif-sur-yvette (FR); TRAIFFORT, Elisabeth, F-75013 Paris (FR); ROUDAUT, Hermine, F-95670 Marly-la-ville (FR); MANN, André, F-67540 Ostwald (FR); SCHOENFELDER, Angèle, F-67450 Lampertheim (FR); TADDEI, Maurizio, I-53035 Monteriggioni (IT); MANETTI, Fabrizio, I-53019 Castelnuovo Berardenga (si) (IT); SOLINAS, Antonio, 07100 Sassari (SS) (IT)
(74) Mandataire: Mena, Sandra
(86) Numéro de dépôt international: PCT/FR2010/000516
(87) Numéro de publication internationale: WO 2011/010013

(56) Documents cités:
- EP-A2- 0 003 640
- US-A1- 2005 085 519
- P. PIERRON: "Mode de Préparation des Acylguanidines Aromatiques." COMPTE RENDUS DES SÉANCES DE L'ACADEMIE DES SCIENCES, vol. 151, 1911, pages 1364-1366, XP008119681

## Description

La présente invention est relative à des dérivés d'acyl-guanidines modulateurs de la voie de signalisation des protéines Hedgehog utilisés à titre de médicaments, notamment pour le traitement de pathologies impliquant un dysfonctionnement tissulaire lié à une dérégulation de la voie de signalisation des protéines Hedgehog, ainsi qu'aux compositions pharmaceutiques les contenant. La présente invention concerne également de nouveaux dérivés d'acyl-guanidines en tant que tels.

La molécule de signalisation Hedgehog (Hh) est une protéine auto-protéolytique sécrétée qui active la voie de signalisation des protéines Hedgehog, une voie de signalisation qui joue un rôle fondamental dans la morphogenèse de nombreux tissus, en particulier dans la formation de l'endoderme et de l'axe embryonnaire, le développement du cerveau et des follicules pileux, ainsi que dans la prolifération cellulaire, et serait impliquée dans le maintien et la réparation tissulaire chez l'adulte (Ingham et al., Genes Dev., 2001, 15, 3059-3087; Marti et al., Trends Neurosci., 2002, 25, 89-96 ; Weschler et al., Annu. Rev. Neurosci., 2001, 24, 385-428).

La protéine Hedgehog et la voie de transduction associée, initialement mises en évidence chez la drosophile, sont conservées chez les vertébrés et les invertébrés. Un seul homologue de Hh est présent chez la Drosophile, alors que trois homologues de Hh : Sonic (Shh), Indian (Ihh) et Desert (Dhh) sont présents chez les mammifères. Parmi ces trois homologues, Shh a été la plus étudiée du fait de son profil d'expression étendu durant le développement. Shh participe à la ventralisation du tube neural en spécifiant le phénotype précoce de plusieurs types neuronaux le long de la ligne médiane ventrale (motoneurones de la moelle épinière, neurones dopaminergiques ou cholinergiques), et en induisant la génération des précurseurs oligodendrocytaires à partir de la moelle épinière ventrale. Par ailleurs, Shh induit la survie des neurones gabaergiques et dopaminergiques, oriente le devenir des précurseurs sérotoninergiques et prévient la mort des neurones dopaminergiques provoquée par la toxine MPP. Il induit enfin la prolifération des précurseurs des cellules granulaires dans le cervelet post-natal précoce. Les autres membres de la famille Hedgehog participent, quant à eux, respectivement au développement du tissu osseux (Ihh), des testicules et des nerfs périphériques (Dhh). En outre, les résultats. obtenus avec Shh s'appliquent également à Dhh et Ihh.

Le rôle régulateur de la voie de signalisation des protéines Hedgehog durant le développement embryonnaire a été largement étudié : Hh a été associée aux processus de maintien et de réparation du tissu normal, à la régulation spatiotemporelle de la prolifération et de la différenciation, permettant ainsi aux tissus en développement d'atteindre leur taille correcte avec les types cellulaires appropriés et des degrés appropriés de vascularisation et d'innervation. Le rôle essentiel de la fonction de signalisation Hh est démontré par les conséquences dramatiques des défauts dans cette voie de signalisation chez le foetus humain, comme l'holoprosencéphalie observée avec les mutants de Shh.

Plus récemment la voie Shh a été identifiée dans le cerveau adulte, où la forme active amino-terminale de la molécule est exprimée dans de nombreuses régions du système nerveux mature à un niveau plus élevé que celui rencontré au cours de la période post-natale précoce (Traiffort et al., Eur. J. Neurosci., 1999, 11, 3199-3214 et 2001, 14, 839-850). Bien que les rôles de Shh chez l'adulte ne soient pas complètement élucidés, il est d'abord apparu, à l'image d'autres molécules neurotrophiques, comme un facteur capable de promouvoir la survie et le maintien du phénotype des cellules du système nerveux (Reilly et al., Mol. Cell. Neurosci., 2002, 19, 88-96; Charytoniuk et al., Eur. J. Neurosci., 2002, 16, 2351-2357). Dans des conditions pathologiques, telles qu'un modèle de la maladie de Parkinson ou un modèle de neuropathie périphérique, Shh est capable de préserver les projections axonales des neurones dopaminergiques dans le striatum ou d'améliorer le temps nécessaire à la récupération motrice consécutive à l'écrasement du nerf sciatique (Tsuboi et al., Exp. Neurol., 2002, 173, 95-104 ; Pepinski et al., J. Pharm. Sci., 2002, 91, 371-387).

Les protéines Hh sont synthétisées sous la forme de précurseurs immatures d'environ 45 kDa qui sont soumis à un clivage intramoléculaire catalysé par la région C-terminale du précurseur. Ce clivage produit un fragment C-terminal de 25 kDa sans fonction supplémentaire connue et en un fragment amino-terminal actif de 19 kDa (dénommée HhNp pour *N-terminal processed domain*) lié à son extrémité C-terminale à une molécule de cholestérol, suffisante pour toutes les activités de signalisation connues des protéines Hedgehog.

La voie de signalisation des protéines Hedgehog comprend trois composants principaux ; le ligand de Hh, un circuit de récepteur transmembranaire, composé du régulateur négatif *Patched* (*Ptc*) et de l'activateur *Smoothened* (*Smo*) et un complexe cytoplasmique qui régule les effecteurs transcriptionnels.

La réponse cellulaire au morphogène Hedgehog est contrôlée par les produits d'expression du gène *Patched* (*Ptc*), un gène suppresseur de tumeurs, et du proto-oncogène *Smoothened* (*Smo*) ; toutefois, le mécanisme exact de la régulation de la voie Hedgehog n'est pas complètement élucidé. Chez les mammifères, il existe deux gènes *Patched* codant respectivement pour *Pfc1* et *Ptc2,* des glycoprotéines à 12 domaines transmembranaires, homologues à des transporteurs bactériens. Le produit du gène *Smo* qui code pour une protéine de la famille des récepteurs couplés aux protéines G, ne possède aucun ligand endogène connu. En l'absence de protéines Hedgehog, *Ptc* bloquerait l'activité constitutive de *Smo.* La liaison de Hedgehog à *Ptc* lèverait cette inhibition et permettrait la transduction du signal par l'intermédiaire de *Smo.* Le mécanisme de régulation de l'activité de *Smo* par *Ptc,* chez les mammifères, pourrait faire intervenir une molécule transportée par *Ptc* et interagissant avec *Smo* (Taipale et al., Nature, 2002, 418, 892-896). L'activation des facteurs de transcription Gli est impliquée dans la cascade d'évènements résultant de l'activité de *Smo.* La protéine transmembranaire de type I, HIP (Hedgehog Intercating Protein), constitue un autre récepteur des molécules Hedgehog qu'il lie avec une affinité comparable à celle de *Ptc* ; HIP a été proposée comme un régulateur négatif de la voie (Ingham *et al.,* précité ; Ho et al., Curr. Opin. Neurobiol., 2002, 12, 57-63 ; Taipale et al., Nature, 2001, 411, 349-354). En outre, les produits du gène *dispatched* (*Disp*), notamment DispA seraient impliqués dans le relargage et l'accumulation dans le milieu extracellulaire des protéines Hedgehog sous forme soluble (Ma et al., Cell, 2002, 111, 63-75).

Des dysfonctionnements de la voie de signalisation Shh ont été associés à de nombreux cancers, en particulier à la suite de la caractérisation de *Ptc* comme gène suppresseur de tumeur. En effet, des mutations inactivatrices de *Ptc* sont associées au Syndrome de Gorlin ou naevomatose basocellulaire, une maladie autosomale dominante caractérisée par des malformations cranofaciales et cérébrales, mais surtout par une incidence élevée de diverses tumeurs, plus particulièrement des carcinomes basocellulaires au niveau cutané et des médulloblastomes, au niveau cérébral. Des souris hétérozygotes pour le gène *Ptc* développent des tumeurs du cervelet suggérant qu'une modification de la voie Shh est à l'origine de ces tumeurs (Goodrich et al., Science, 1997, 277, 1109-1113).

Des mutations des gènes *Ptc* ou *Smo* humains sont également observées dans des tumeurs primitives neuroectodermales du système nerveux central, principalement des médulloblastomes (30% des cas), mais aussi dans des formes sporadiques de carcinomes basocellulaires (respectivement 40% et 20% des cas pour *Ptc* et *Smo*). En outre, des mutations de Shh (H133Y) sont également associées à des carcinomes basocellulaires. Les mutations de *Smo* qui concernent principalement deux acides aminés situés dans le septième domaine hydrophobe du récepteur (W535L et S533N), induisent une activation constitutive de la voie qui échappe au contrôle négatif de *Ptc.* En revanche, celles de *Ptc* conduisent à une diminution de l'inhibition qu'il exerce sur *Smo* en l'absence de Shh. Dans les deux cas, une activation de la voie Shh en résulte et conduit à une puissante activité mitogénique démontrée dans des cultures de précurseurs de cellules granulaires de cervelet en développement, et à un blocage de l'étape terminale de différenciation de ces neuroblastes (Traiffort et al., Eur. J ; Neurosci., 1999, précité ; Charytoniuk et al., J. Physiol. Paris, 2002, 96, 9-16 ; Dahmane et al., Development, 1999, 126, 3089-3100; Wallace et al., Curr. Biol., 1999, 22, 103-114 ; Weshler-Reya et al., Neuron., 1999, 22, 103-114). De même, l'expression de *Smo* portant une de ces mutations dans des souris transgéniques conduit à la présence de carcinomes basocellulaires, ce qui démontre l'implication directe de *Smo* dans le développement de ces tumeurs (Xie et al., Nature, 1998, 391, 90-92).

En dehors des carcinomes basocellulaires et des médulloblastomes, d'autres types de tumeurs ont été associés à un défaut de la voie de signalisation Hedgehog ; la localisation de ces tumeurs est étroitement corrélée aux sites d'expression des composantes de la voie au cours du développement embryonnaire. A titre d'exemple non-limitatif on peut citer : des cancers du sein et des méningiomes associés à des mutations de *Ptc,* des glioblastomes associés à des mutations de Gli, des cancers gastro-intestinaux, notamment les cancers primaires de l'estomac, des cancers de la prostate, des fibromes et des dermoïdes ovariens, des rhabdomyosarcomes, des cancers du poumon à petites cellules, des carcinomes oraux à cellules squameuses. Récemment, Shh a été associée au psoriasis.

Du fait du rôle crucial de la voie de signalisation des protéines Hedgehog dans de nombreux processus physiologiques, et par conséquent de l'importance des pathologies liées à son dysfonctionnement, les composantes de cette voie, telles que les protéines *Smoothened, Patched* (*Patched 1* et *Patched 2*), les protéines *Dispatched* (*Dispatched 1* et *Dispatched 2*) ou bien encore la protéine HIP, représentent des cibles pour la mise au point de nouvelles molécules capables de moduler (activer ou inhiber) cette voie et donc de réguler positivement ou négativement le développement [prolifération, différenciation, migration, survie (apoptose)] et/ou l'activité de cellules différenciées et de cellules souches, *in vitro* et/ou *in vivo* chez l'embryon ou chez l'adulte.

De telles molécules sont utiles dans le traitement des tumeurs associées à une hyperactivation de la voie Hedgehog : les tumeurs du tissu nerveux (médulloblastomes, tumeurs primitives neuroectodermiques, glioblastomes, méningiomes et oligodendrogliomes), les tumeurs cutanées (carcinomes basocellulaires, trichoépithéliomes), les tumeurs des tissus musculaires et osseux (rhabdomyosarcomes, ostéosarcomes) et les tumeurs d'autres tissus (rein, vessie).

De telles molécules sont également utiles dans le traitement des pathologies de type neuro-dégénératif nécessitant un blocage de la voie Hedgehog (maladie de Parkinson, Chorée de Huntington, maladie d'Alzheimer, sclérose en plaques, maladie du motoneurone), et des maladies dans lesquelles le blocage de la voie de signalisation Hedgehog pourrait être bénéfique comme le diabète.

De telles molécules sont également utiles dans le traitement médical ou chirurgical (chirurgie plastique ou réparatrice, greffe de tissus ou d'organes) de nombreuses pathologies aiguës, subaiguës ou chroniques, génétiques ou acquises - impliquant un dysfonctionnement tissulaire lié à une dérégulation de la voie Hedgehog -, pour induire la formation, la régénération, la réparation et/ou l'augmentation de l'activité de tissus tels que de manière non-limitative : le tissu nerveux [système nerveux central (cerveau) et périphérique (neurones sensoriels, moteurs, sympathiques)], l'os, le cartilage, les testicules, le foie, la rate, l'intestin, le pancréas, les reins, les muscles lisses et squelettiques, le coeur, les poumons, la peau et le système pileux, les muqueuses, les cellules sanguines et les cellules du système immunitaire. A titre d'exemple non-limitatif de ces pathologies, on peut citer notamment les neuropathies et les maladies neuromusculaires associées, le diabète, l'alopécie, les brûlures, les ulcérations (peau et muqueuses) et les troubles de la spermatogenèse.

Différentes molécules, capables de moduler l'activité de la voie Hedgehog, ont été identifiées :
- les protéines Hedgehog et des polypeptides dérivés (fragments, variants...), notamment des agonistes et des antagonistes des protéines Hedgehog (Demande Internationale PCT WO 01/98344 au nom de BIOGEN) ; du fait de leur taille, ces protéines et les polypeptides dérivés ne peuvent pas passer la barrière hématoencéphalique et ne peuvent donc pas être administrés par voie systémique, notamment pour le traitement des tumeurs cérébrales liées à une hyperactivation de la voie de signalisation des protéines Hedgehog. En outre, de telles molécules sont peu stables, et difficiles à produire et à purifier ;
- des molécules organiques hétérocycliques (Demande Internationale PCT WO 01/74344 au nom de CURIS et Chen et al., PNAS, 2002, 99, 14071-14076) ;
- des molécules hétérocycliques azotées (Demandes Internationales PCT WO 01/19800, WO 01/26644 et WO 02/30421 au nom de CURIS et Kamenetsky et al., J. Biol., 2002, 1, 1-19) ; et
- des stéroïdes végétaux dérivés de *Veratrum* spp (jervine, cyclopamine et cycloposine) et de *Solanum* spp. (solanidine), substitués en position 16, 17 ou 18 par une amine ou un dérivé d'amine, et du cholestérol : Brevet américain US 6,432,970 et Demandes Internationales PCT WO 99/52534 et WO 01/27135 au nom de JOHNS HOPKINS UNIVERSITY SCHOOL OF MEDICINE; Brevet américain US 6,291,516 ; Demande Internationale PCT WO 00/41545 au nom de ONTOGENY INC. ; Demande Internationale PCT WO 02/30462 au nom de CURIS ; Talpale et al., Nature, 2000, 406, 1005-1009 ; Berman et al., Science, 2002, 297, 1559-1561). Toutefois, la cyclopamine est un agent tératogène à l'origine d'holoprosencéphalie et de cyclopie pour l'embryon chez les mammifères, et il a également été démontré que des concentrations en cyclopamine supérieures à 10 µM s'avéraient être cytotoxiques pour les cellules (Borzillo et al., Curr. Top Med. Chem., 2005, 5(2), 147-57). En ce qui concerne les autres composés dérivés de stéroïdes végétaux, leur absence de toxicité chez les mammifères n'a pas encore été démontrée ;
- la mifepristone (17β-hydroxy 11β-(4-diméthylamino phényl) 17α-(prop-1-ynyl)estra-4,9-dien 3-one), également dénommée RU-486 ou RU-38486 (Brevet français FR 2 850 022 au nom du CNRS) pour laquelle une activité inhibitrice de l'activité de la voie de signalisation des protéines Hedgehog a été démontrée ;
- des dérivés urées ou thiourées antagonistes de la voie de signalisation des protéines Hedgehog ont également été décrits dans la Demande US 2005/0085519 A1.

Les molécules SANT74 et le SANT75 ayant une structure analogue à celle du SAG, composé activateur synthétique de type chlorobenzothiophène (CAS N° : 364590-63-6) répondant à la formule suivante : 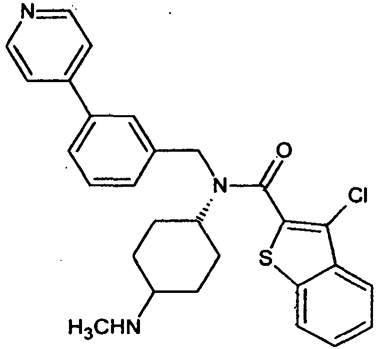 sont également connus pour être des inhibiteurs stables permettant de contrôler efficacement la conformation de l'activateur *Smo* (Yang et al., The Journal of Biological Chemistry, publié le 14 avril 2009).

D'autres composés inhibiteurs de la voie de signalisation Hedgehog ont également été décrits récemment : des inhibiteurs à base de pyridyle (Demande Internationale PCT WO 2006/028958 au nom de GENENTECH INC. et CURIS INC.) et de bisamide (Demande Internationale PCT WO 2007/059157 au nom de GENENTECH INC. et CURIS INC.).

D'autres molécules agissant notamment sur les facteurs de transcription de la famille Gli ont également été décrites (Mahindroo et al., J. Med. Chem. 2009, 52, 3829-3845).

Il ressort de ce qui précède qu'il n'existe actuellement aucune molécule permettant une modulation de l'activité de la voie de signalisation des protéines Hedgehog dont l'absence de toxicité ait été vérifiée par des essais cliniques chez l'Homme.

En conséquence, les inventeurs se sont donné pour but de pourvoir à de nouveaux composés modulateurs (stimulateurs ou inhibiteurs) de la voie de signalisation des protéines Hedgehog qui répondent mieux aux besoins de la pratique, notamment en ce qu'ils sont simples à synthétiser et potentiellement utilisables en thérapie humaine.

Cet objectif est atteint par les composés de formule **(I)** qui sont décrits ci-après et qui constituent le premier objet de l'invention dans la mesure où ces molécules présentent l'avantage de comporter une fonction principale de type acyl-guanidine qui s'obtient à partir de matières premières aisément disponibles. La fonction guanidine, en tant que base, est salifiable, ce qui a l'avantage de produire des composés ayant une bonne solubilité en milieu aqueux. L'ensemble des composés de formule **(I)** est obtenu de manière très commode en utilisant des réactions chimiques simples bien connues de l'homme de l'art.

En conséquence, la présente invention a pour objet les composés de formule **(I)** suivante : 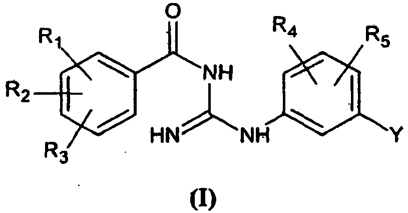 dans laquelle:
- R₁, R₂ et R₃, identiques ou différents et indépendamment les uns des autres, représentent un atome d'hydrogène ou d'halogène, un radical hydroxyle, un groupe alkyle, perfluoroalkyle, alcoxy éventuellement substitué, alkylthio, nitrile, ou un hétérocycle fusionné obtenu à partir de deux des R₁, R₂ et R₃ fusionnés avec deux atomes de carbone adjacents du cycle phényle auxquels ils sont liés, le cycle phényle avec ledit hétérocycle fusionné représentant de préférence un benzodioxole, un oxindole, un benzoxazolone, ou une quinoléine ;
- Y représente un groupe hétéroaryle mono- ou polycyclique, - NH-(C=O)-R₆, -(C=O)-NH-R₆ ou -NH-(C=O)-NH-R₆, dans lequel R₆ représente un groupe aryle mono- ou polycyclique non substitué ; un groupe aryle comportant un ou plusieurs substituants choisis parmi un atome d'halogène, un radical alkyle, alcoxy ou alcoxyaryle, mono- ou dialkylamino, un groupe aryle ou hétéroaryle, un hétérocycle ; un groupe hétéroaryle mono- ou polycyclique ; un radical alkyle linéaire ou ramifié ; un groupe hydrocarboné mono- ou polycyclique saturé ou insaturé ;
- R₄ et R₅, identiques ou différents et indépendamment l'un de l'autre, représentent un atome d'hydrogène ou d'halogène, un groupe alcoxy, alkylthio, alkyle, perfluoroalkyle, nitrile ou nitro,
à titre de médicaments.

Ainsi que cela est démontré dans les exemples qui illustrent la présente invention, les composés de formule **(I)** conformes à l'invention présente une activité inhibitrice de la voie de signalisation des protéines Hedgehog, et sont donc utiles pour le traitement des pathologies nécessitant une modulation de la voie Hedgehog comme le cancer, les maladies neurodégénératives et le diabète.

Les composés de formule **(I)** conformes à la présente invention peuvent être divisés en sous-unités **A, B, C** (ou **C'** ou **C"**) et **D** (ou **D'** ou **D"**) et représentés par les formules **(I-a), (I-b), (I-c)** et **(I-d)** suivantes : 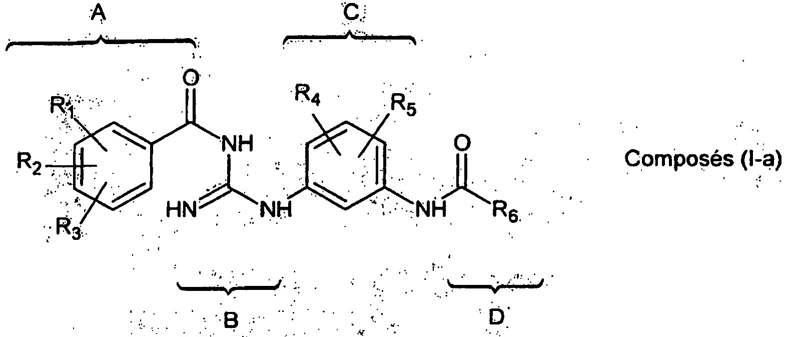 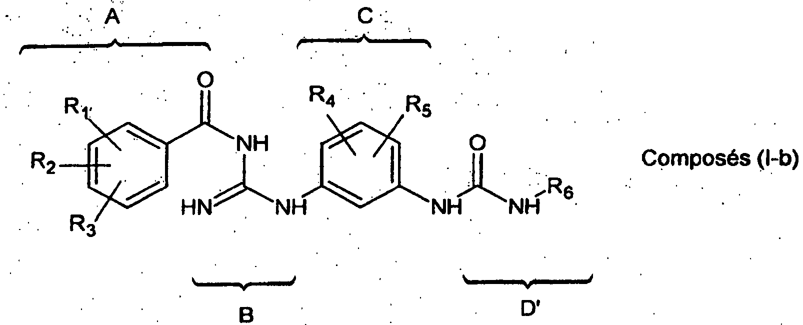 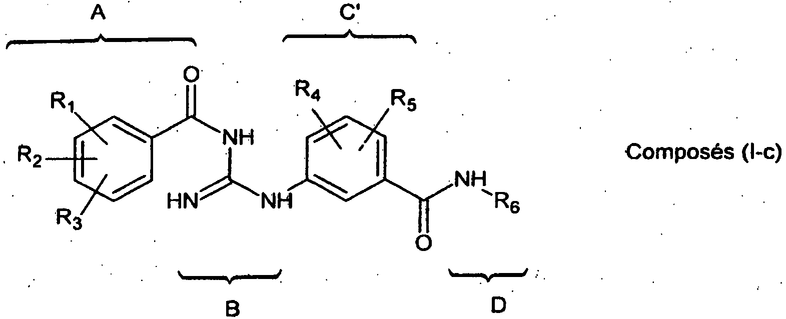 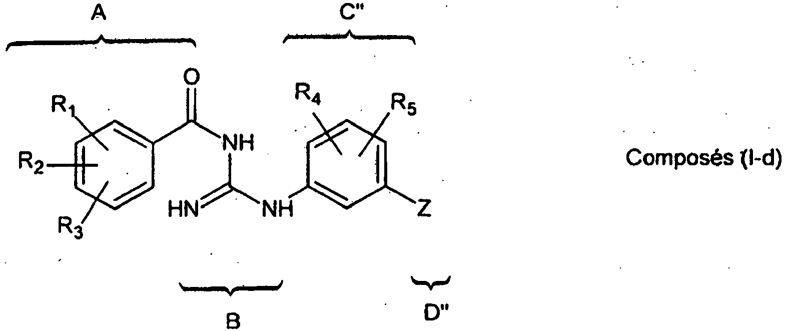 dans lesquelles R₁, R₂, R₃, R₄, R₅ et R₆ ont les mêmes significations que celles indiquées ci-dessus.

Dans ces formules, la sous-unité A correspond à une partie acyl-aryle, la sous-unité B à une guanidine, la sous-unité C à un groupe 1,3-diaminoaryle, la sous-unité C' à un groupe 1,3-aminophénoyle, la sous-unité C" à un groupe aminophényle, la sous-unité D à un résidu alkyloyle, aroyle ou hétéroaroyle ; la sous-unité D' à un résidu alkylamino, arylamino ou hétéroarylamino ; la sous-unité D" (groupement -Z) à un résidu hétéroaryle mono- ou polycyclique.

Au sens de la présente invention, on entend par :
- Alkyle : un groupe aliphatique hydrocarboné saturé, linéaire ou ramifié, ayant de 1 à 5 atomes de carbone, de préférence de 1 à 2 atomes de carbone. Le terme "ramifié" signifie qu'au moins un groupe alkyle inférieur tel qu'un méthyle ou un éthyle est porté par une chaîne alkyle linéaire. Le terme alkyle "inférieur" désigne
   un alkyle ayant 1 ou 2 atomes de carbone ; le terme "alkyle supérieur" désigne un groupe alkyle linéaire ou ramifié ayant de 3 à 5 atomes de carbone. A titre de groupe alkyle, on peut mentionner par exemple les groupes méthyle, éthyle, n-propyle, i-propyle, n-butyle, t-butyle et n-pentyle.
- Atome d'halogène : désigne un atome de brome, de chlore, d'iode ou de fluore ; les désignations brome, chlore et fluore étant préférées ;
- Perfluoroalkyle : désigne un groupe alkyle tel que .défini ci-dessus dans lequel tous les atomes d'hydrogène ont été remplacés par des atomes de fluore. Parmi les groupes perfluoroalkyle, les groupes trifluorométhyle et perfluoroéthyle sont préférés ;
- Alcoxy : désigne un groupe O-alkyle dans lequel le groupe alkyle peut prendre la même signification que celle indiquée ci-dessus. A titre d'exemple de groupes alcoxy, on peut notamment citer les groupes méthoxy, éthoxy, n-propoxy, iso-propoxy, n-butoxy et pentoxy ;
- Alkylthio : désigne un groupe alkyl-S dans lequel le groupe alkyle peut prendre la même signification que celle indiquée ci-dessus. A titre d'exemples de groupe alkylthio, on peut notamment citer les groupes méthylthio, éthylthio, iso-propylthio, butylthio et pentylthio ;
- Groupe aryle : désigne tout groupe fonctionnel ou substituant dérivé d'au moins un cycle aromatique ; un cycle aromatique correspond à tout groupe mono- ou polycyclique plan comportant un système π délocalisé dans lequel chaque atome du cycle comporte une orbitale p, lesdites orbitales p se recouvrant les unes les autres ; parmi de tels groupes aryle, on peut mentionner les groupes phényle, benzylcyclobutène, pentalène, naphthalène, benzylphényle, et anthracène ;
- Groupe hétéroaryle : désigne tout groupe fonctionnel ou substituant dérivé d'au moins un cycle aromatique tel que défini ci-dessus et contenant au moins un hétéroatome choisi parmi P, S, O et N ; parmi les groupes hétéroaryle, on peut mentionner les groupes furane, pyridine, pyrrole, thiophène, imidazole, pyrazole, oxazole, isoxazole, thiazole, pyridine, pyrazine, pyrimidine, pyridazine, benzofurane, isobenzofurane, indole, isoindole, benzothiophène, benzo[*c*]thiophène, benzimidazole, indazole, benzoxazole, benzisoxazole, benzothiazole, quinoléine, isoquinoléine, quinoxaline, quinazoline, cinnoline, purine, et acridine ;
- Groupe hydrocarboné mono- ou polycyclique saturé ou insaturé : désigne tout groupe fonctionnel ou substituant dérivé d'un cycle non aromatique comportant au moins 3 atomes de carbone comportant éventuellement, de façon optionnelle, un ou plusieurs hétéroatomes choisis parmi P, S, O et N. Parmi de tels groupes, on peut notamment citer le cyclopropyle, le cyclobutyle, le cyclopentyle, le cyclohexyle ; le groupe cyclohexyle étant préféré.

Selon une forme de réalisation préférée de l'invention, les composés de formule **(I)** sont choisis parmi ceux dans lesquels :
- R₁, R₂ et R₃, identiques ou différents, représentent un atome d'hydrogène, un radical méthyloxy ou éthyloxy, ou un dioxolane fusionné avec deux atomes de carbone adjacents du cycle phényle auquel ils sont liés ;
- R₄ et R₅, identiques ou différents, représentent un atome d'hydrogène, de chlore, de brome, de fluore, ou un radical méthyle ou méthoxy, et de préférence un atome d'hydrogène, de chlore, de fluore ou un radical méthyle ; et
- Y représente un groupe hétéroaryle mono- ou polycyclique, -NH-(C=O)-R₆, -(C=O)-NH-R₆ ou -NH-(C=O)-NH-R₆ dans lequel R₆ représente un groupe phényle, éventuellement substitué par un atome d'halogène, un radical alkyle, diaminoalkyle ou alcoxy, un groupe cycloalkyle, ou un groupe aryle ; un groupe cycloalkyle ; un groupe pyridinyle ; un groupe naphtyle ; un groupe furyle ; un groupe thiophényle ; un radical isopropyle.

Selon un mode de réalisation préféré de la présente invention, le groupe Y est choisi parmi l'indole ou l'imidazole fusionné à un groupe thiazol, lorsque ledit groupe Y représente un groupe hétéroaryle mono- ou polycyclique.

Selon un autre mode de réalisation préféré de la présente invention, R₆ représente un groupe phényle, éventuellement substitué par un atome de chlore, un radical méthoxy, une morpholine, un groupe phényle ou phénoxy ; un groupe cyclohexyle ; un groupe pyridinyle ; un groupe naphtyle ; un groupe furyle ; lorsque ledit groupe Y représente un groupe -NH-(C=O)-R₆, -(C=O)-NH-R₆ ou -NH-(C=O)-NH-R₆.

Un autre objet de la présente invention concerne de nouveaux dérivés d'acyl-guanidines en tant que tels, répondant à la formule **(I)** suivante : 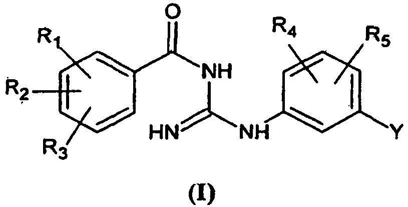 dans laquelle :
- R₁, R₂ et R₃, identiques ou différents et indépendamment les uns des autres, représentent un atome d'hydrogène ou d'halogène, un radical hydroxyle, un groupe alkyle, perfluoroalkyle, alcoxy éventuellement substitué, alkylthio, nitrile, ou un hétérocycle fusionné obtenu à partir de deux des R₁, R₂ et R₃ fusionnés avec deux atomes de carbone adjacents du cycle phényle auxquels ils sont liés, le cycle phényle avec ledit hétérocycle fusionné représentant de préférence un benzodioxole, un oxindole, un benzoxazolone, ou une quinoléine ;
- Y représente un groupe hétéroaryle mono- ou polycyclique choisi parmi l'indole ou l'imidazole fusionné à un groupe thiazol, ou Y représente un groupe -NH-(C=O)-R₆, -(C=O)-NH-R₆ ou -NH-(C=O)-NH-R₆, dans lequel R₆ représente un groupe aryle mono- ou polycyclique non substitué ; un groupe aryle comportant un ou plusieurs substituants choisis parmi un atome d'halogène, un radical alkyle, alcoxy ou alcoxyaryle, mono- ou dialkylamino, un groupe aryle ou hétéroaryle, un hétérocycle ; un groupe hétéroaryle mono- ou polycyclique ; un radical alkyle linéaire ou ramifié ; un groupe hydrocarboné mono- ou polycyclique saturé ou insaturé ;
- R₄ et R₅, identiques ou différents et indépendamment l'un de l'autre, représentent un atome d'hydrogène ou d'halogène, un groupe alcoxy, alkylthio, alkyle, perfluoroalkyle, nitrile ou nitro.

Selon une forme de réalisation préférée de l'invention, les composés de formule **(I)** en tant que tels sont choisis parmi ceux dans lesquels :
- R₁, R₂ et R₃, identiques ou différents, représentent un atome d'hydrogène, un radical méthyloxy ou éthyloxy, ou un dioxolane fusionné avec deux atomes de carbone adjacents du cycle phényle auquel ils sont liés ;
- R₄ et R₅, identiques ou différents, représentent un atome d'hydrogène, de chlore, de brome, de fluore, ou un radical méthyle ou méthoxy, et de préférence un atome d'hydrogène, de chlore, de fluore ou un radical méthyle ; et
- Y représente un groupe hétéroaryle mono- ou polycyclique choisi parmi l'indole ou l'imidazole fusionné à un groupe thiazol, ou Y représente un groupe -NH-(C=O)-R₆, -(C=O)-NH-R₆ ou -NH-(C=O)-NH-R₆ dans lequel R₆ représente un groupe phényle, éventuellement substitué par un atome d'halogène, un radical alkyle, diaminoalkyle ou alcoxy, un groupe cycloalkyle, ou un groupe aryle ; un groupe cycloalkyle ; un groupe pyridinyle ; un groupe naphtyle ; un groupe furyle ; un groupe thiophényle ; un radical isopropyle.

Selon un mode de réalisation encore plus préféré de la présente invention, dans les composés de formule **(I)** en tant que tels, R₆ représente un groupe phényle, éventuellement substitué par un atome de chlore, un radical méthoxy, une morpholine, un groupe phényle ou phénoxy ; un groupe cyclohexyle ; un groupe pyridinyle ; un groupe naphtyle ; un groupe furyle ; lorsque ledit groupe Y représente un groupe -NH-(C=O)-R₆, =(C=O)-NH-R₆ ou -NH-(C=O)-NH-R₆.

A titre de composés de formule **(I)**, on peut en particulier citer, à titre non limitatif :
- le 3,4,5-triméthoxy-N-(N-(3-(4-méthoxybenzamido)phényl)carbamimidoyl)benzamide : 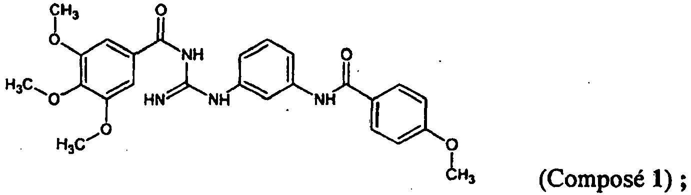
- le N-(N-(3-(2-chlorobenzamido)phényl)carbamimidoyi)-3,4,5-triméthoxybenzamide : 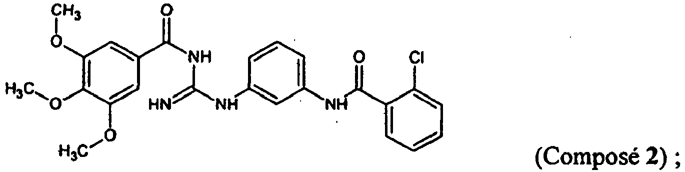
- le 3,4,5-triméthoxy-N-(N-(3-(3-méthoxybenzamido)phényl)carbamimidoyl)benzamide : 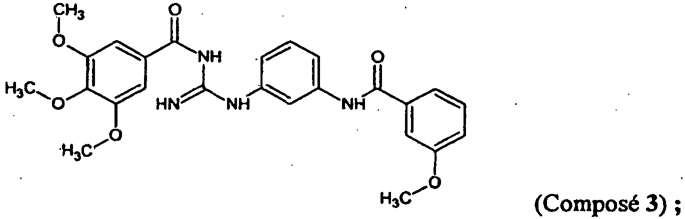
- le N-(3-(3-(3,4,5-triméthoxybenzoyl)guanidino)phényl)biphényl-4-carboxamide : 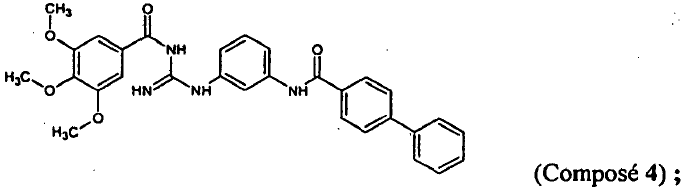
- le N-(N-(3-(cyclohexanecarboxamido)phényl)carbamimidoyl)-3,4,5-triméthoxybenzamide : 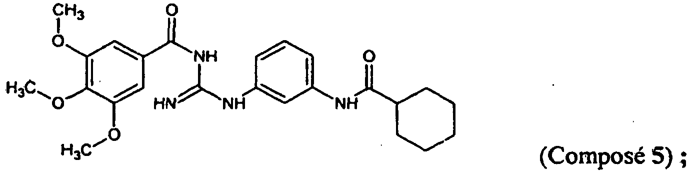
- le N-(3-(3-(3,4,5-triméthoxybenzoyl)guanidino)phényl)-2-naphthamide : 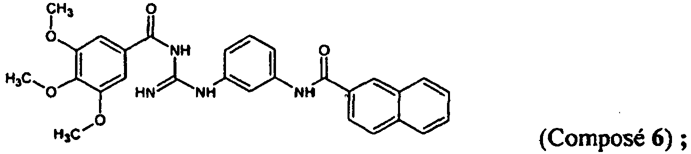
- le N-(3-(3-(3,4,5-triméthoxybenzoyl)guanidino)phényl)isonicotinamide : 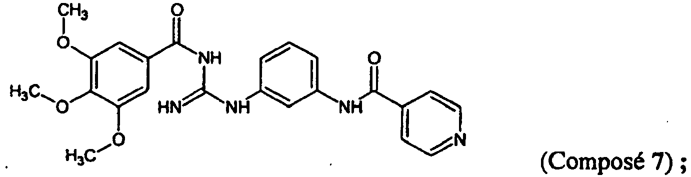
- le N-(3-(3-(3,4,5-triméthoxybenzoyl)guanidino)phényl)furan-2-carboxamide : 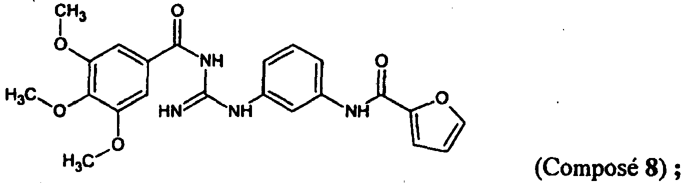
- le N-(N-(3-benzamidophényl)carbamimidoyl)-3,5-diméthoxybenzamide : 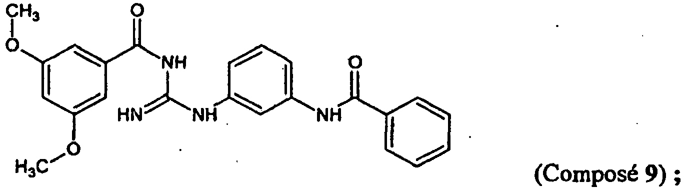
- le N-(N-(3-benzamidophényl)carbamimidoyl)-4-éthoxy-3,5-diméthoxybenzamide : 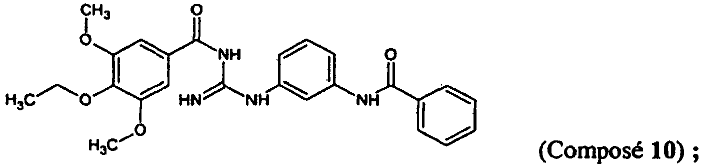
- le N-(N-(3-benzamidophényl)carbamimidoyl)-3,4-diéthoxy-5-méthoxybenzamide : 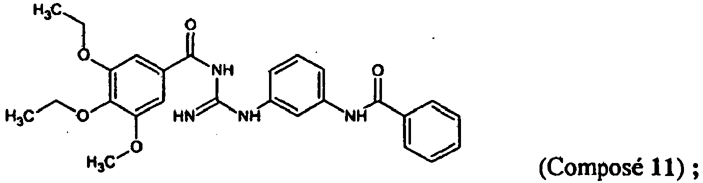
- le N-(N-(3-benzamidophényl)carbamimidoyl)-7-méthoxybenzo[*d*][1,3]dioxole-5-carboxamide : 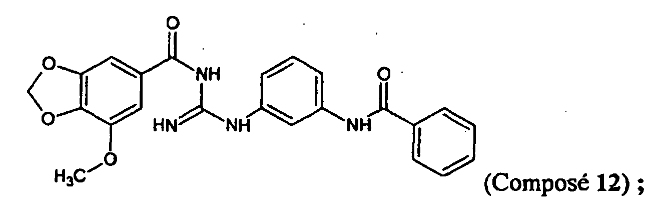
- le N-(N-(3-benzamidophényl)carbamimidoyl)-2,4-diméthoxybenzamide : 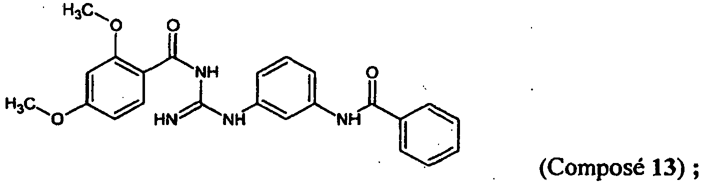
- le N-(N-(3-benzamido-4-fluorophényl)carbamimidoyl)-3,4,5-triméthoxybenzamide : 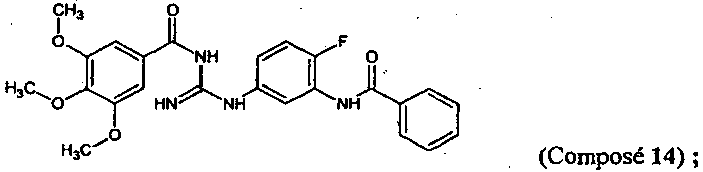
- le N-(N-(3-benzamidophényl)carbamimidoyl)-3,4,5-triméthoxybenzamide : 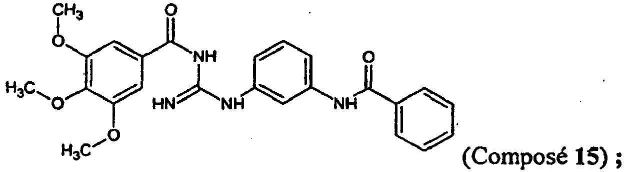
- le N-(N-(3-benzamido-4-chlorophényl)carbamimidoyl)-3,4,5-triméthoxybenzamide : 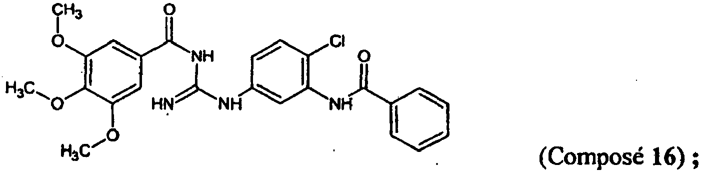
- le N-(2-chloro-5-(3-(3,4,5-triméthoxybenzoyl)guanidino)phényl)biphényl-4-carboxamide : 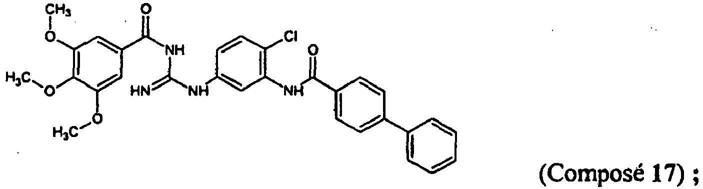
- le 3,4,5-triméthoxy-N-(N-(3-(4-morpholinobenzamido)phényl)carbamimidoyl)benzamide: 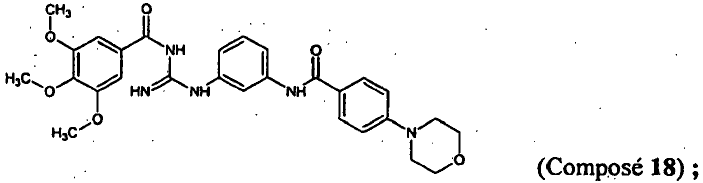
- le N-(N-(3-(1*H*-indol-2-yl)phényl)carbamimidoyl)-3,4,5-triméthoxybenzamide : 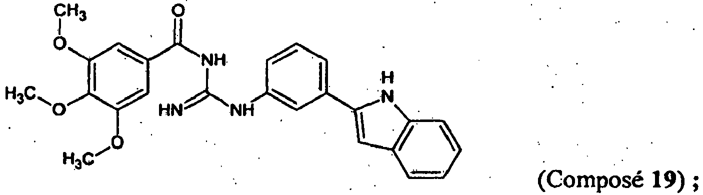
- le N-(N-(4-chloro-3-(4-méthoxyphénylcarbamoyl)phényl)carbamimidoyl)-3,4,5-triméthoxybenzamide : 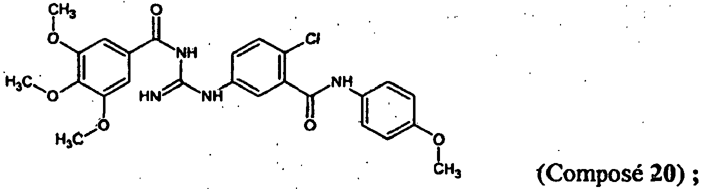
- le N-(N-(4-chloro-3-(phénylcarbamoyl)phényl)carbamimidoyl)-3,4,5-triméthoxybenzamide : 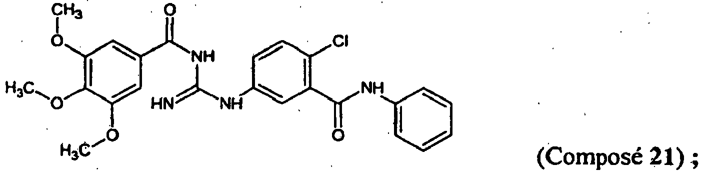
- le 3,4,5-triméthoxy-N-(N-(4-méthyl-3-(phénylcarbamoyl)phényl)carbamimidoyl)benzamide : 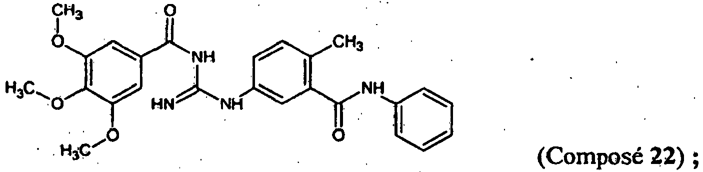
- le 4'-fluoro-N-(3-(3-(3,4,5-triméthoxybenzoyl)guanidino)phényl)biphényl-4-carboxamide : 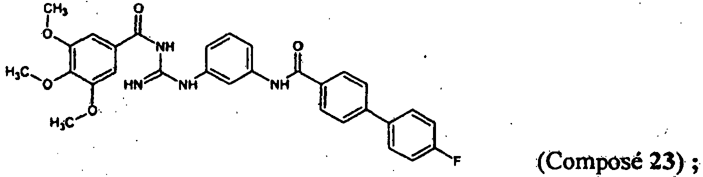
- le N-(2-méthyl-5-(3-(3,4,5-triméthoxybenzoyl)guanidino)phényl)biphényl-4-carboxamide : 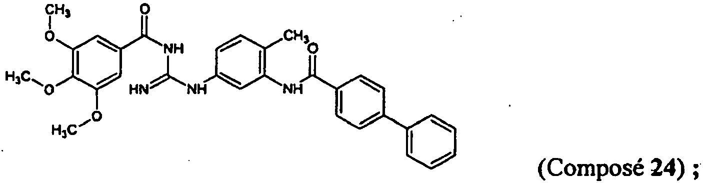
- le N-(2-méthyl-5-(3-(3,4,5-triméthoxybenzoyl)guanidino)phényl)biphényl-3-carboxamide : 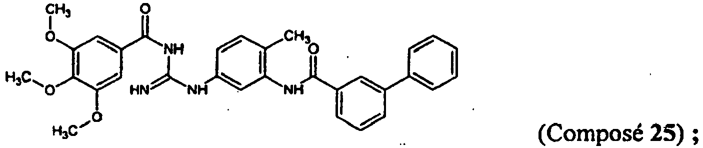
- le N-(2-chloro-5-(3-(3,4,5-triméthoxybenzoyl)guanidino)phényl)biphényl-3-carboxamide : 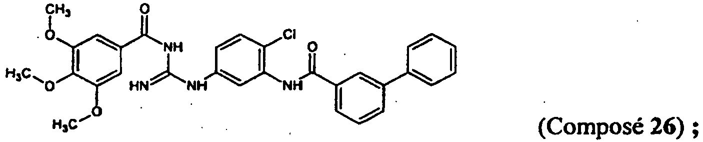
- le N-N-(3-benzamido-4-méthylphényl)carbamimidoyl)-3,4,5-triméthoxybenzamide : 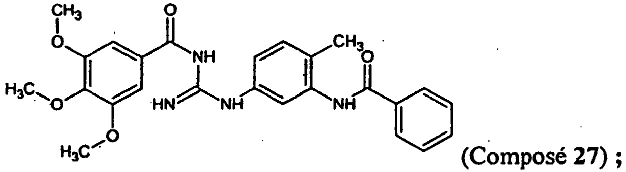
- le N-N-(3-(imidazo[2,1-*b*]thiazol-6-yl)phényl)carbamimidoyl)-3,4,5-triméthoxybenzamide : 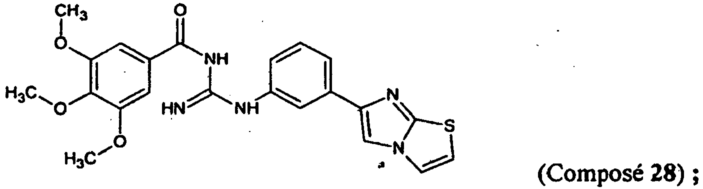
- le N-(N-(3-(1*H*-indol-1-yl)phényl)carbamimidoyl)-3,4,5-triméthoxybenzamide : 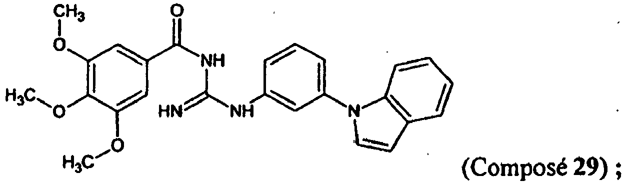
- le 4'-fluoro-N-(2-méthyl-5-(3-(3,4,5-triméthoxybenzoyl)guanidino)phényl)biphényl-4-carboxamide : 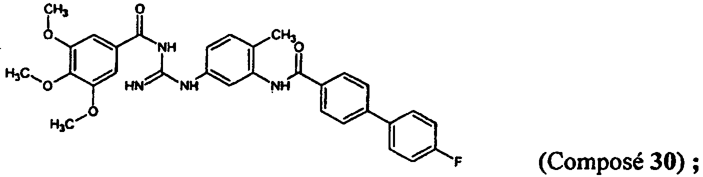
- le 3,4,5-triméthoxy-N-(N-(4-méthyl-3-(4-(pyridin-4-yl)benzamido)phényl)carbamimidoyl)-benzamide : 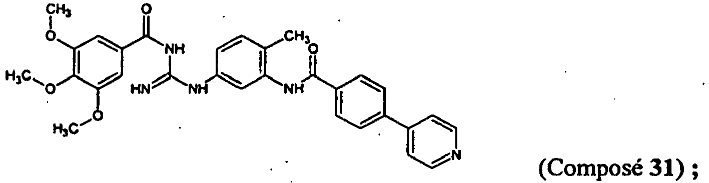
- le N-(N-(4-chloro-3-(4-phénoxybenzamido)phényl)carbamimidoyl)-3,4,5-triméthoxybenzamide : 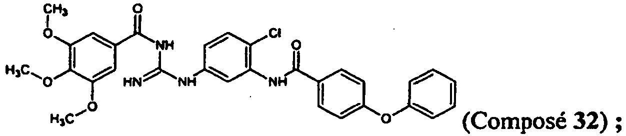
- le N-(3-(3-benzoylguanidino)phényl)biphényl-4-carboxamide : 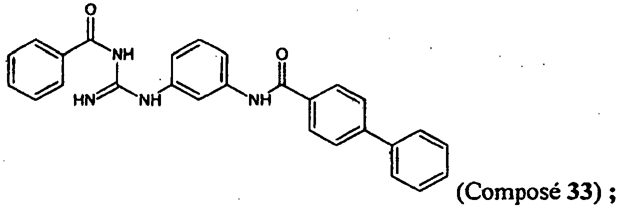
- le N-(2-méthyl-3-(3-(3,4,5-triméthoxybenzoyl)guanidino)phényl)biphényl-4-carboxamide : 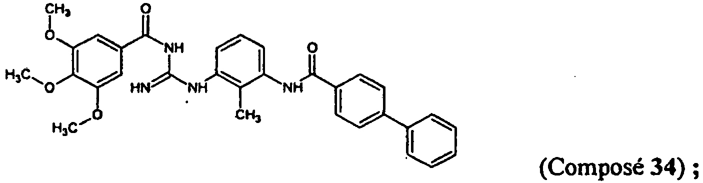
- le N-(4-méthyl-3-(3-(3,4,5-triméthoxybenzoyl)guanidino)phényl)biphényl-4-carboxamide : 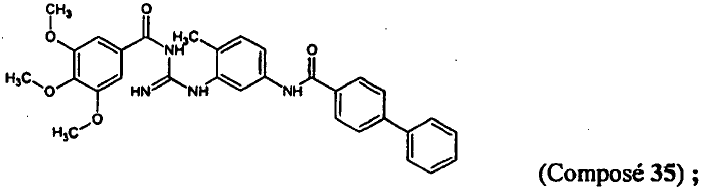
- le N-(N-(3-benzamidophényl)carbamimidoyl)benzamide : 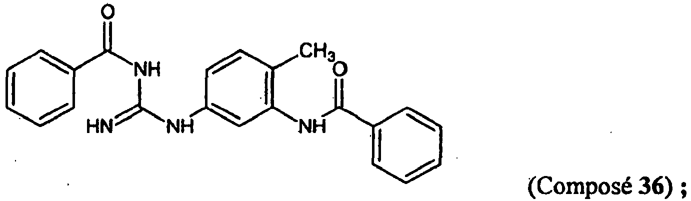
- le N-(N-(3-(3-biphényl-4-ylureido)phényl)carbamimidoyl)-3,4,5-triméthoxybenzamide : 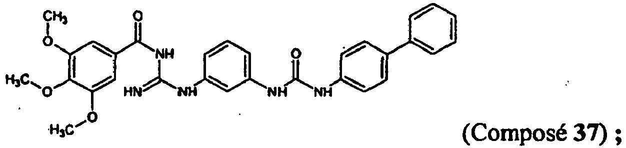
- le N-(N-(4-chloro-3-(3-phénylureido)phényl)carbamimidoyl)-3,4,5-triméthoxybenzamide :

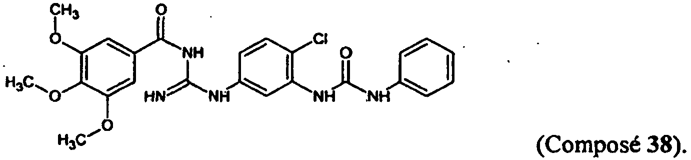

Parmi ces composés, les composés suivants sont particulièrement préférés dans la mesure où ils présentent une activité d'inhibition de la voie de signalisation des protéines Hedgehog qui est supérieure ou égale à 80% d'inhibition, ladite inhibition étant mesurée après activation de la voie de signalisation des protéines Hedgehog avec le SAG, selon la méthode décrite par Chen et al. (Proc. Natl. Acad. Sci. USA, 2002, 99, 14071) :
- le 3,4,5-triméthoxy-N-(N-(3-(4-méthoxybenzamido)phényl)carbamimidoyl)benzamide (Composé **1**) ;
- le N-(N-(3-(2-chlorobenzamido)phényl)carbamimidoyl)-3,4,5-triméthoxybenzamide (Composé **2**) ;
- le 3,4,5-triméthoxy-N-(N-(3-(3-méthoxybenzamido)phényl)carbamimidoyl)benzamide (Composé **3**) ;
- le N-(3-(3-(3,4,5-triméthoxybenzoyl)guanidino)phényl)-2-naphthamide (Composé **6**) ;
- le N-(3-(3-(3,4,5-triméthoxybenzoyl)guanidino)phényl)furan-2-carboxamide (Composé **8**) ;
- le N-(N-(3-benzamidophényl)carbamimidoyl)-3,5-diméthoxybenzamide (Composé **9**) ;
- le N-(N-(3-benzamidophényl)carbamimidoyl)-7-méthoxybenzo[*d*][1,3]dioxole-5-carboxamide (Composé **12**) ;
- le N-(N-(3-benzamidophényl)carbamimidoyl)-2,4-diméthoxybenzamide (Composé **13**) ;
- le N-(N-(3-benzamidophényl)carbamimidoyl)-3,4,5-triméthoxybenzamide (Composé **15**) ;
- le N-(2-chloro-5-(3-(3,4,5-triméthoxybenzoyl)guanidino)phényl)biphényl-4-carboxamide (Composé **17**) ;
- le N-(N-(3-(1*H*-indol-2-yl)phényl)carbamimidoyl)-3,4,5-triméthoxybenzamide (Composé **19**) ;
- le N-(N-(4-chloro-3-(phénylcarbamoyl)phényl)carbamimidoyl)-3,4,5-triméthoxybenzamide (Composé **21**) ;
- le 4'-fluoro-N-(3-(3-(3,4,5-triméthoxybenzoyl)guanidino)phényl)biphényl-4-carboxamide (Composé **23**) ;
- le N-(2-méthyl-5-(3-(3,4,5-triméthoxybenzoyl)guanidino)biphényl-4-carboxamide (Composé **24**) ;
- le N-(N-(3-(1*H*-indol-1-yl)phényl)carbamimidoyl)-3,4,5-triméthoxybenzamide (Composé **29**) ;
- le 4'-fluoro-N-(2-méthyl-5-(3-(3,4,5-triméthoxybenzoyl)guanidino)phényl)biphényl-4-carboxamide (Composé **30**) ;
- le N-(N-(4-chloro-3-(4-phénoxybenzamido)phényl)carbamimidoyl)-3,4,5-triméthoxybenzamide (Composé **32**) ;
- le N-(N-(4-chloro-3-(3-phénylureido)phényl)carbamimidoyl)-3,4,5-triméthoxybenzamide (Composé **38**).

Les composés de formule **(I)** conformes à l'invention peuvent être facilement préparés, généralement en trois ou quatre étapes, selon des procédés de synthèse analogues aux procédés classiques connus de l'homme du métier.

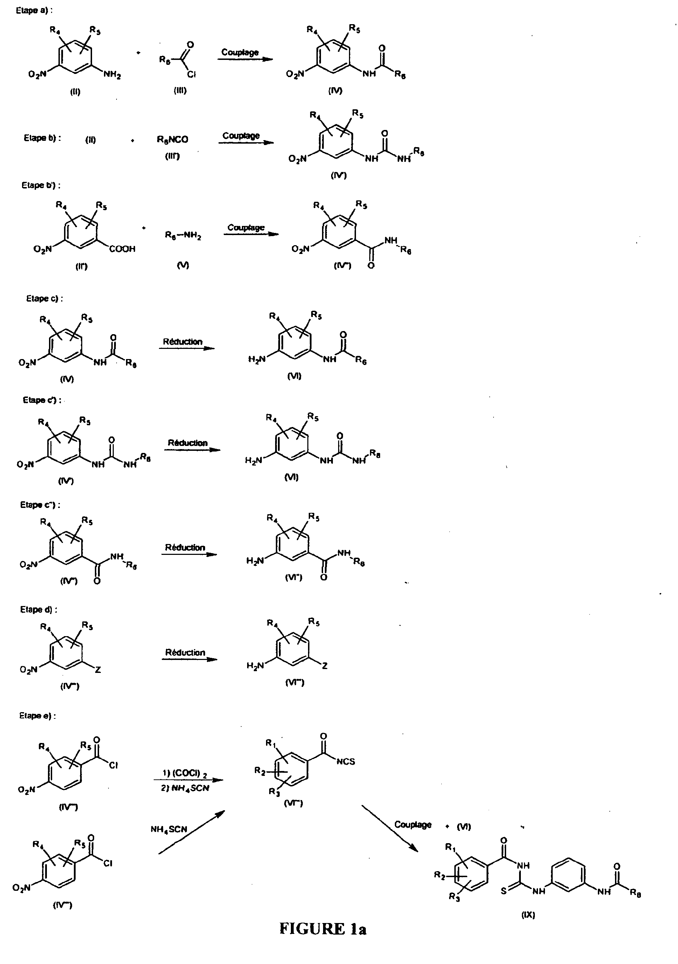

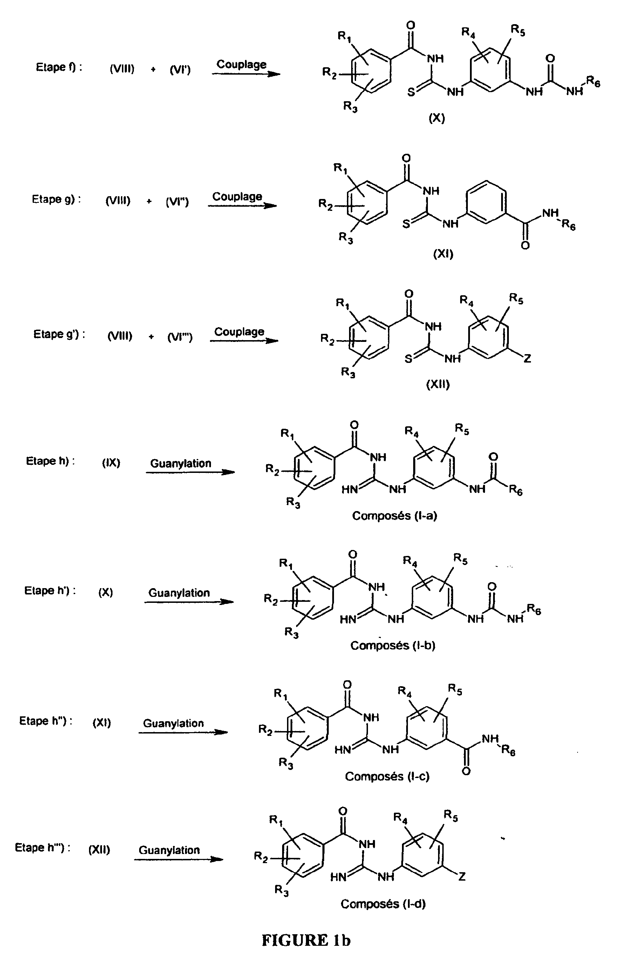

Les schémas de synthèse générale des composés de formule **(I)** conforme à l'invention, dans leurs quatre variantes **(I-a)**, **(I-b)**, **(I-c)** et **(I-d)**, peuvent être représentés selon les figures 1a et 1b annexées.

Conformément au schéma de synthèse représenté sur les figures 1a et 1b annexées, dans une **étape a)**, utile pour obtenir des composés de formule **(I-a)**, on condense une 3-nitroaniline commerciale de formule **(II)** dans laquelle les radicaux R₄ et R₅ ont les mêmes significations que celles indiquées ci-dessus pour les composés de formule **(I)** avec un chlorure d'acide de formule **(III)** dans laquelle R₆ a la même signification que celle indiquée ci-dessus pour les composés de formule **(I)**, par exemple selon la méthode de Schotten-Baumann, pour obtenir le composé amide de formule **(IV)** correspondant. **L'étape b)** permet un couplage entre une 3-nitroaniline commerciale de formule **(II)** possédant des résidus R₄ et R₅ comme indiqué dans la formule **(I),** et un isocyanate commercial **(III')**, pour obtenir une nitro-urée de formule **(IV')**. L'étape **b')** consiste à condenser un acide 3-nitrobenzoïque commercial de formule **(II')** avec une amine de formule **(V),** dans lesquelles R₄, R₅ et R₆ ont les mêmes significations que celles indiquées ci-dessus pour les composés de formule **(I),** pour obtenir le composé nitro-amide de formule **(IV")** correspondant.

D'autres méthodes conventionnelles bien connues de l'homme du métier pour former une liaison amide peuvent également être utilisées pour réaliser les **étapes a), b) et b')** de condensation.

Dans les **étapes c), c'), c")** et **d),** le groupe nitro des composés de formule **(IV)**, **(IV')** et **(IV")** est réduit en amine pour obtenir respectivement les anilines répondant aux formules **(VI), (VI')** et **(VII")**. Le composé 3-nitroaromatique de formule **(IV"')**, dans laquelle R₄, R₅ et Z ont les mêmes significations que celles indiquées ci-dessus pour les composés de formule **(I)**, est obtenu selon des procédés classiques connus de l'homme du métier (Yang et al., Angew. Chem. Int., Ed. 2008, 47, 1473 ; Burkholder et al., Tetrahedron Lett., 2001, 42, 3077 : Zhang et al., J. Org. Chem., 2005, 70, 5164 ; Aggarwal et al., Synth. Comm., 2006, 36, 875 ; Demande Internationale PCT WO 2006/050506 au nom de CURIS). Le composé 3-nitroaromatique **(IV"')** est ensuite soumis à une étape de réduction pour obtenir l'aniline correspondante **(VI"')**, cette étape de réduction pouvant être réalisée en milieu réducteur, par exemple par action d'un agent réducteur tel que le dichlorure de plomb ou le dichlorure d'étain, ou bien encore par hydrogénation, en utilisant par exemple une activation par les micro-ondes. D'autres méthodes d'hydrogénation peuvent être également utilisées en fonction de la nature des substituants R₄ et R₅ éventuellement présents sur le cycle phényle. A cet égard, lorsque R₄ et/ou R₅ représentent un atome d'halogène tel que le chlore, le brome, ou l'iode, l'étape de réduction est de préférence réalisée par action du dichlorure d'étain. Dans tous les autres cas, on préfère réaliser une hydrogénation catalytique en présence de Pd/C ou de Nickel de Raney.

Au cours des étapes **e)** et **f)**, on prépare un acylisothiocyanate de formule **(VIII)** dans laquelle les radicaux R₁ à R₃ ont la même signification que celle indiquée ci-dessus pour les composés de formule **(I)**, à partir d'un acide benzoïque de formule **(VII)** ou d'un chlorure d'acide benzoïque de formule **(VII')**, par exemple dans un milieu solvant au reflux (acétonitrile ou acétone) en présence, par exemple, de phosgène et de thiocyanate d'ammonium. Le composé de formule **(VIII)**, un benzoylisothiocyanate, ainsi obtenu est ensuite couplé à un composé de formule **(VI)** ou de formule **(VI')** pour conduire aux composés acyl-thiourées de formule **(IX)** et **(X)** correspondants. Dans les étapes **g)** et **g')**, le même benzoylisocyanate **(VIII)** est condensé au reflux dans un solvant avec les anilines **(VI")** et **(VI"')**, pour conduire aux acyl-thiourées de formules **(XI)** et **(XII)**. Généralement, les composés de formules **(IX)**, **(X)**, **(XI)** et **(XII)**, sont obtenus sous la forme de solides qui sont ensuite purifiés de façon classique par recristallisation dans un alcool (Rasmussen et al., Synthesis, 1988, 456-459).

La transformation des acyl-thiourées en acyl-guanidines peut être réalisée selon des méthodes bien connues de l'homme du métier, telle que celle décrite par Shirada et al., Tetrahedron Lett., 2006, 47, 1945. Les acyl-thiourées **(IX), (X), (XI)** et **(XII)** sont ainsi transformées en acyl-guanidines **(I-a)**, **(I-b)**, **(I-c)** et (I-d)**.** La réalisation des **étapes h**), **h'**), **h"**) et **h"'**) dans l'acétonitrile, en présence de chlorhydrate de 1-éthyl-3(3-diméthylamino)propyl carbodiimide (EDCI) et d'un excès d'hexaméthyldisilazane (HMDS), permet d'obtenir de très bons rendements en acyl-guanidines **(I-a)**, **(I-b)**, **(I-c)** et **(I-d)**. Ces composés sont obtenus sous forme de solides, et peuvent être transformés sous forme de sels solubles dans l'eau, l'un des avantages des composés de formule **(I)** étant leur solubilité dans l'eau. Parmi les sels dérivés on peut citer les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques, tels que l'acide méthane sulfonique et l'acide éthane sulfonique, arylsulfoniques, tels que le benzène sulfonique et le paratoluène sulfonique, ou arylcarboxyliques, les sels formés avec l'acide chlorhydrique étant les sels préférés. La transformation des composés d'acyl-guanidines **(I-a)**, **(I-b)**, **(I-c)** et **(I-d)** sous forme de sels est réalisée dans des conditions stoechiométriques, par simple mélange avec l'acide choisi.

Les composés de formule **(I)** conformes à l'invention présentent la propriété de moduler négativement (effet inhibiteur) ou positivement (effet activateur) le voie de signalisation des protéines Hedgehog et peuvent donc être utilisés, à titre de principe actif, pour la préparation d'une composition pharmaceutique destinée aux traitements des pathologies associées à une hyperactivation ou à un déficit de la voie de signalisation des protéines Hedgehog.

Par conséquent, la présente invention a également pour objet les composés de formule **(I)** à titre de médicaments, pour les traitements suivants :
i) à titre de médicament destiné au traitement des tumeurs associées à une hyperactivation de la voie de signalisation des protéines Hedgehog ; de telles tumeurs sont notamment, de manière non limitative, les tumeurs du tissu nerveux (médulloblastomes, tumeurs primitives neuroectodermiques, glioblastomes, méningiomes et oligodendrogliomes), les tumeurs cutanées (carcinomes basocellulaires, trichoépithéliomes, mélanomes), les tumeurs des tissus musculaires et osseux (rhabdomyosarcomes, ostéosarcomes) et les tumeurs d'autres tissus (rein, vessie, prostate, poumon, estomac, pancréas, sein, foie),
ii) à titre de médicament destiné au traitement de maladies liées au développement cérébral (holoprosencéphalie), les composés de formule **(I)** pouvant être utilisés *in vitro* pour contrôler et moduler le renouvellement des cellules souches humaines ou animales, au traitement d'accidents vasculaires cérébraux et cardiovasculaires, ainsi qu'aux maladies de l'oligodentrocyte et des cellules de Schwann (qui assurent l'isolation électrique des axones),
iii) à titre de médicament destiné au traitement des pathologies nécessitant une modulation de la voie Hedgehog, notamment des pathologies de type neuro-dégénératif comme la maladie de Parkinson, la Chorée de Huntington, la maladie d'Alzheimer, la sclérose en plaques et la maladie du motoneurone, ou bien d'autres pathologies dans lesquelles la modulation de la voie de signalisation Hedgehog pourrait être bénéfique, comme le diabète.

La posologie utile variera en fonction de l'affection à traiter, de la voie et du rythme d'administration, ainsi que de la nature et du poids de l'espèce à traiter (humaine ou animale) ; elle peut varier par exemple de 1 mg à 2 g par jour chez l'adulte par voie orale.

En plus des diverses applications à titre de médicaments mentionnées ci-dessus, les composés de formule **(I)** peuvent également être utilisés comme marqueurs pour détecter la présence de protéines, ou comme outils de diagnostics pour cribler des protéines, dans les tissus ou les lignées cellulaires. Plus particulièrement, les composés de formule **(I)** peuvent être utilisés comme marqueurs ou outils de diagnostic pour détecter ou cribler la protéine *Smoothened,* ou des protéines apparentées telles que *Patched* (*Patched 1* et *Patched 2*), les protéines *Dispatched (Dispatched 1* et *Dispatched 2)*, ou bien encore la protéine HIP. Un autre objet de la présente invention est une composition pharmaceutique, caractérisée par le fait qu'elle comprend, à titre de principe actif, au moins un composé de formule **(I)** tel que défini précédemment, et au moins un excipient pharmaceutiquement acceptable.

Au sein des compositions pharmaceutiques conformes à l'invention, le ou les composés de formule **(I)** sont de préférence utilisés en une quantité permettant d'administrer des doses unitaires comprises entre 1 mg et 2 g environ.

L'homme du métier choisira un ou plusieurs excipients pharmaceutiquement acceptables en fonction de la voie d'administration de la composition pharmaceutique. Bien entendu, l'homme de l'art veillera, à cette occasion, à ce que le ou les excipients utilisés soient compatibles avec les propriétés intrinsèques attachées à la composition conforme à la présente invention.

En outre, la forme du médicament ou de la composition pharmaceutique (par exemple, une solution, une suspension, une émulsion, des comprimés, des gélules, des suppositoires, etc...) dépendra de la voie d'administration choisie.

Ainsi, au sens de la présente invention, le médicament ou la composition pharmaceutique peut être administré par n'importe quelle voie appropriée, par exemple par la voie orale, anale, locale, systémique, intraveineuse, intramusculaire ou mucosale, ou bien en utilisant un patch, ou encore sous forme encapsulée dans, ou immobilisée sur, des liposomes, des microparticules, des microcapsules, et analogues.

On peut notamment citer, à titre d'exemples non limitatifs d'excipients appropriés pour une administration par voie orale, le talc, le lactose, l'amidon et ses dérivés, la cellulose et ses dérivés, les polyéthylèneglycols, les polymères d'acide acrylique, la gélatine, le stéarate de magnésium, des matières grasses animales, végétales ou synthétiques, les dérivés de la paraffine, les glycols, les stabilisants, les conservateurs, les anti-oxydants, les agents mouillants, les anti-agglomérants, les dispersants, les émulsionnants, les agents modifiants du goût, les agents de pénétrations, de solubilisation, etc...

Les techniques de formulation et d'administration des médicaments et compositions pharmaceutiques sont bien connues dans la technique ici considérée, l'homme du métier pouvant notamment se référer à l'ouvrage Remington's Pharmaceutical Sciences, (21st edition).

Outre les dispositions qui précèdent, l'invention comprend encore d'autres dispositions qui ressortiront de la description qui va suivre, qui se réfère à des exemples de synthèse des composés de formule **(I)**, à un exemple de mise en oeuvre des composés de formule **(I)** selon la présente invention, ainsi qu'aux dessins annexés dans lesquels :
- les figures 1a et 1b illustrent les voies de synthèses générales des composés de formule **(I)**,
- la figure 2 représente une série de photos prises au microscope à fluorescence montrant la compétition des composés de formule **(I)** avec la bodipycyclopamine,
- la figure 3 représente les courbes d'inhibition des Composés **23** et **24**, Cur61414 et GDC-0449 sur la bodycyclopamine,
- la figure 4 représente une coupe frontale de la zone sous ventriculaire (ZSV) de cerveaux de souris adultes hybridées avec une ribosonde antisens pour le gène *Ptc.* Les coupes sont issues de souris ayant reçu la protéine ShhN recombinante seule (A), en présence de Cur61414 (B), ou en présence de Composé **24** (C). La flèche montre un marquage spécifique de l'ARNm de *Ptc.* La barre d'échelle correspond à 0,1 mm. 3 à 5 coupes de cerveaux sont hybridées pour chaque condition.

### EXEMPLE 1 : SYNTHÈSES DE DIFFÉRENTS COMPOSÉS DE FORMULE (I)

Dans ces exemples, les réactions ont été conduites sous atmosphère de gaz inerte (azote) en utilisant des techniques de Schlenk (standard). Les solvants ont été séchés selon des méthodes standards et distillés sous azote avant utilisation. Tous les réactifs ont été obtenus dans le commerce et utilisés tels quels sans purification préalable.

Les spectrométries de masse (ESI+) ont été enregistrées sur un spectromètre LC/MSD vendu sous la référence Agilent^{®} 1100. Les spectres de résonance magnétique nucléaire (RMN) ont été enregistrés sur un appareil Bruker^{®} AC200 à 200 MHz (¹H) ou sur un appareil Bruker^{®} AC400 à 400 MHz (¹H) ou à 100 MHz (¹³C).

### A) Synthèse du Composé 19

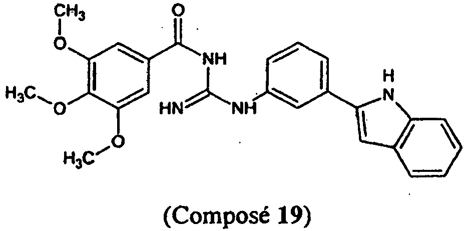

### 1) Préparation de l'aniline de formule (VI"') (figure 1a)

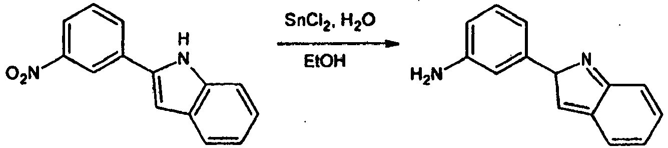

Le 2-(3-nitro-phényl)-1*H*-indole est préparé selon la méthode décrite dans Yang et al., Angew. Chem., Int., Ed. 2008, 47, 1473. Le dérivé nitro-indole (1,19 g, 5 mmol) est dissous dans 32 mL d'éthanol, et le milieu est chauffé à 80°C. Du SnCl₂, H₂O (3,8 g, 5 éq., 16 mmol) est ajouté en une fois. Le milieu est ensuite mis à chauffer pendant encore 2 heures, puis versé sur un mélange eau/glace et alcanisé avec du Na₂CO₃. Le mélange est ensuite extrait avec de l'acétate d'éthyle. La phase organique est lavée avec une solution saturée de NaCl, puis séchée et concentrée sous vide pour obtenir des cristaux. Ce résidu est recristallisé dans l'éthanol pour obtenir un solide (980 mg, rendement = 86%).
Pf = 134°C ; **[ES/MS]** m/z 210 [M + 1]⁺

### 2) Préparation de l'acyl-thiourée de formule (XII) (figure 1b)

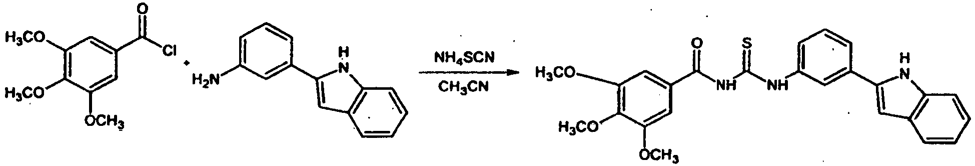

Du thiocyanate d'ammonium (123 mg, 1,2 éq., 1,63 mmol) et du chlorure de 3,4,5-triméthoxybenzoyle (345 mg, 1,1 éq., 1,5 mmol) sont dissous dans 5 mL d'acétone. Le mélange est chauffé pendant 1 heure au reflux. L'aniline obtenue lors de l'étape précédente (315 mg, 1 éq., 1,45 mmol) est ajouté, et le reflux est maintenu pendant 1 heure supplémentaire. Le mélange est ensuite versé sur de l'eau, puis filtré et recristallisé dans l'acétonitrile pour obtenir l'acyl-thiourée de formule **(XII)** (320 mg, 52%).
Pf = 136°C, **[ES/MS]** m/z 462 [M + 1]⁺

### 3) Obtention du Composé 19 (figure 1 b)

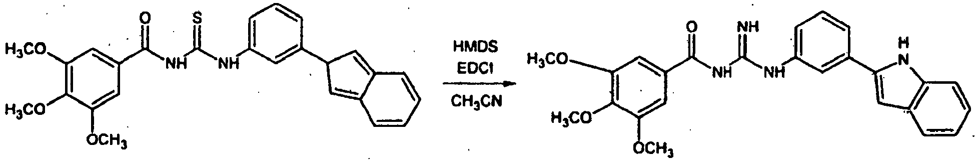

L'acyl-thiourée de formule **(XII)** obtenue précédemment (69 mg, 0,15 5 mmol) et l'hexaméthyldizilazane (0,32 mL, 10 éq., 1,5 mmol) sont dissous dans 1,5 mL d'acétonitrile, puis refroidis à 0°C. Du chlorhydrate de 1-éthyl-3(3-diméthylamino)propyl carbodiimide (58 mg, 2 éq., 0,3 mmol) est ensuite ajouté. Le mélange est agité à température ambiante pendant 5 heures. Le milieu réactionnel est versé dans de l'eau, extrait à l'acétate d'éthyle, lavé avec une solution saturée de NaCl, puis séchée et concentrée sous vide. Le résidu obtenu est purifié par chromatographie sur SiO₂ (éluant : acétate d'éthyle/heptane: 1/1). On récupère un solide blanc correspondant au Composé **19** (52 mg, 78%).
Pf = 149°C ; **[ES/MS]** m/z 446 [M + 1]⁺

### 4) Préparation du chlorhydrate du Composé 19

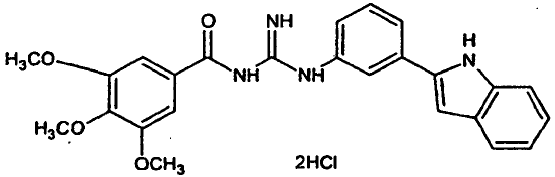

Le Composé **19** (55 mg, 0,123 mmol) est dissout dans 5 mL d'iso-propanol. Une solution d'HCl est ensuite ajoutée dans l'Et₂O (0,14 mL, 0,27 mmol), puis le milieu est agité pendant 2 heures. Le milieu réactionnel est évaporé, puis le sel est cristallisé dans l'éther. On obtient alors le chlorhydrate du Composé **19** (43 mg, 68%) ayant un Pf de 225°C.

### B) Synthèse du Composé 20

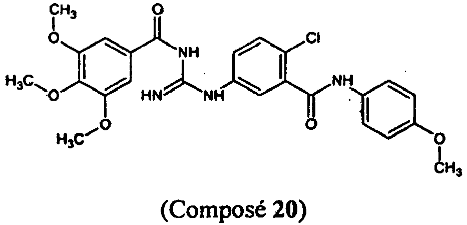

### 1) Préparation du chlorure d'acide de formule (III) (figure 1a)

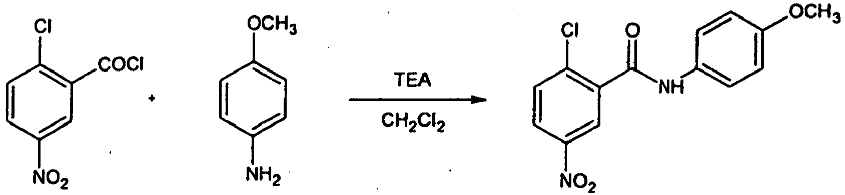

L'acide 2-chloro-5-nitro benzoïque (6,04 g, 0,03 mol) est dissout dans 200 mL de dichlorométhane. On ajoute ensuite du chlorure d'oxalyle (3,88 mL, 1,5 éq., 0,045 mol), puis goutte à goutte du diméthylformamide (DMF), et on agite le milieu réactionnel pendant 3 heures. On évapore ensuite le solvant pour récupérer le chlorure d'acide de formule **(III).**

### 2) Préparation de l'amide de formule (IV') (figure 1a)

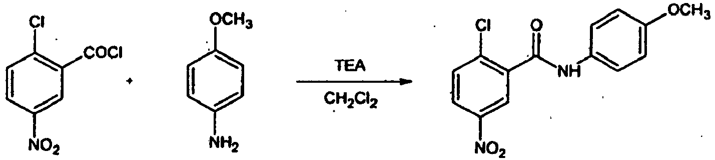

La 4-méthoxy-aniline (1,02 g, 1 éq., 8,3 mmol) et la triéthylamine (1,4 mL, 1,2 éq., 10 mmol) sont dissous dans 12 mL de dichlorométhane. On ajoute ensuite le chlorure d'acide de formule **(III)** préparé précédemment (2, g, 1 éq., 0,9 mmol), dissout dans 15 mL de CH₂Cl₂, et on agite le milieu réactionnel pendant 12 heures. On ajoute ensuite de l'eau et de l'acétate d'éthyle, avant de récupérer la phase organique, de la sécher et de la concentrer sous vide. Ce résidu est cristallisé dans l'isopropanol pour obtenir un solide (2,12 g, 83%).
Pf = 143°C ; **[ES/MS]** m/z 307 [M + 1]⁺

### 3) Préparation de l'aniline de formule (VII") (figure 1a)

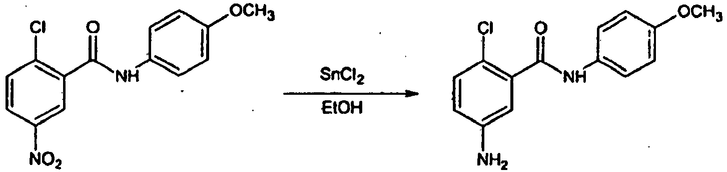

L'amide de formule **(IV')** obtenue précédemment (1,53 g, 5 mmol) est dissous dans 30 mL d'éthanol absolu, puis chauffé à 80°C. Du SnCl₂, H₂O (3,3 g) est ajouté, et le chauffage est maintenu pendant encore 2 heures. Le solvant est évaporé, puis le résidu est lavé avec de l'eau, puis alcanisé avec du Na₂CO₃ saturé dans l'eau. Le mélange est ensuite extrait avec de l'acétate d'éthyle. La phase organique est lavée avec une solution saturée de NaCl, puis séchée et concentrée pour obtenir un résidu qui est recristallisé dans l'isopropanol. On récupère un solide (845 mg, 61%).
Pf= 132-133°C ; **[ES/MS]** m/z 277 [M + 1]⁺

### 4) Préparation de l'acyl-thiourée de formule (XI) (figure 1b)

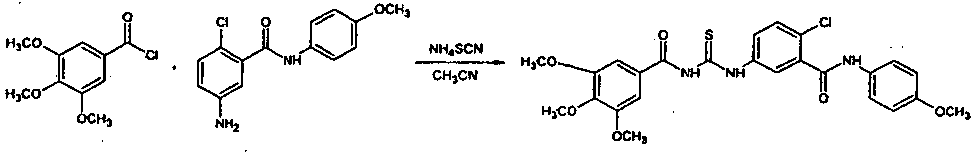

Du thiocyanate d'ammonium (82 mg, 1,2 éq., 1,09 mmol) et du chlorure d'acide de formule **(VII)** (ici du 3,4,5-triméthoxybenzoyle) (230 mg, 1,1 éq., 1 mmol) sont dissous dans 5 mL d'acétone. Le mélange est chauffé pendant 1 heure au reflux. L'aniline obtenue lors de l'étape précédente (250 mg, 1 éq., 0,9 mmol) est ajouté, et le reflux est maintenu pendant 1 heure supplémentaire. Le mélange est ensuite versé sur de l'eau glacé, puis filtré et recristallisé dans l'acétonitrile pour obtenir l'acyl-thiourée de formule **(XI)** (180 mg, 38%).
Pf= 165°C, **[ES/MS]** m/z 556 [M + 1]⁺

### 5) Obtention du Composé 20

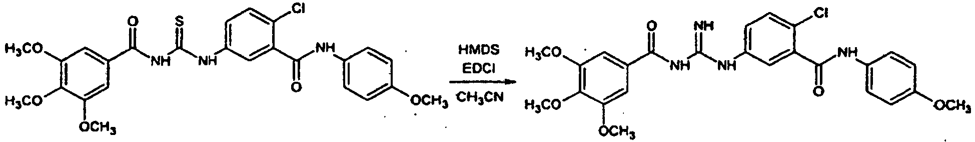

L'acyl-thiourée de formule **(XI)** obtenue précédemment (66 mg, 0,125 mmol) et l'hexaméthyldizilazane (0,26 mL, 1,25 mmol) sont dissous dans 1,5 mL d'acétonitrile, puis refroidis à 0°C dans un bain de glace. Du chlorhydrate de 1-éthyl-3(3-diméthylamino)propylcarbodiimide (48 mg, 2 éq., 0,25 mmol) est ensuite ajouté. Le mélange est agité à température ambiante pendant 5 heures. Le milieu réactionnel est versé sur de la glace, et la phase aqueuse est extraite plusieurs fois avec de l'acétate d'éthyle. La phase organique est lavée avec une solution saturée de NaCl, puis séchée et concentrée sous vide. Le résidu obtenu est cristallisé dans un mélange isopropanol/heptane, puis purifié par chromatographie sur SiO₂ (éluant : acétate d'éthyle/heptane : 1/1, puis acétate d'éthyle pur), pour obtenir 51 mg du Composé **20.**
Pf= 105°C; **[ES/MS]** m/z 513 [M + 1]⁺

### C) Synthèse du Composé 24

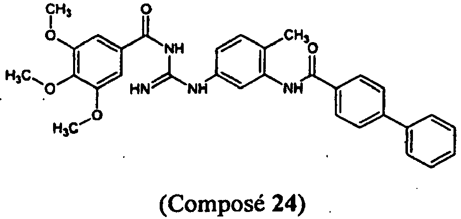

### 1) Préparation de l'amide de formule (IV) (figure 1a)

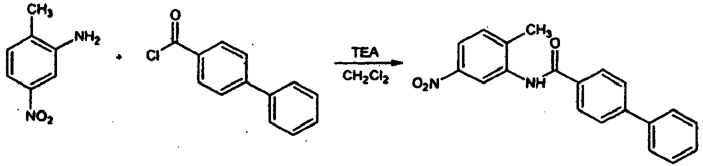

La 2-méthyl-5-nitro-aniline (1,26 g, 1 éq., 8,3 mmol) est dissout dans 40 mL de dichlorométhane. On ajoute ensuite de la triéthylamine (1,4 mL, 1,2 éq., 10 mmol), puis goutte à goutte du chlorure de 4-phényl-benzoyle (2,1 g, 10 mmol, préparé à partir de l'acide de chlorure d'oxalyle correspondant) en solution dans 20 mL de dichlorométhane, et on agite le milieu à température ambiante pendant 4 heures. La phase organique est ensuite diluée avec du CH₂Cl₂, lavée avec de l'eau, puis séchée avec du Na₂SO₄. La phase organique est ensuite concentrée, puis les cristaux récupérés sont ensuite recristallisés dans le méthanol pour obtenir 2,36 g (rendement = 85%) d'amide de formule **(IV).**
Pf = 186°C ; **[ES/MS]** m/z 332 [M + 1]⁺

### 2) Préparation de l'aniline de formule (VI) (figure 1a)

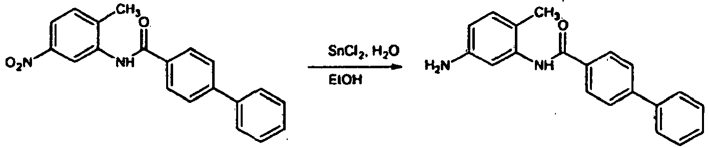

L'amide de formule **(IV)** obtenu (1,1 g, 3 mmol) est ensuite dissous dans 32 mL d'éthanol, et le milieu est chauffé à 80°C. Du SnCl₂, H₂O (3,8 g, 5 éq., 16 mmol) est ajouté en une fois. Le milieu est ensuite mis à chauffer pendant encore 2 heures, puis versé sur un mélange eau/glace et alcanisé avec du Na₂CO₃. Le mélange est ensuite extrait avec de l'acétate d'éthyle. La phase organique est lavée avec une solution saturée de NaCl, puis séchée et concentrée sous vide pour obtenir des cristaux. Ce résidu est recristallisé dans l'éthanol pour obtenir un solide (920 mg, 66%).
Pf = 96°C ; **[ES/MS]** m/z 303 [M + 1]⁺

### 3) Préparation de l'acyl-thiourée de formule (IX) (figure 1a)

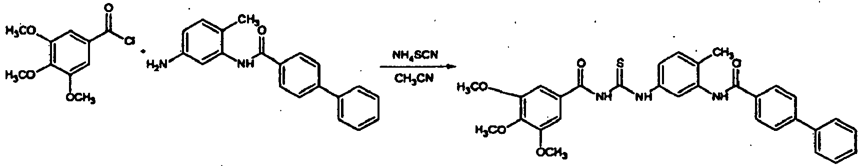

Du thiocyanate d'ammonium (123 mg, 1,2 éq., 1,63 mmol) et du chlorure de 3,4,5-triméthoxybenzoyle (345 mg, 1,1 éq., 1,5 mmol) sont dissous dans 5 mL d'acétone. Le mélange est chauffé pendant 1 heure au reflux. L'aniline obtenue lors de l'étape précédente (408 mg, 1 éq., 1,35 mmol) est ajouté, et le reflux est maintenu pendant 1 heure supplémentaire. Le mélange est ensuite versé sur de l'eau, puis filtré et recristallisé pour obtenir l'acyl-thiourée de formule **(IX)** (587 mg, 78%).
Pf= 173°C ; **[ES/MS]** m/z 556 [M + 1]⁺

### 4) Obtention du Composé 24

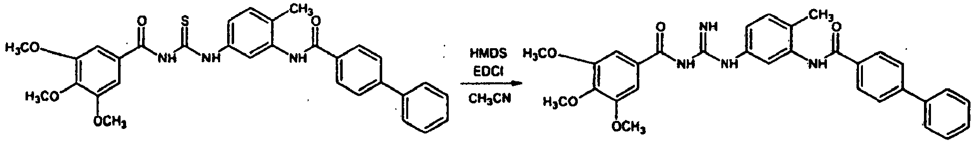

L'acyl-thiourée de formule **(IX)** obtenu précédemment (250 mg, 0,125 mmol) et l'hexaméthyldizilazane (0,95 mL, 1,25 mmol) sont dissous dans 6 mL d'acétonitrile, puis refroidis à 0°C dans un bain de glace. Du chlorhydrate de 1-éthyl-3(3-diméthylamino)propylcarbodiimide (171 mg, 2 éq., 0,9 mmol) est ensuite ajouté. Le mélange est agité à température ambiante pendant 5 heures. Le milieu réactionnel est versé sur de la glace, et la phase aqueuse est extraite plusieurs fois avec de l'acétate d'éthyle. La phase organique est lavée avec une solution saturée de NaCl, puis séchée et concentrée sous vide. Le résidu obtenu est cristallisé dans un mélange isopropanol/heptane, puis purifié par chromatographie sur SiO₂ (éluant : acétate d'éthyle/heptane : 4/1, puis acétate d'éthyle pur), pour obtenir 161 mg du Composé **24.**
Pf = 135°C ; **[ES/MS]** m/z 513 [M + 1]⁺

### D) Synthèse du Composé 38

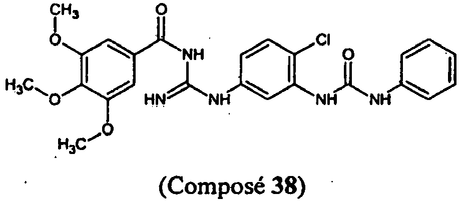

### 1) Préparation de l'adduit de formule (IV') (figure 1a)

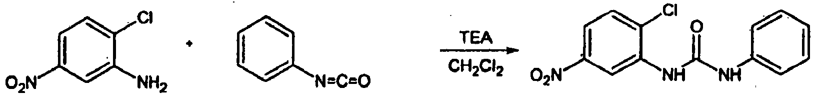

La 2-chloro-5-nitro-aniline (5,17 g, 30 mmol) et le phénylisocyanate (3,57 g, 3,5 mL, 30 mmol) sont dissous dans 30 mL de tétrahydrofuranne (THF), puis chauffé à reflux pendant 4 heures. Le résidu obtenu est évaporé sous vide et purifié par chromatographie sur SiO₂ (éluant : acétate d'éthyle/heptane : 3/7) pour obtenir l'adduit de formule **(IV')** (116 mg, 79%).
Pf = 142°C; **[ES/MS]** m/z 292 [M + 1]⁺

### 2) Préparation de l'aniline de formule (VI') (figure 1a)

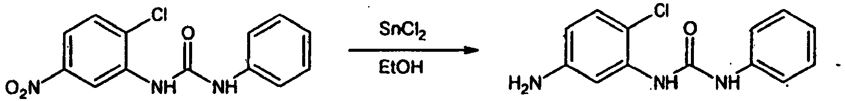

L'adduit de formule **(IV')** (514 mg, 2 mmol) est dissous dans 20 mL d'éthanol, et le milieu est chauffé à 80°C. Du SnCl₂, H₂O (2,3 g, 5 éq., 10 mmol) est ajouté en une fois. Le milieu est ensuite mis à chauffer pendant encore 2 heures, puis versé sur un mélange eau/glace et alcanisé avec du Na₂CO₃. Le mélange est ensuite extrait avec de l'acétate d'éthyle. La phase organique est lavée avec une solution saturée de NaCl, puis séchée et concentrée sous vide pour obtenir des cristaux. Ce résidu est purifié par chromatographie sur SiO₂ pour obtenir une huile (404 mg, 89%).
Pf = 134°C ; **[ES/MS]** m/z 228 [M + 1]⁺

### 3) Préparation de l'acyl-thiourée de formule (X) (figure 1b)

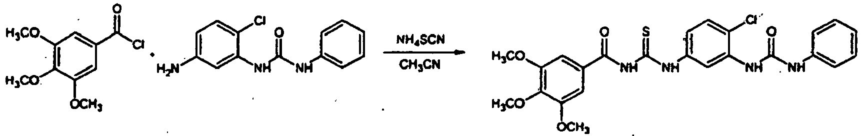

Du thiocyanate d'ammonium (82 mg, 1,2 éq., 1,09 mmol) et du chlorure de 3,4,5-triméthoxybenzoyle (230 mg, 1,1 éq., 1 mmol) sont dissous dans 5 mL d'acétone. Le mélange est chauffé pendant 1 heure au reflux. L'aniline obtenue lors de l'étape précédente (204 mg, 1 éq., 0,9 mmol) est ajoutée, et le reflux est maintenu pendant 1 heure supplémentaire. Le mélange est ensuite versé sur de l'eau, puis filtré et recristallisé dans l'acétonitrile pour obtenir l'acyl-thiourée de formule **(X)** (185 mg, 42%).
Pf= 250°C, **[ES/MS]** m/z 481 [M + 1]⁺

### 4) Obtention du Composé 38

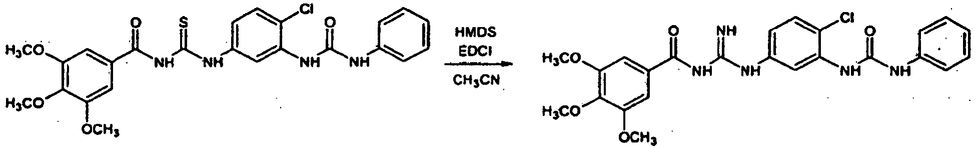

L'acyl-thiourée de formule **(X)** obtenue précédemment (72 mg, 0,15 mmol) et l'hexaméthyldizilazane (0,32 mL, 10 éq., 1,5 mmol) sont dissous dans 1,5 mL d'acétonitrile, puis refroidis à 0°C dans un bain de glace. Du chlorhydrate de 1-éthyl-3(3-diméthylamino)propylcarbodiimide (58 mg, 2 éq., 0,3 mmol) est ensuite ajouté. Le mélange est agité à température ambiante pendant 5 heures. Le milieu réactionnel est versé dans l'eau, et la phase aqueuse est extraite avec de l'acétate d'éthyle. La phase organique est lavée avec une solution saturée de NaCl, puis séchée et concentrée sous vide. Le résidu obtenu est purifié par chromatographie sur SiO₂ (éluant : acétate d'éthyle/heptane : 1/1) pour obtenir le Composé **38** (35 mg, 52%) ayant un Pf de 179°C.

Les analyses obtenues pour tous les composés de formule **(I),** synthétisés par analogie selon les procédés détaillés ci-dessus, sont données ci-après :
- Composé **1 :** 3,4,5-triméthoxy-N-(N-(3-(4-méthoxybenzamido)phényl)carbamimidoyl)benzamide (C₂₅H₂₆N₄O₆)
   Poids Moléculaire (PM) = 478 ; **[ES/MS]** m/z 479 [M + 1]⁺; Pf = 126°C
- Composé **2:** N-(N-(3-(2-chlorobenzamido)phényl)carbamimidoyl)-3,4,5-triméthôxybenzamide (C₂₄H₂₃ClN₄O₅)
   PM = 482 ; **[ES/MS]** m/z 483 [M + 1]⁺; Pf = 139°C
- Composé **3:** 3,4,5-triméthoxy-N-(N-(3-(3-méthoxybenzamido)phényl)carbamimidoyl)benzamide (C₂₅H₂₆N₄O₅)
   PM = 478 ; **[ES/MS]** m/z 479 [M + 1]⁺; Pf = 118°C
- Composé **4:** N-(3-(3-(3,4,5-triméthoxybenzoyl)guanidino)phényl)biphényl-4-carboxamide
   PM = 524; **[ES/MS]** m/z 525 [M + 1]⁺; Pf = 134°C
- Composé **5:** N-(N-(3-(cyclohexanecarboxamido)phényl)carbamimidoyl)-3,4,5-triméthoxybenzamide (C₂₄H₃₀N₄O₅)
   PM = 454 ; **[ES/MS]** m/z 455 [M + 1]⁺; Pf =168°C
- Composé **6:** N-(3-(3-(3,4,5-triméthoxybenzoyl)guanidino)phényl)-2-naphthamide (C₂₇H₂₃N₄O₅)
   PM = 483 ; **[ES/MS]** m/z 484 [M + 1]⁺; Pf= 154°C
- Composé **7:** N-(3-(3-(3,4,5-triméthoxybenzoyl)guanidino)phényl)isonicotinamide (C₂₃H₂₃N₅O₆)
   PM = 449 ; **[ES/MS]** m/z 450 [M + 1]⁺; Pf = 126°C
- Composé **8:** N-(3-(3-(3,4,5-triméthoxybenzoyl)guanidino)phényl)furan-2-carboxamide (C₂₂H₂₂N₄O₆)
   PM = 438 ; **[ES/MS]** m/z 439 [M + 1]⁺; Pf = 151°C
- Composé **9 :** N-(N-(3-benzamidophényl)carbamimidoyl)-3,5-diméthoxybenzamide (C₂₃H₂₂N₄O₄)
   PM = 418 ; **[ES/MS]** m/z 419 [M + 1]⁺; Pf = 125°C
- Composé **10:** N-(N-(3-benzamidophényl)carbamimidoyl)-4-éthoxy-3,5-diméthoxybenzamide (C₂₅H₂₆N₄O₅)
   PM = 462 ; **[ES/MS]** m/z 463 [M + 1]⁺; Pf = 127°C
- Composé **11** : N-(N-(3-benzamidophényl)carbamimidoyl)-3,4-diéthoxy-5-méthoxybenzamide (C₂₆H₂₈N₄O₅)
   PM = 476 ; **[ES/MS]** m/z 477 [M + 1]⁺; Pf = 129°C
- Composé **12:** N-(N-(3-benzamidophényl)carbamimidoyl)-7-méthoxybenzo[*d*][1,3]dioxole-5-carboxamide (C₂₃H₂₀N₄O₅)
   PM = 432 ; **[ES/MS]** m/z 433 [M + 1]⁺ ; Pf= 140°C
- Composé **13 :** N-(N-(3-benzamidophényl)carbamimidoyl)-2,4-diméthoxybenzamide (C₂₃H₂₂N₄O₄)
   PM = 418 ; **[ES/MS]** m/z 419 [M + 1⁺; Pf= 136°C
- Composé **14:** N-(N-(3-benzamido-4-fluorophényl)carbamimidoyl)-3,4,5-triméthoxybenzamide (C₂₄H₂₃FN₄O₅)
   PM = 466 ; **[ES/MS]** m/z 467 [M + 1]⁺; Pf = 153°C
- Composé **15:** N-(N-(3-benzamidophényl)carbamimidoyl)-3,4,5-triméthoxybenzamide (C₂₄H₂₄N₄O₅)
   PM = 448 ; **[ES/MS]** m/z 449 [M + 1]⁺; Pf = 138°C
- Composé **16:** N-(N-(3-benzamido-4-chlorophényl)carbamimidoyl)-3,4,5-triméthoxybenzamide (C₂₄H₂₃ClN₄O₅)
   PM = 482 ; **[ES/MS]** m/z 483 [M + 1]⁺; Pf = 146°C
- Composé **17:** N-(2-chloro-5-(3-(3,4,5-triméthoxybenzoyl)guanidino)phényl)biphényl-4-carboxamide
   PM = 559 ; **[ES/MS]** m/z 560 [M + 1]⁺; Pf = 177°C
- Composé **18:** 3,4,5-triméthoxy-N-(N-(3-(4-morpholinobenzamido)phényl)carbamimidoyl)benzamide (C₂₈H₃₁N₅O₆)
   PM = 533 ; **[ES/MS]** m/z 531 [M + 1]⁺; Pf= 137°C
- Composé **19:** N-(N-(3-(1*H-*indol-2-yl)phényl)carbamimidoyl)-3,4,5-triméthoxybenzamide (C₂₅H₂₄N₄O₄)
   PM = 444 ; **[ES/MS]** m/z 445 [M + 1]⁺; Pf = 225°C
- Composé **20:** N-(N-(4-chloro-3-(4-méthoxyphénylcarbamoyl)phényl)carbamimidoyl)-3,4,5-triméthoxybenzamide (C₂₅H₂₅ClN₄O₆)
   PM =512 ; **[ES/MS]** m/z 513 [M + 1 ]⁺; Pf = 105°C
- Composé **21 :** N-(N-(4-chloro-3-(phénylcarbamoyl)phényl)carbamimidoyl)-3,4,5-triméthoxybenzamide (C₂₄H₂₃ClN₄O₅)
   PM = 482 ; **(ES/MS]** m/z 483 [M + 1]⁺; Pf = 142-146°C
- Composé **22:** 3,4,5-triméthoxy-N-(N-(4-méthyl-3-(phénylcarbamoyl)phényl)carbamimidoyl)benzamide
   PM = 462 ; **[ES/MS]** m/z 463 [M + 1]⁺; Pf = 114°C
- Composé **23:** 4'-fluoro-N-(3-(3-(3,4,5-triméthoxybenzoyl)guanidino)phényl)biphényl-4-carboxamide (C₃₀H₂₇FN₄O₅)
   PM = 542 ; **[ES/MS]** m/z 543 [M + 1]⁺ ; Pf = 127°C
- Composé **24:** N-(2-méthyl-5-(3-(3,4,5-triméthoxybenzoyl)guanidino)phényl)biphényl-4-carboxamide (C₃₁H₃₀N₄O₅)
   PM = 538 ; **[ES/MS]** m/z 539 [M + 1]⁺; Pf = 155°C
- Composé **25:** N-(2-méthyl-5-(3-(3,4,5-triméthoxybenzoyl)guanidino)phényl)biphényl-3-carboxamide (C₃₁H₃₀N₄O₅)
   PM = 538 ; **[ES/MS]** m/z 539 [M + 1]⁺; Pf = 128°C
- Composé **26:** N-(2-chloro-5-(3-(3,4,5-triméthoxybenzoyl)guanidino)phényl)biphényl-3-carboxamide
   PM = 559 ; **[ES/MS]** m/z 560 [M + 1 ]⁺ ; Pf = 142°C
- Composé **27 :** N-N-(3-benzamido-4-méthylphényl)carbamimidoyl)-3,4,5-triméthoxybenzamide
   PM = 462 ; **[ES/MS]** m/z 463 [M + 1]⁺ ; Pf = 121°C
- Composé **28 :** N-N-(3-(imidazo[2,1-*b*]thiazol-6-yl)phényl)carbamimidoyl)-3,4,5-triméthoxybenzamide (C₂₂H₂₁N₅O₄S)
   PM = 451 ; **[ES/MS]** m/z 452 [M + 1]⁺ ; Pf = 74°C
- Composé **29:** N-(N-(3-(1*H*-indol-1-yl)phényl)carbamimidoyl)-3,4,5-triméthoxybenzamide (C₂₅H₂₄N₄O₄)
   PM = 444 ; **[ES/MS]** m/z 445 [M + 1]⁺ ; Pf= 163°C
- Composé **30 :** 4'-fluoro-N-(2-méthyl-5-(3-(3,4,5-triméthoxybenzoyl)guanidino)phényl)biphényl-4-carboxamide (C₃₁H₂₉FN₄O₅)
   PM = 556 ; **[ES/MS]** m/z 557 [M + 1]⁺; Pf = 121°C
- Composé **31:** 3,4,5-triméthoxy-N-(N-(4-méthyl-3-(4-(pyridin-4-yl)benzamido)phényl)carbamimidoyl)-benzamide (C₃₀H₂₉N₅O₅)
   PM = 539 ; **[ES/MS]** m/z 540 [M + 1]⁺; Pf= 156°C
- Composé **32 :** N-(N-(4-chloro-3-(4-phénoxybenzamido)phényl)carbamimidoyl)-3,4,5-triméthoxybenzamide (C₃₀H₂₇ClN₄O₆)
   PM = 575 ; **[ES/MS]** m/z 576 [M + 1]⁺ ; Pf = 132°C
- Composé **33 :** N-(3-(3-benzoylguanidino)phényl)biphényl-4-carboxamide (C₂₇H₂₂N₄O₂)
   PM = 434 ; **[ES/MS]** m/z 435 [M + 1]⁺; Pf= 148°C
- Composé **34:** N-(2-méthyl-3-(3-(3,4,5-triméthoxybenzoyl)guanidino)phényl)biphényl-4-carboxamide (C₃₁H₃₀N₄O₅)
   PM = 538 ; **[ES/MS]** m/z 539 [M + 1]⁺; Pf= 125°C
- Composé **35:** N-(4-méthyl-3-(3-(3,4,5-triméthoxybenzoyl)guanidino)phényl)biphényl-4-carboxamide (C₃₁H₃₀N₄O₅)
   PM = 538 ; **[ES/MS]** m/z 539 [M + 1]⁺; Pf = 132°C
- Composé **36:** N-(N-(3-benzamidophényl)carbamimidoyl)benzamide (C₂₂H₂₀N₄O₂)
   PM = 372 ; **[ES/MS]** m/z 373 [M + 1]⁺; Pf = 127°C
- Composé **37:** N-(N-(3-(3-biphényl-4-ylureido)phényl)carbamimidoyl)-3,4,5-triméthoxybenzamide (C₃₀H₂₉N₅O₅)
   PM = 539 ; **[ES/MS]** m/z 540 [M + 1]⁺; Pf = 173°C
- Composé **38 :** N-(N-(4-chloro-3-(3-phénylureido)phényl)carbamimidoyl)-3,4,5-triméthoxybenzamide (C₂₄H₂₅N₅O₅)
   PM = 463 ; **[ES/MS]** m/z 464 [M + 1]⁺; Pf= 147°C

### EXEMPLE 2: MISE EN ÉVIDENCE DE L'EFFET MODULATEUR DES COMPOSÉS DE FORMULE (I) SUR LA VOIE DE SIGNALISATION DES PROTÉINES HEDGEHOG ET DE LEUR FIXATION SUR LE RECEPTEUR SMOOTHENED

L'effet des composés de formule **(I)** conformes à l'invention sur l'inhibition de la voie de signalisation des protéines Hedgehog a été déterminé *in vitro* par analyse de la différenciation de la lignée de cellules fibroblastiques pluripotentes C3H10T1/2 (ATCC) après activation de cette voie dans ces cellules par un activateur synthétique : le SAG. L'activité *in vivo* de l'un des composés a été mise en évidence sur les cellules de la zone sous-ventriculaire du cerveau de souris adulte après injection stéréotaxique en présence de la protéine Sonic Hedgehog. La capacité des composés de formule **(I)** à se lier au récepteur *Smoothened* de souris à également été déterminée par compétition avec la bodycyclopamine, un composé fluorescent dérivé de la cyclopamine et se fixant sur les domaines transmenbranaires du récepteur, comme décrit par Chen et al., Genes Dev., 2002, 16, 2743.

### 1) Matériels et méthodes

### 1) 1- Inhibition de la voie Hedgehog par les composés de formule (I)

Les composés de formule **(I)** à tester ont été dissous dans le diméthylsulfoxyde jusqu'à une concentration de 10 mM, puis stockés à une température de -20°C jusqu'à utilisation.

La lignée de cellules fibroblastiques pluripotentes C3H10T1/2 a été cultivée dans les conditions recommandées par l'American Type Culture Collection (ATCC). L'activation de ces cellules a été réalisée en utilisant 0,1 µM de SAG selon les méthodes décrites par Chen et al., Proc. Natl. Acad. Sci. USA, 2002, 99, 14071 et Frank-Kamenetsky et al., J. Biol., 2002, 1, 10.

L'activation par le SAG provoque la différenciation de la lignée cellulaire et leur permet d'exprimer la phosphatase alcaline. On a ainsi pu mesurer l'activité de la voie de signalisation des protéines Hedgehog via la mesure de l'activité phosphatase alcaline.

Les cellules C3H10T1/2 ont été ensemencées sur des plaques de 96 puits à une densité de 5.10³ cellules par puits, 24 heures avant l'addition des composés à tester à une concentration variant de 1 nM à 30 µM et en présence de 0,1 µM de SAG, en utilisant comme milieu de culture du DMEM (Dulbecco's Modified Eagles's Medium) avec 10% de sérum de veau foetal. Les essais ont été réalisés en quadruplate. Les plaques ont ensuite été incubées pendant 5 à 6 jours à une température de 37°C sous atmosphère à 5% de CO₂. Les cellules ont ensuite été lavées dans un tampon phosphate froid ("Phosphate Buffer Serum" : PBS), puis lysées par sonication à 4°C dans 50 µL d'une solution contenant 0,9% de NaCl et 0,2% de Triton X-100.

A titre comparatif, l'activité d'autres inhibiteurs connus de la voie de signalisation des protéines Hedgehog :
- le CURIS 61414 (Cur61414), tel que décrit par exemple par Frank-Kamenetsky M. et al., J. Biol., 2002, 1, 10, et répondant à la formule suivante : 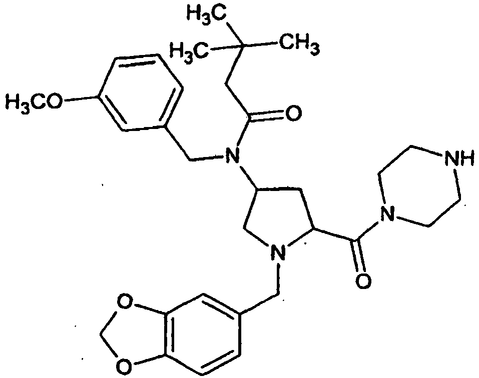
- la cyclopamine, tel que décrit par Indardona et al., Development, 1998, 125, 3553, et répondant à la formule suivante : 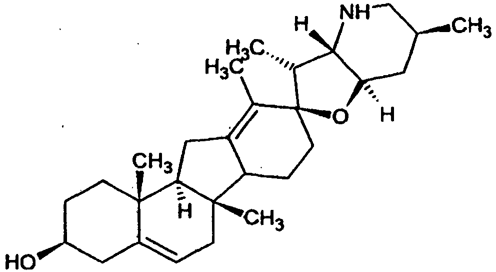
- le GDC-0449, tel que décrit par Miller-Moslin et al., J. Med. Chem., 2009, 52, 3954-3968, de formule : 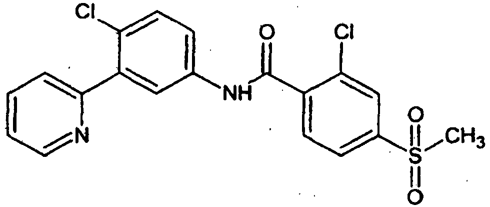 ont été testés dans les mêmes conditions que celles utilisées pour tester les différents composés de formule **(I)** conformes à l'invention.

La mesure de l'activité phosphatase alcaline dans les lysats ainsi obtenus a ensuite été réalisée selon la méthode décrite par Pepinsky et al. (J. Biol. Chem., 1998, 273, 14037). Après addition de 100 µL de tampon réactionnel (200 mM Tris-HCl ; pH 10,5 ; 0,4 M de 2-amino-2-méthylpropanol et 8 mM de MgCl₂) et de 50 µL de substrat (4 mM de p-nitrophénylphosphatedisodium), les lysats ont été incubés à 37°C pendant 30 à 60 minutes, puis la densité optique a été lue à une longueur d'onde de 415 nm.

### 1) 2- Compétition des composés de formule (I) avec la bodipycyclopamine

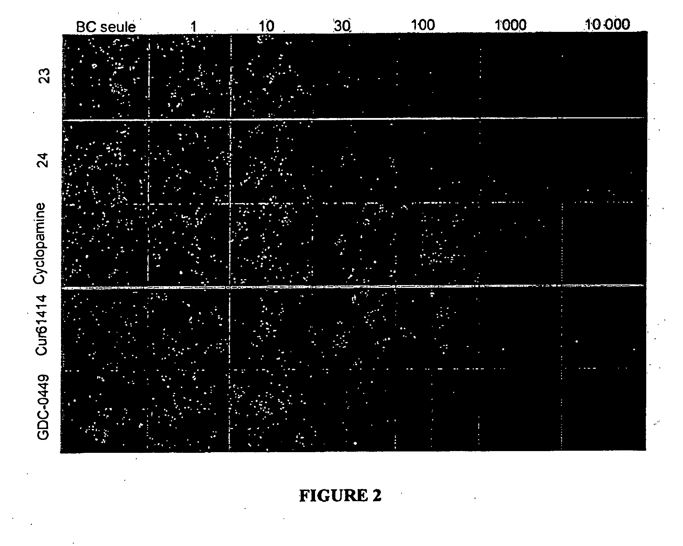

Les cellules HEK293 sont ensemencées à 70 000 cellules par puits sur lamelles de verre traitées à la poly-D-lysine en plaque 24 puits et transfectées le lendemain par 0,25 µg de plasmide encodant la protéine *Smoothened* de souris en utilisant 0,7 µL de Fugene6 (Roche biochemicals) en suivant le protocole décrit par le fournisseur (i. e. 0,7 µL de Fugene 6 sont ajoutés à 24 µL de DMEM sans aucun additif, dans chaque puits. Le mélange est ensuite incubé pendant 5 minutes à température ambiante, 0,25 µg d'ADN plasmidique sont ajoutés, puis l'ensemble est mélangé et incubé pendant 20 à 30 minutes à température ambiante. 25 µL du mélange ainsi préparé sont alors ajoutés directement dans le milieu de culture des cellules, dans chaque puits de la plaque contenant 24 puits). Après 48 heures, le milieu de culture est éliminé, les cellules rincées une fois avec 1 mL d'une solution tampon phosphate PBS (Phosphate Buffered Saline), puis fixées 20 minutes en présence d'une solution glacée de paraformaldéhyde (PFA) à 4%, de glucose à 0,12 M dans une solution tampon phosphate PBS. Les cellules sont ensuite rincées une fois et lavées 2 fois pendant 5 minutes avec 1 ml d'une solution tampon phosphate PBS, 0,5% de sérum de veau foetal (PBS-SVF). Ensuite, 1 mL de bodipycyclopamine (BC) (Chen, J. K., Taipale, J., Cooper, M. K., and Beachy, P. A., Genes Dev., 2002, 16(21), 2743-2748), diluée à 5 nM dans du PBS-SVF, en présence ou non de concentrations croissantes des composés à tester, est appliqué sur les cellules pendant 2 heures à 37°C. Les cellules sont ensuite lavées 2 fois pendant 5 minutes avec 1 mL de PBS-SVF, puis mises en présence de 1 mL d'une solution tampon phosphate PBS 1X. Enfin, les lamelles sont montées sur une lame de verre en présence de Vectashield contenant du DAPI (4',6'-DiAmidino-2-Phényle Indole) pour marquer les noyaux cellulaires (Vector). Des séries de trois photos par lamelle sont prises au microscope à fluorescence (DMRXA2, Leica; logiciel openlab3.1.2, improvision) (figure 2). L'intensité de la fluorescence est ensuite analysée à l'aide du logiciel Simple PCI 6.2 (Hamamatsu Corporation), puis rapportée à la surface des noyaux présents sur la photographie. Cette intensité dépend de l'inhibition de la bodipycyclopamine par les composés analysés.

### 1) 3- Activité inhibitrice des composés de formule (I) in vivo vis-à-vis de l'activation de la voie Shh au niveau des niches des précurseurs neuraux dans le cerveau de souris

Des souris swiss mâles adultes (âgées de 8 semaines, 35 g) ont été anesthésiés avec un mélange de kétamine (Mérial^{®}, Lyon, France) (0,1 mg/g) et de xylazine (Bayer^{®}, Puteaux, France) (0,01 mg/g) par injection intra-péritonéale (i. p.). La protéine Shh recombinante (290 ng dans une solution tampon de 150 mM de chlorure de sodium NaCl et 0,5 mM de dithithériol DDT) a été diluée dans 4,5 µL d'une solution de 2-hydropropyl-bétacyclodextrine (HBC) à 45% dans du PBS comprenant ou non 4,5 pmol de Cur61414 ou de Composé **24.** Ce mélange a été injecté stéréotaxiquement dans le ventricule latéral (VL) droit (n = 5 animaux pour chaque groupe), aux coordonnées suivantes données par rapport à l'axe du Bregma : antéropostérieur + 0,2 mm; latéral + 0,8 mm ; dorsoventral - 2,5 mm. L'atlas de référence pour les coordonnées stéréotaxiques est : The mouse brain in stereotaxic coordinates, Georges Paxinos, Keith B. J., Franklin, 2nd edition, 2001, Academic Press (San Diego, Etats-Unis).

La détection de l'ARN messager de *Patched* par hybridation in situ a été réalisée 48 heures après l'injection, tel que décrit par Traiffort et al., Eur. J. Neursci, 1999, 11, 3199-3214.

### 2) Résultats

### 2) 1- Inhibition de la voie Hedgehog par les composés de formule (I)

Des résultats obtenus avec des composés de formule **(I)** sont reportés dans le tableau 1 ci-après. Pour chacun des composés, la concentration qui permet d'inhiber 50% de l'activité phosphatase alcaline (IC₅₀) après induction par le SAG à 0,1 µM a été évaluée. Dans ce tableau, la lettre **A** correspond à une IC₅₀ comprise entre 3 et 30 nM, la lettre **B** correspond à une IC₅₀ comprise entre 30 et 300 nM et la lettre C à une IC₅₀ comprise entre 300 et 1000 nM.

**TABLEAU 1**

| **COMPOSÉS** | **IC₅₀ (µM)** |
|---|---|
| **Cur61414*** | **C** |
| **Cyclopamine*** | **C** |
| **GDC-0449*** | **A** |
| **15** | **C** |
| **16** | **C** |
| **17** | **B** |
| **18** | **B** |
| **19** | **B** |
| **20** | **C** |
| **21** | **C** |
| **22** | **B** |
| **23** | **A** |
| **24** | **A** |
| **28** | **C** |
| **29** | **C** |
| **30** | **A** |

| | |
|---|---|
| * : Composés de référence ne faisant pas partie de l'invention | |

### 2) 2- Compétition des composés de formule (I) avec la bodipycyclopamine

L'incubation en présence de concentration croissante de composés se traduit par une inhibition progressive de la fixation de la bodicyclopamine sur les cellules transfectées par le récepteur *Smoothened*, et donc de la fluorescence observée. La figure 2 montre un exemple d'expérience de compétition réalisée en parallèle pour la cyclopamine, le Cur61414, le GDC-0449 et les Composés **23** et **24.**

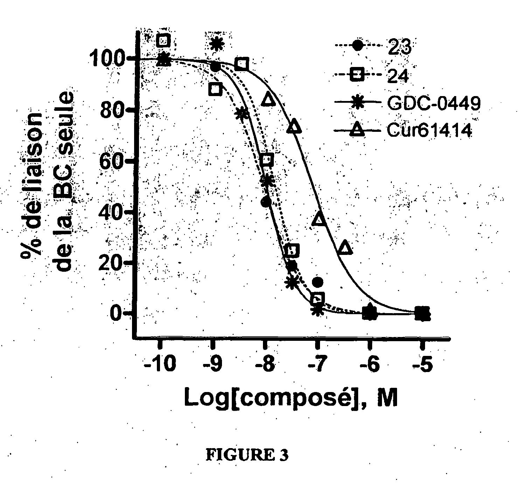

Trois expériences de compétition ont été réalisées indépendamment, et les courbes d'inhibition correspondantes ont été tracées sur la figure 3. La concentration qui permet d'inhiber 50% de la liaison bodipycyclopamine (IC₅₀) pour les composés de formule **(I)** et les composés de référence a été mesurée. Les résultats obtenus sont reportés dans le tableau 2 ci-après. Dans ce tableau, la lettre **A** correspond à une IC₅₀ comprise entre 3 et 30 nM et la lettre **B** correspond à une IC₅₀ comprise entre 30 et 300 nM.

**TABLEAU 2**

| **COMPOSÉS** | **IC₅₀ (µM)** |
|---|---|
| **Cur61414*** | **B** |
| **Cyclopamine*** | **B** |
| **GDC-0449*** | **A** |
| **23** | **A** |
| **24** | **A** |

| | |
|---|---|
| * : Composés de référence ne faisant pas partie de l'invention | |

Ces résultats démontrent que les composés de formule **(I)** conformes à l'invention sont des modulateurs de la voie de signalisation des protéines Hedgehog et qu'ils se lient au récepteur *Smoothened.* Ils sont par conséquent utiles pour le traitement des pathologies nécessitant un blocage de la voie Hedgehog, comme le cancer, ou pour le traitement de pathologies nécessitant une modulation de la voie Hedgehog, comme les maladies neurodégénératives et le diabète.

Certains de ces composés démontrent une affinité égale, voire supérieure, à celle du GDC-0449 actuellement en phase clinique.

### 2) 3- Activité inhibitrice des composés de formule (I) in vivo vis-à-vis de l'activation de la voie Shh au niveau des niches des précurseurs neuraux dans le cerveau de souris

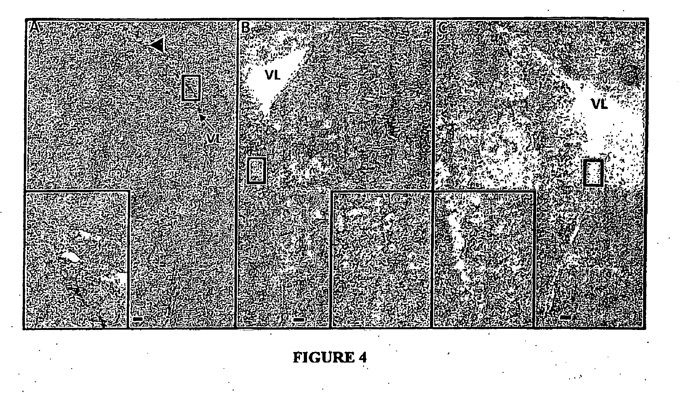

L'injection dans le ventricule latéral de cerveau de souris de la protéine recombinante Sonic Hedgehog permet de stimuler la voie Shh dans la zone sous ventriculaire (ZSV), une région qui contient les cellules souches et les précurseurs neuraux dans le cerveau de mammifères (Charytoniuk *et al.,* 2002 ; Loulier *et al.,* 2006 ; Angot *et al.,* 2008). Ces cellules sont capables de générer de nouveaux neurones et de nouvelles cellules gliales. La régulation de ces cellules en prolifération du cerveau mature fait intervenir la voie de signalisation Shh (Ahn et Joyner, Nature, 2005, 437, 894-897). L'implication de la voie Shh dans le développement de tumeurs du système nerveux central pourrait s'expliquer par des modifications de l'activité de la voie au niveau des régions de neurogénèse dans le cerveau adulte. Ainsi, le blocage de la voie de signalisation Shh au niveau de cette niche de neurogénèse peut être considéré comme un bon indice de l'activité antagoniste d'une molécule. L'injection de Shh au niveau du ventricule latérale se traduit par l'induction de gènes cibles dont Glil et *Patched* (*Ptc*). Nous avons mesuré l'induction de l'ARN messager du gène *Ptc* par hybridation *in situ* à l'aide d'une ribosonde spécifique. Cette induction est visible suite à l'injection de la protéine recombinante seule, mais disparaît lorsque l'on ajoute à cette protéine le Composé **24** ou le Cur61414 (figure 4, qui démontre une diminution de l'activité de la protéine ShhN sur l'expression de *Patched* dans la zone sous ventriculaire de souris en présence du Composé **24 et** du Cur61414 (A, B, C)).

Ces résultats démontrent la capacité des composés de formule **(I)** et du Cur61414 à inhiber la voie Shh *in vivo* chez le rongeur adulte, et suggèrent l'intervention de la protéine *Smoothened,* qui s'exprime au niveau des précurseurs neuraux, dans cette inhibition.

L'ensemble des expériences réalisées met en lumière la capacité des composés de formule **(I)** à moduler la voie Shh aussi bien *in vitro*, qu'*in vivo.* Leur activité pourrait s'expliquer par une liaison à la protéine *Smoothened* sur un site concurrent de la bodicyclopamine.

## Revendications

1. Composés **caractérisés en ce qu'**ils répondent à la formule **(I)** suivante : 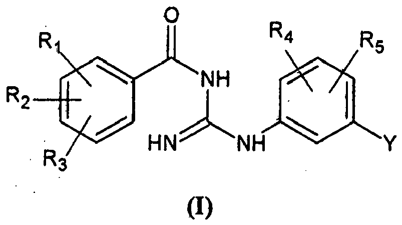 dans laquelle :
- R₁, R₂ et R₃, identiques ou différents et indépendamment les uns des autres, représentent un atome d'hydrogène ou d'halogène, un radical hydroxyle, un groupe alkyle, perfluoroalkyle, alcoxy éventuellement substitué, alkylthio, nitrile, ou un hétérocycle obtenu à partir de deux des R₁, R₂ et R₃ fusionnés avec deux atomes de carbone adjacents du cycle phényle auxquels ils sont liés ;
- Y représente un groupe hétéroaryle mono- ou polycyclique,-NH-(C=O)-R₆, -(C=O)-NH-R₆ ou -NH-(C=O)-NH-R₆, dans lequel R₆ représente un groupe aryle mono- ou polycyclique non substitué ; un groupe aryle comportant un ou plusieurs substituants choisis parmi un atome d'halogène, un radical alkyle, alcoxy ou alcoxyaryle, mono- ou dialkylamino, un groupe aryle ou hétéroaryle, un hétérocycle ; un groupe hétéroaryle mono- ou polycyclique ; un radical alkyle linéaire ou ramifié ; un groupe hydrocarboné mono- ou polycyclique saturé ou insaturé ;
- R₄ et R₅, identiques ou différents et indépendamment l'un de l'autre, représentent un atome d'hydrogène ou d'halogène, un groupe alcoxy, alkylthio, alkyle, perfluoroalkyle, nitrile ou nitro,
à titre de médicaments.

2. Composés de formule **(I)** selon la revendication 1, **caractérisés en ce qu'**ils sont choisis parmi ceux dans lesquels :
- R₁, R₂ et R₃, identiques ou différents, représentent un atome d'hydrogène, un radical méthyloxy ou éthyloxy, ou un dioxolane fusionné avec deux atomes de carbone adjacents du cycle phényle auquel ils sont liés ;
- R₄ et R₅, identiques ou différents, représentent un atome d'hydrogène, de chlore, de brome, de fluore, ou un radical méthyle ou méthoxy ; et
- Y représente :
- un groupe hétéroaryle mono- ou polycyclique, de préférence choisi parmi l'indole ou l'imidazole fusionné à un groupe thiazol, ou
- un groupe -NH-(C=O)-R₆, -(C=O)-NH-R₆ ou -NH-(C=O)-NH-R₆ dans lequel R₆ représente un groupe phényle, éventuellement substitué par un atome d'halogène, un radical alkyle, diaminoalkyle ou alcoxy, un groupe cycloalkyle, ou un groupe aryle ; un groupe cycloalkyle ; un groupe pyridinyle ; un groupe naphtyle ; un groupe furyle ; un groupe thiophényle ; un radical isopropyle, de préférence R₆ représente un groupe phényle, éventuellement substitué par un atome de chlore, un radical méthoxy, une morpholine, un groupe phényle ou phénoxy ; un groupe cyclohexyle ; un groupe pyridinyle ; un groupe naphtyle ; un groupe furyle.

3. Composés de formule **(I)** selon la revendication 1 ou la revendication 2, **caractérisés par le fait qu'**ils sont choisis parmi :
- le 3,4,5-triméthoxy-N-(N-(3-(4-méthoxybenzamido)phényl)carbamimidoyl)benzamide : 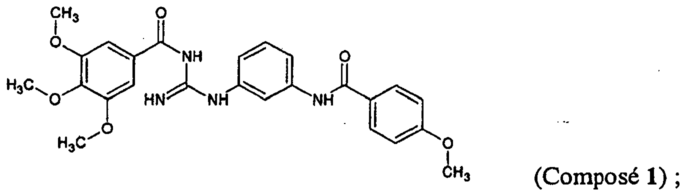
- le N-(N-(3-(2-chlorobenzamido)phényl)carbamimidoyl)-3,4,5-triméthoxybenzamide : 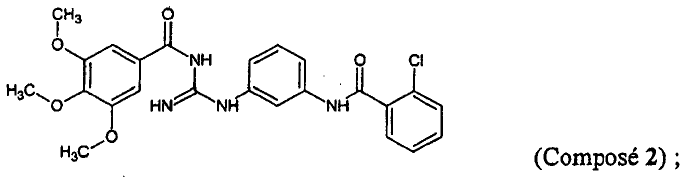
- le 3,4,5-triméthoxy-N-(N-(3-(3-méthoxybenzamido)phényl)carbamimidoyl)benzamide : 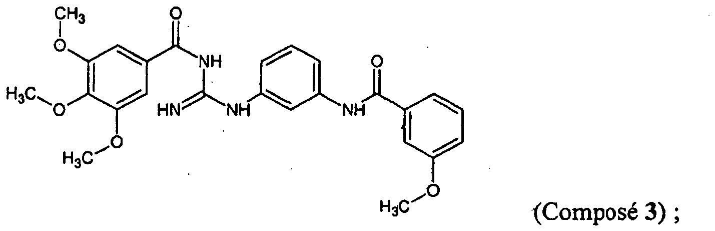
- le N-(3-(3-(3,4,5-triméthoxybenzoyl)guanidino)phényl)biphényl-4-carboxamide : 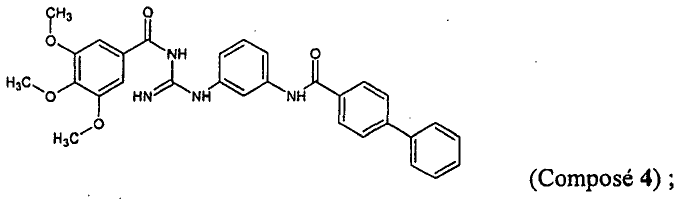
- le N-(N-(3-(cyclohexanecarboxamido)phényl)carbamimidoyl)-3,4,5-triméthoxybenzamide : 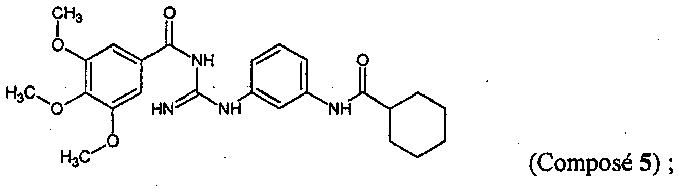
- le N-(3-(3-(3,4,5-triméthoxybenzoyl)guanidino)phényl)-2-naphthamide ; 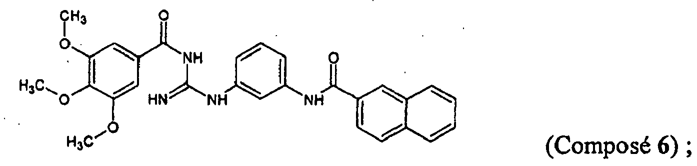
- le N-(3-(3-(3,4,5-triméthoxybenzoyl)guanidino)phényl)isonicotinamide : 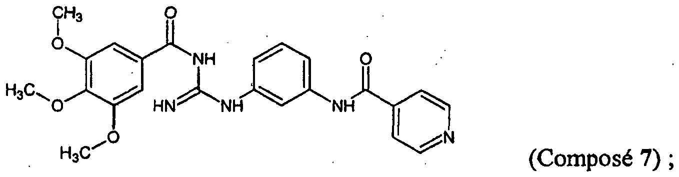
- le N-(3-(3-(3,4,5-triméthoxybenzoyl)guanidino)phényl)furan-2-carboxamide : 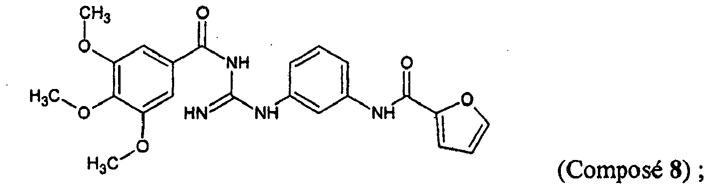
- le N-(N-(3-benzamidophényl)carbamimidoyl)-3,5-diméthoxybenzamide : 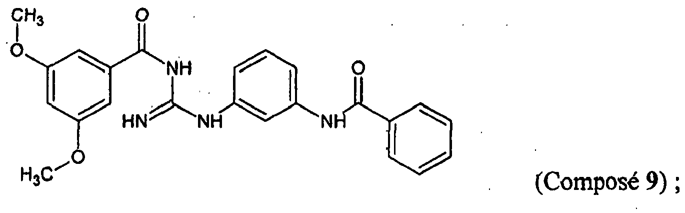
- le N-(N-(3-benzamidophényl)carbamimidoyl)4-éthoxy-3,5-diméthoxybenzamide : 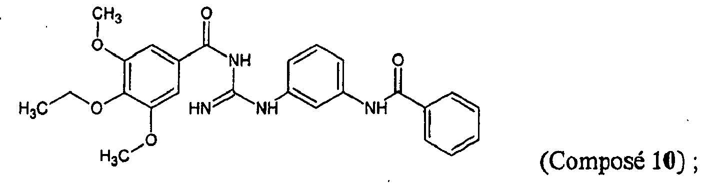
- le N-(N-(3-benzamidophényl)carbamimidoyl)-3,4-diéthoxy-5-méthoxybenzamide: 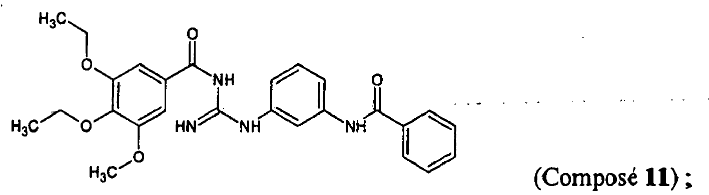
- le N-(N-(3-benzamidophényl)carbamimidoyl)-7-méthoxybenzo[d][1,3]dioxole-5-carboxamide : 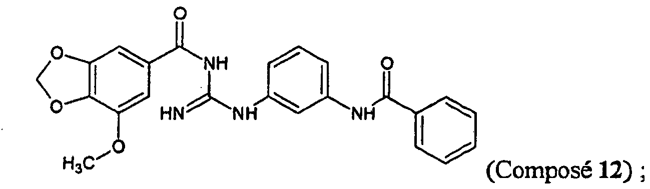
- le N-(N-(3-benzamidophényl)carbamimidoy)-2,4-diméthoxybenzamide : 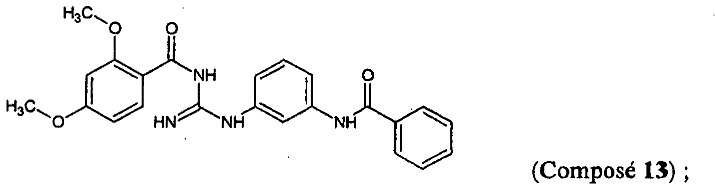
- le N-(N-(3-benzamido-4-fluorophényl)carbamimidoyl)-3,4,5-triméthoxybenzamide : 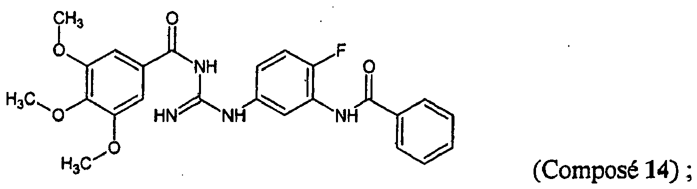
- le N-(N-(3-benzamidophényl)carbamimidoy)-3,4,5-triméthoxybenzamide : 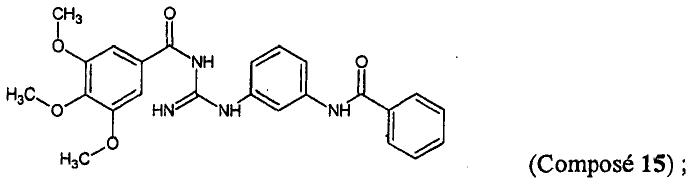
- le N-(N-(3-benzamido-4-chlorophényl)carbamimidoyl)-3,4,5-triméthoxybenzamide : 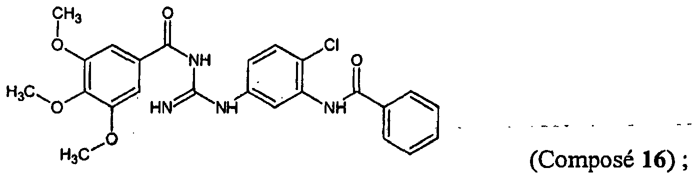
- le N-(2-chloro-5-(3-(3,4,5-triméthoxybenzoyl)guanidino)phényl)biphényl-4-carboxamide : 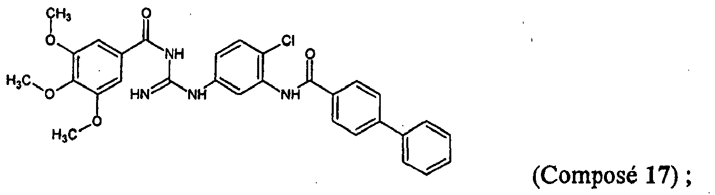
- le 3,4,5-triméthoxy-N-(N-(3-(4-morpholinobenzamido)phényl)carbamimidoyl)benzamide : 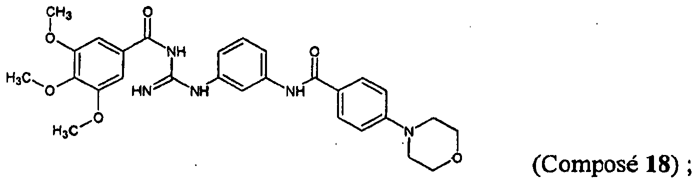
- le N-(N-(3-(1*H*-indol-2-yl)phényl)carbamimidoyl)3,4,5-triméthoxybenzamide : 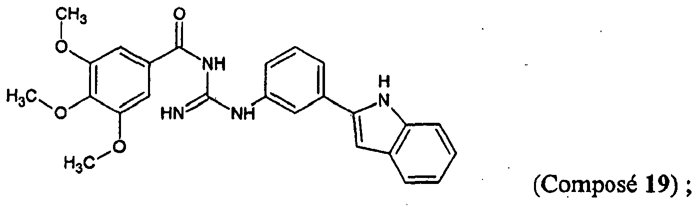
- le N-(N-(4-chloro-3-(4-méthoxyphénylcarbamoyl)phényl)carbamimidoyl)-3,4,5-triméthoxybenzamide: 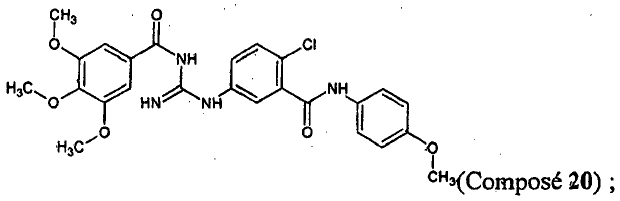
- le N-(N-(4-chloro-3-(phénylcarbamoyl)phényl)carbamimidoyl)-3,4,5-triméthoxybenzamide : 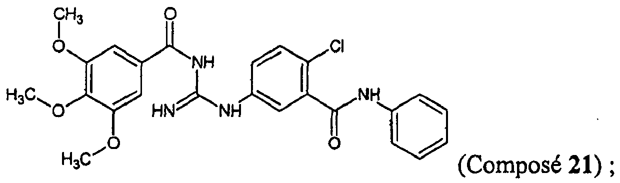
- le 3,4,5-triméthoxy-N-(N-(4-méthyl-3-(phénylcarbamoyl)phényl)carbamimidoyl)benzamide : 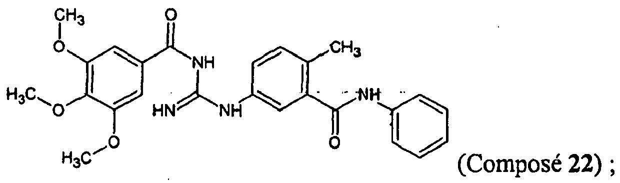
- le 4'-fluoro-N-(3-(3-(3,4,5-triméthoxybenzoyl)guanidino)phényl)biphényl-4-carboxamide : 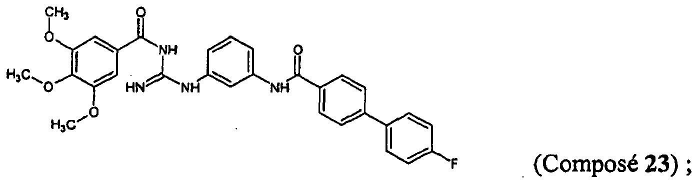
- le N-(2-methyl-5-(3-(3,4,5-triméthoxybenzoyl)guanidino)phényl)biphényl-4-carboxamide : 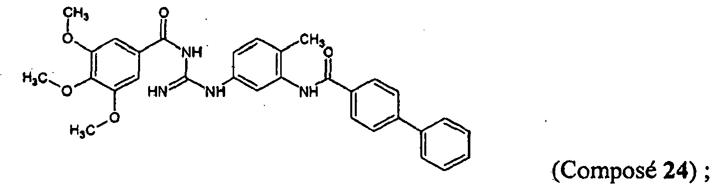
- le N-(2-méthyl-5-(3-(3,4,5-triméthoxybenzoyl)guanidino)phényl)biphényl-3-carboxamide : 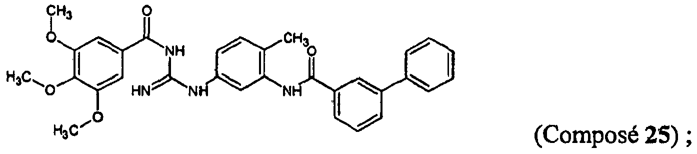
- le N-(2-chloro-5-(3-(3,4,5-triméthoxybenzoyl)guanidino)phényl)biphényl-3-carboxamide : 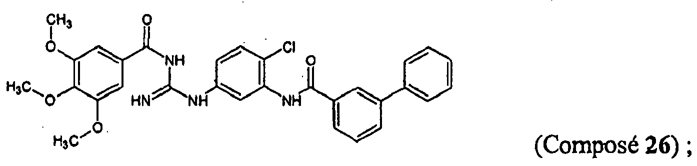
- le N-N-(3-benzamido-4-méthylphényl)carbamimidoyl)3,4,5-triméthoxybenzamide : 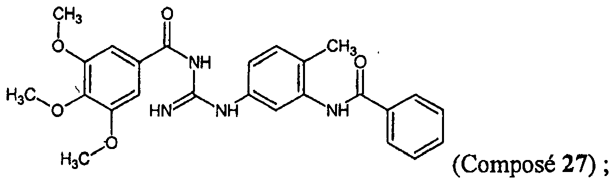
- le N-N-(3-(imidazo[2,1-*b*]thiazol-6-yl)phényl)carbamimidoyl)3,4,5-triméthoxybenzamide : 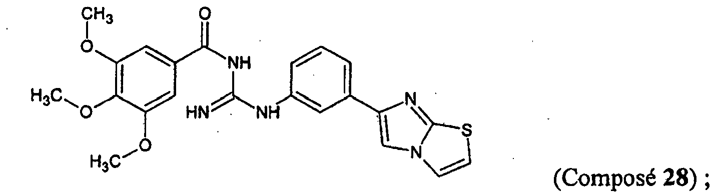
- le N-(N-(3-(1*H*-indol-1-yl)phényl)carbamimidoy)-3,4,5-triméthoxybenzamide : 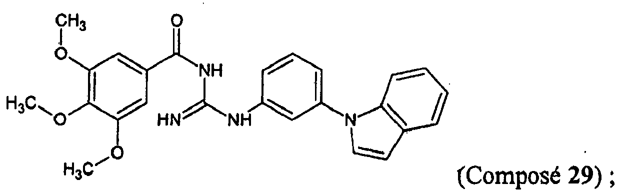
- le 4'-fluoro-N-(2-méthyl-5-(3-(3,4,5-triméthoxybenzoyl)guanidino)phényl)biphényl-4-carboxamide : 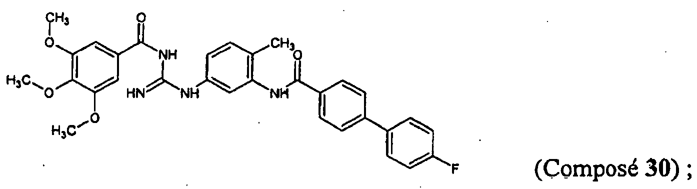
- le 3,4,5-triméthoxy-N-(N-(4-méthyl-3-(4-(pyridin-4-yl)benzamido)phényl)carbamimidoyl)-benzamide : 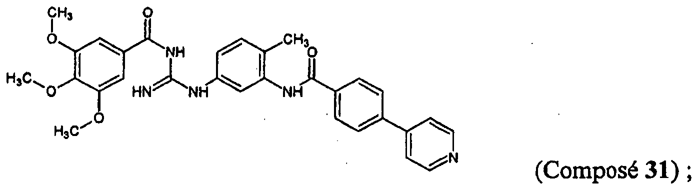
- le N-(N-(4-chloro-3-(4-phénoxybenzamido)phényl)carbamimidoyl)-3,4,5-triméthoxybenzamide : 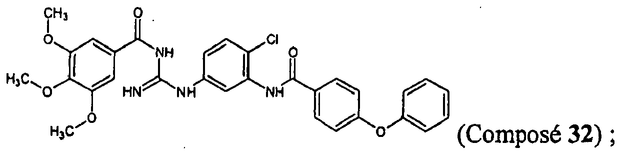
- le N-(3-(3-benzoylguanidino)phényl)biphényl-4-carboxamide : 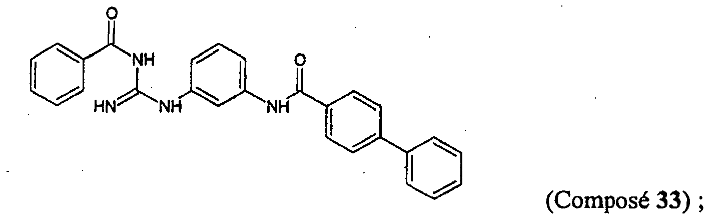
- le N-(2-méthyl-3-(3-(3,4,5-triméthoxybenzoyl)guanidino)phényl)biphényl-4-carboxamide : 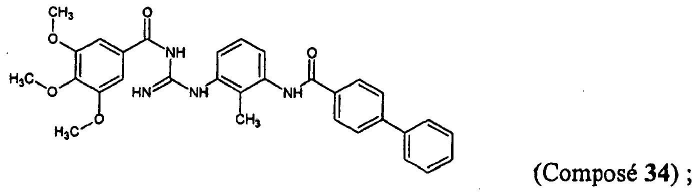
- le N-(4-méthyl-3-(3-(3,4,5-triméthoxybenzoyl)guanidino)phényl)biphényl-4-carboxamide : 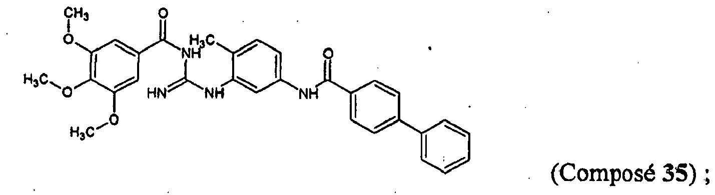
- le N-(N-(3-benzamidophényl)carbamimidoyl)benzamide : 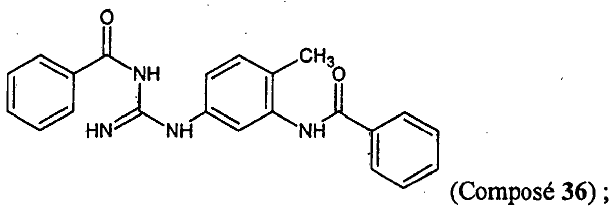
- le N-(N-(3-(3-biphényl-4-ylureido)phényl)carbamimidoyl)3,4,5-triméthoxybenzamide : 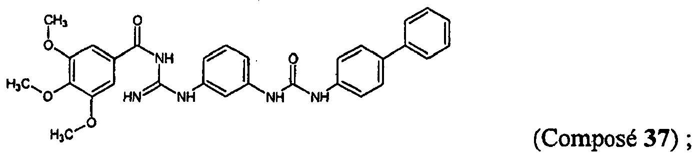
- le N-(N-(4-chloro-3-(3-phénylureido)phényl)carbamimidoyl)-3,4,5-triméthoxybenzatnide :

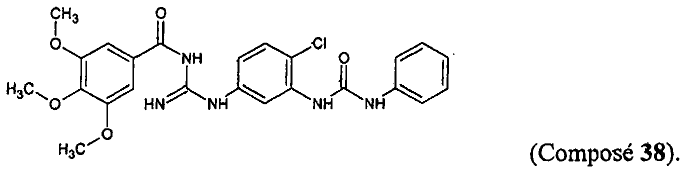

4. Composés selon la revendication 3, **caractérisés par le fait qu'**ils sont choisis parmi :
- le 3,4,5-triméthoxy-N-(N-(3-(4-méthoxybenzamido)phényl)carbamimidoyl)benzamide (Composé **1**) ;
- le N-(N-(3-(2-chlorobenzamido)phényl)carbamimidoyl)-3,4,5-triméthoxybenzamide (Composé **2**) ;
- le 3,4,5-triméthoxy-N-(N-(3-(3-méthoxybenzamido)phényl)carbamimidoyl)benzamide (Composé **3**) ;
- le N-(3-(3-(3,4,5-triméthoxybenzoyl)guanidino)phényl)-2-naphthamide (Composé **6**) ;
- le N-(3-(3-(3,4,5-triméthoxybenzoyl)guanidino)phényl)furan-2-carboxamide (Composé **8**) ;
- le N-(N-(3-benzamidophényl)carbamimidoyl)-3,5-diméthoxybenzamide (Composé **9**) ;
- le N-(N-(3-benzamidophényl)carbamimidoyl)-7-méthoxybenzo[*d*][1,3]dioxole-5-carboxamide (Composé **12**) ;
- le N-(N-(3-benzamidophényl)carbamimidoyl)-2,4-diméthoxybenzamide (Composé **13**) ;
- le N-(N-(3-benzamodophényl)carbamimidoyl)-3,4,5-triméthoxybenzamide (Composé **15**) ;
- le N-(2-chloro-5-(3-(3,4,5-triméthoxybenzoyl)guanidino)phényl)biphényl-4-carboxamide (Composé **17**) ;
- le N-(N-(3-(*1H*-indol-2-yl)phényl)carbamidoyl)-3,4,5-triméthoxybenzamide (Composé **19**) ;
- le N-(N-(4-chloro-3-(phénylcarbamoyl)phényl)carbamimidoyl)-3,4,5-triméthoxybenzamide (Composé **21**) ;
- le 4'-fluoro-N-(3-(3-(3,4,5-triméthoxybenzoyl)guanidino)phényl)biphényl-4-carboxamide (Composé **23**) ;
- le N-(2-méthyl-5-(3-(3,4,5-triméthoxybenzol)guanidino)phényl)biphényl-4-carboxamide (Composé **24**) ;
- le N-(N-(3-(*1H*-indol-1-yl)phényl)carbamimidoyl)-3,4,5-triméthoxybenzamide (Composé **29**) ;
- le 4'-fluoro-N-(2-méthyl-5-(3-(3,4,5-triméthoxybenzoyl)guanidino)phényl)biphényl-4-carboxamide (Composé **30**) ;
- le N-(N-(4-chloro-3-(4-phénoxybenzamido)phényl)carbamimidoyl)-3,4,5-triméthoxybenzamide (Composé **32**) ;
- le N-(N-(4-chloro-3-(3-phénylureido)phényl)carbamimidoyl)-3,4,5-triméthoxybenzamide (Composé **38**).

5. Composés de formule **(I)** selon l'une des revendications 1 à 4, **caractérisés en ce qu'**ils se présentent sous forme de sels, de préférence de sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques, tels que l'acide méthane sulfonique et l'acide éthane sulfonique, arylsulfoniques, tels que le benzène sulfonique et le paratoluène sulfonique, ou arylcarboxyliques, et plus préférablement, de sels formés avec l'acide chlorhydrique.

6. Composés de formule **(I)** selon l'une des revendications 1 à 5, pour une utilisation à titre de médicament destiné au traitement des tumeurs associées à une hyperactivation de la voie de signalisation des protéines Hedgehog.

7. Composés de formule **(I)** selon la revendication 6, pour une utilisation à titre de médicament pour le traitement des tumeurs du tissu nerveux (médulloblastomes, tumeurs primitives neuroectodermiques, glioblastomes, méningiomes et oligodendrogliomes), les tumeurs cutanées (carcinomes basocellulaires, trichoépithéliomes), les tumeurs des tissus musculaires et osseux (rhabdomyosarcomes, ostéosarcomes) et les tumeurs d'autres tissus (rein, vessie, prostate, poumon, estomac, pancréas, sein, foie).

8. Composés de formule **(I)** selon l'une des revendications 1 à 5, pour une utilisation à titre de médicament destiné au traitement des pathologies de type neuro-dégénératif.

9. Composés de formule **(I)** selon la revendication 8, pour une utilisation à titre de médicament pour le traitement de la maladie de Parkinson, la Chorée de Huntington, la maladie d'Alzheimer, la sclérose en plaques et la maladie du motoneurone.

10. Composés de formule **(I)** selon l'une des revendications 1 à 5, pour une utilisation à titre de médicament destiné au traitement de maladies liées au développement cérébral (holoprosencéphalie), au traitement d'accidents vasculaires cérébraux et cardiovasculaires, ainsi qu'aux maladies de l'oligodentrocyte et des cellules de Schwann.

11. Composés de formule **(I)** selon la revendication 10, pour une utilisation *in vitro* pour contrôler et moduler le renouvellement des cellules souches humaines ou animales.

12. Composés de formule **(I)** selon l'une des revendications 1 à 5, pour une utilisation à titre de médicament destiné au traitement du diabète.

13. Composés de formule **(I)** selon l'une des revendications 1 à 5, comme marqueurs pour détecter la présence de protéines, telles que les protéines *Smoothened, Patched* (*Patched 1* et *Patched 2*), *Dispatched* (*Dispatched 1* et *Dispatched 2*) et HIP, dans les tissus ou les lignées cellulaires.

14. Composés de formule **(I)** selon l'une des revendications 1 à 5, comme outils de diagnostic pour cribler des protéines, telles que les protéines *Smoothehed, Patched* (*Patched 1* et *Patched 2*), *Dispatched* (*Dispatched 1* et *Dispatched 2*) et HIP, dans les tissus ou les lignées cellulaires.

15. Composition pharmaceutique **caractérisée par le fait qu'**elle comprend, à titre de principe actif, au moins un composé de formule **(I)** tel que défini selon l'une quelconque des revendications 1 à 5, et au moins un excipient pharmaceutiquement acceptable.

16. Composés **caractérisés en ce qu'**ils répondent à la formule **(I)** suivante : 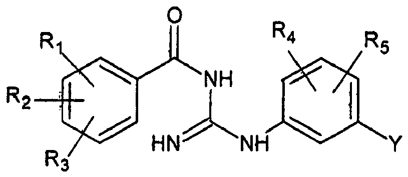 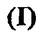 dans laquelle :
- R₁, R₂ et R₃, identiques ou différents et indépendamment les uns des autres, représentent un atome d'hydrogène ou d'halogène, un radical hydroxyle, un groupe alkyle, perfluoroalkyle, alcoxy éventuellement substitué, alkylthio, nitrile, ou un hétérocycle obtenu à partir de deux des R₁, R₂ et R₃ fusionnés avec deux atomes de carbone adjacents du cycle phényle auxquels ils sont liés ;
- Y représente un groupe hétéroaryle mono- ou polycyclique choisi parmi l'indole ou l'imidazole fusionné à un group thiazol, ou Y représente un groupe -NH-(C=O)-R₆, -(C=O)-NH-R₆ ou -NH-(C=O)-NH-R₆, dans lequel R₆ représente un groupe aryle mono- ou polycyclique non substitué ; un groupe aryle comportant un ou plusieurs substituants choisis parmi un atome d'halogène, un radical alkyle, alcoxy ou alcoxyaryle, mono ou dialkylamino, un groupe aryle ou hétéroaryle, un hétérocyclique ; un groupe hétéroaryle mono- ou polycyclique ; un radical alkyle linéaire ou ramifié ; un groupe hydrocarboné mono- ou polycyclique saturé ou insaturé ;
- R₄ et R₅, identiques ou différents et indépendamment l'un de l'autre, représentent un atome d'hydrogène ou d'halogène, un groupe alcoxy, alkylthio, alkyle, perfluoroalkyle, nitrile ou nitro.

17. Composés de formule **(I)** selon la revendication 16, **caractérisés en ce qu'**ils sont choisis parmi ceux dans lesquels :
- R₁, R₂ et R₃, identiques ou différents, représentent un atome d'hydrogène, un radical méthyloxy ou éthyloxy, ou un dioxolane fusionné avec deux atomes de carbone adjacents du cycle phényle auquel ils sont liés ;
- R₄ et R₅, identiques ou différents, représentent un atome d'hydrogène, de chlore, de brome, de fluore, ou un radical méthyle ou méthoxy ; et
- Y représente:
- un groupe hétéroaryle mono- ou polycyclique choisi parmi l'indole ou l'imidazole fusionné à un groupe thiazol, ou
- Y représente un groupe -NH-(C=O)-R₆, -(C=O)-NH-R₆ ou -NH-(C=O)-NH-R₆ dans lequel R₆ représente un groupe phényle, éventuellement substitué par un atome d'halogène, un radical alkyle, diaminoalkyle ou alcoxy, un groupe cycloalkyle, ou un groupe aryle ; un groupe cycloalkyle ; un groupe pyridinyle ; un groupe naphtyle ; un groupe furyle ; un groupe thiophényle ; un radical isopropyle, de préférence R₆ représente un groupe phényle, éventuellement substitué par un atome de chlore, un radical méthoxy, une morpholine, un groupe phényle ou phénoxy ; un groupe cyclohexyle ; un groupe pyridinyle ; un groupe naphtyle ; un groupe furyle ; lorsque ledit groupe Y représente un groupe -NH-(C=O)-R₆, -(C=O)-NH-R₆ ou -NH-(C=O)-NH-R₆

18. Composés de formule **(I)** selon la revendication 16 ou la revendication 17, **caractérisés par le fait qu'**ils sont choisis parmi :
- le 3,4,5-triméthoxy-N-(N-(3-(4-méthoxybenzamido)phényl)carbamimidoyl)benzamide : 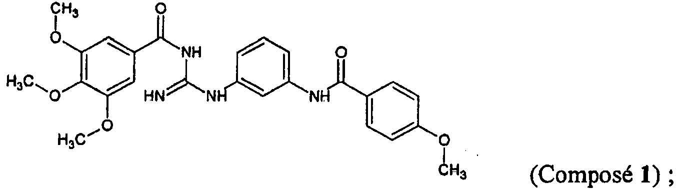
- le N-(N-(3-(2-chlorobenzamido)phényl)carbamimidoyl)-3,4,5-triméthoxybenzamide : 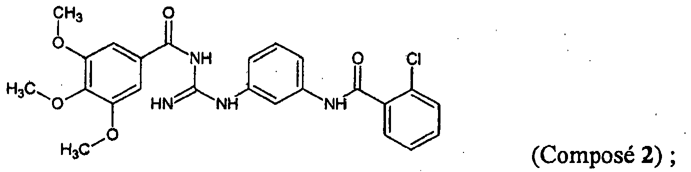
- le 3,4,5-triméthoxy-N-(N-(3-(3-méthoxybenzamido)phényl)carbamimidoyl)benzamide : 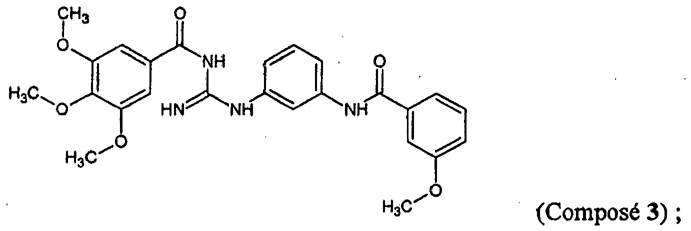
- le N-(3-(3-(3,4,5-triméthoxybenzoyl)guanidino)phényl)biphényl-4-carboxamide : 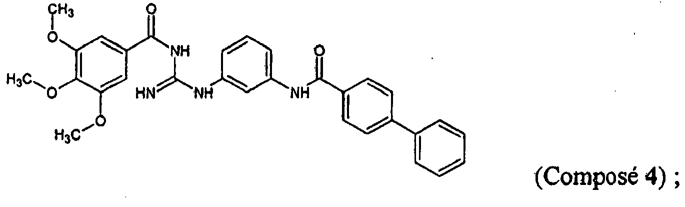
- le N-(N-(3-(cyclohexanecarboxamido)phényl)carbamimidoyl)-3,4,5-triméthoxybenzamide 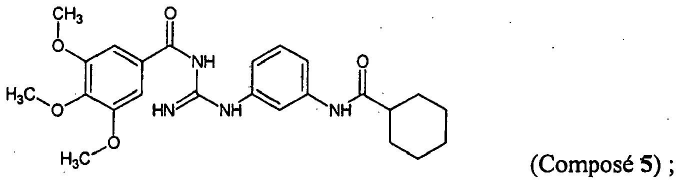
- le N-(3-(3-(3,4,5-triméthoxybenzoyl)guanidino)phényl)-2-naphthamide : 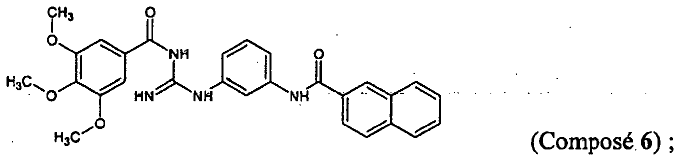
- le N-(3-(3-(3,4,5-triméthoxybenzoyl)guanidino)phényl)isonicotinamide : 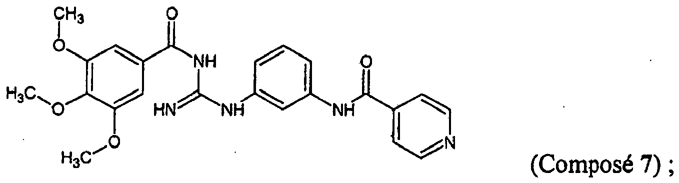
- le N-(3-(3-(3,4,5-triméthoxybenzoyl)guanidino)phényl)furan-2-carboxamide : 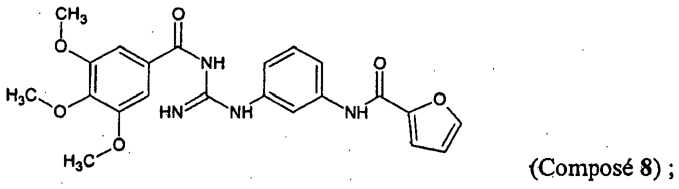
- le N-(N-(3-benzamidophényl)carbamimidoyl)3,5-diméthoxybenzamide : 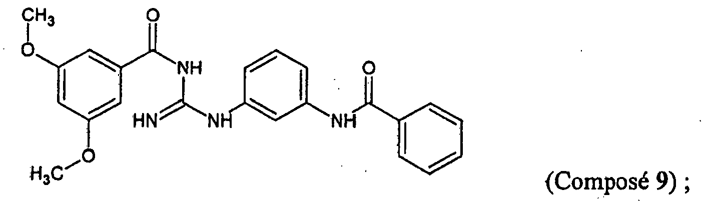
- le N-(N-(3-benzamidophényl)carbamimidoyl)-4-éthoxy-3,5-diméthoxybenzamide : 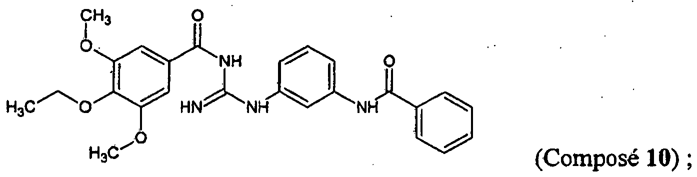
- le N-(N-(3-benzamidophényl)carbamimidoyl)-3,4-diéthoxy-5-méthoxybenzamide : 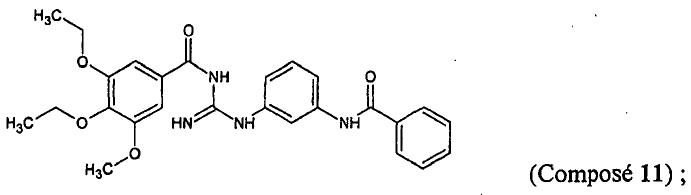
- le N-(N-(3-benzamidophényl)carbamimidoyl)-7-méthoxybenzo[*d*][1,3]dioxole-5-carboxamide : 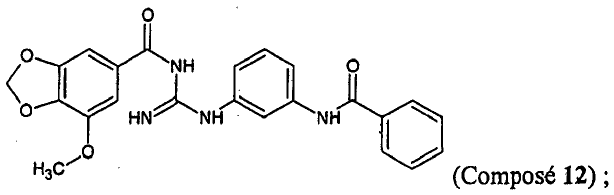
- le N-(N-(3-benzamidophényl)carbamimidoyl)-2,4-diméthoxybenzamide : 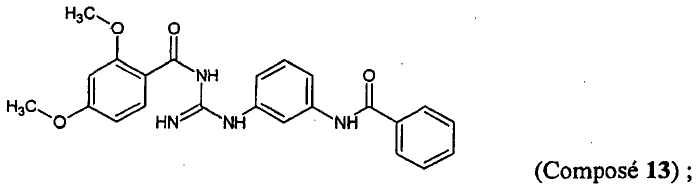
- le N-(N-(3-benzamido-4-fluorophényl)carbamimidoyl)-3,4,5-triméthoxybenzamide : 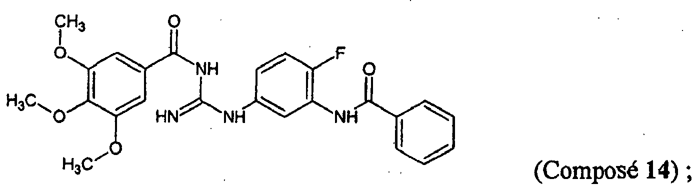
- le N-(N-(3-benzamidophényl)carbamimidoyl)-3,4,5-triméthoxybenzamide : 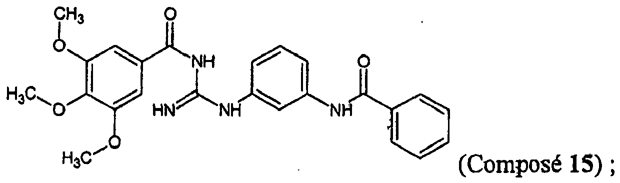
- le N-(N-(3-benzamido-4-chlorophényl)carbamimidoyl)-3,4,5-triméthoxybenzamide : 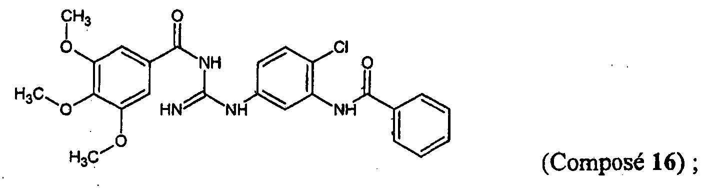
- le N-(2-chloro-5-(3-(3,4,5-triméthoxybenzoyl)guanidino)phényl)biphényl-4-carboxamide : 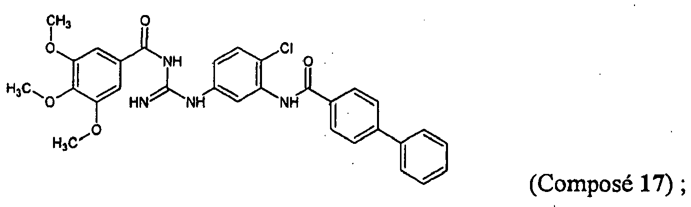
- le 3,4,5-triméthoxy-N-(N-(3-(4-morpholinobenzamido)phényl)carbamimidoyl)benzamide : 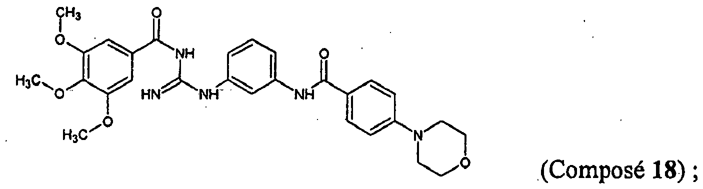
- le N-(N-(3-(1*H*-indol-2-yl)phényl)carbamimidoyl)3,4,5-triméthoxybenzamide : 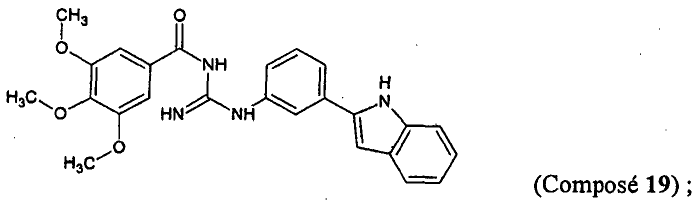
- le N-(N-(4-chloro-3-(4-méthoxyphénylcarbamoyl)phényl)carbamimidoyl)-3,4,5-triméthoxybenzamide : 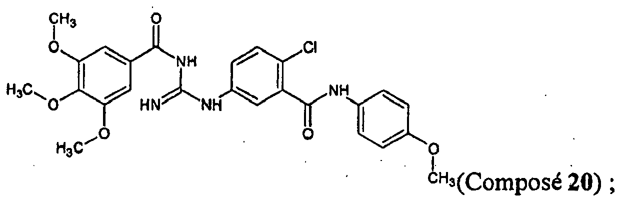
- le N-(N-(4-chloro-3-(phénylcarbamoyl)phényl)carbamimidoyl)-3,4,5-triméthoxybenzamide : 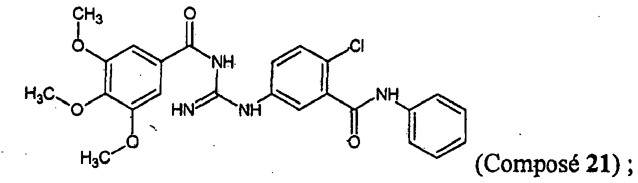
- le 3,4,5-triméthoxy-N-(N-(4-méthyl-3-(phénylcarbamoyl)phényl)carbamimidoyl)benzamide: 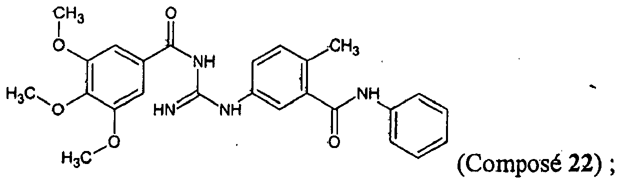
- le 4'-fluoro-N-(3-(3-(3,4,5-triméthoxybenzoyl)guanidino)phényl)biphényl-4-carboxamide : 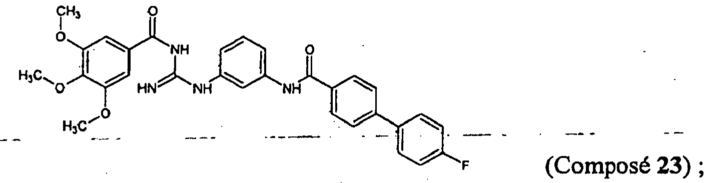
- le N-(2-méthyl-5-(3-(3,4,5-triméthoxybenzoyl)guanidino)phényl)biphényl-4-carboxamide : 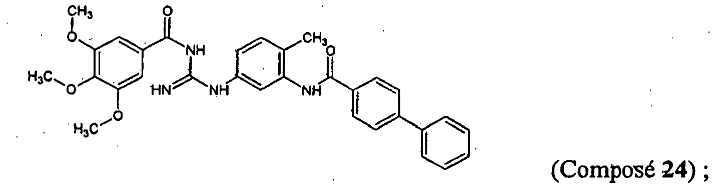
- le N-(2-méthyl-5-(3-(3,4,5-triméthoxybenzoyl)guanidino)phényl)biphényl-3-carboxamide : 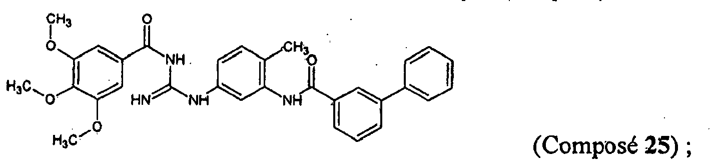
- le N-(2-chloro-5-(3-(3,4,5-triméthoxybenzoyl)guanidino)phényl)biphényl-3-carboxamide : 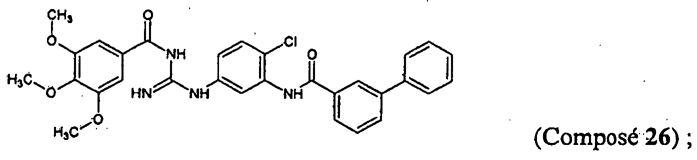
- le N-N-(3-benzamido-4-méthylphényl)carbamimidoyl)-3,4,5-triméthoxybenzamide : 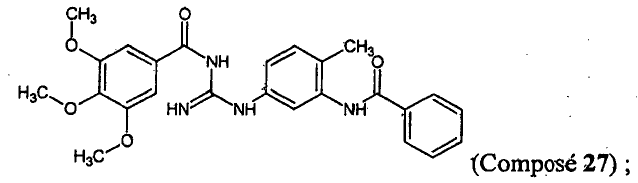
- le N-N-(3-(imidazo[2,1-*b*]thiazol-6-yl)phényl)carbamimidoyl)-3,4,5-triméthoxybenzamide : 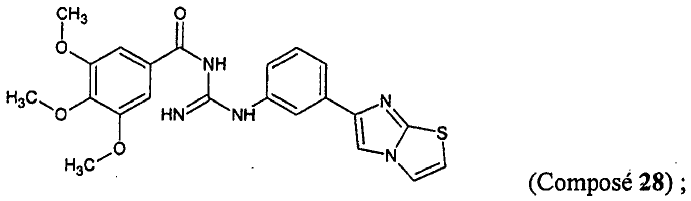
- le N-(N-(3-(1*H*-indol-1-yl)phényl)carbamimidoyl)-3,4,5-triméthoxybenzamide : 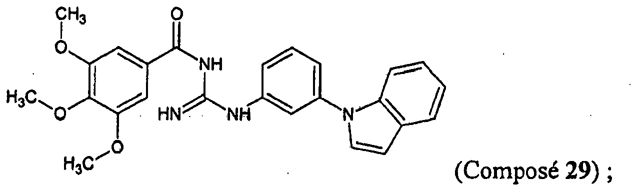
- le 4'-fluoro-N-(2-méthyl-5-(3-(3,4,5-triméthoxybenzoyl)guanidino)phényl)biphényl-4-carboxamide : 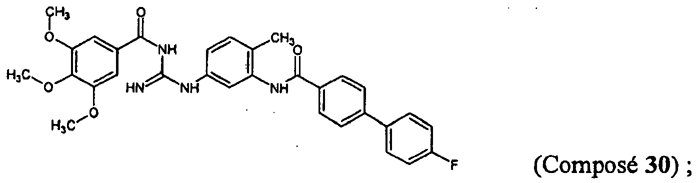
- le 3,4,5-triméthoxy-N-(N-(4-méthyl-3-(4-(pyridin-4-yl)benzamido)phényl)carbamimidoyl)-benzamide : 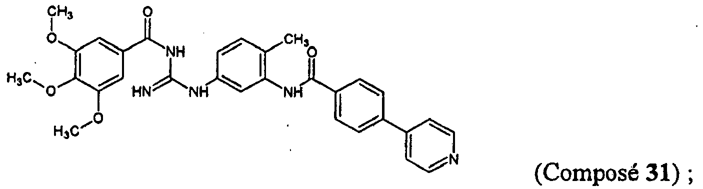
- le N-(N-(4-chloro-3-(4-phénoxybenzamido)phényl)carbamimidoyl)-3,4,5-triméthoxybenzamide : 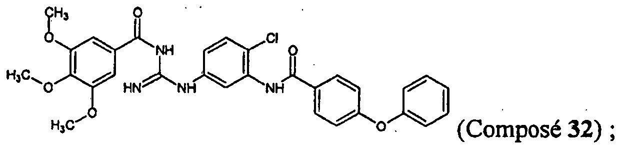
- le N-(3-(3-benzoylguanidino)phényl)biphényl-4-carboxamide : 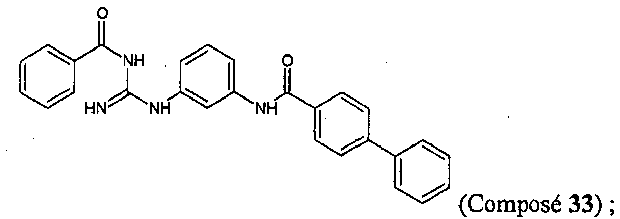
- le N-(2-méthyl-3-(3-(3,4,5-triméthoxybenzoyl)guanidino)phényl)biphényl-4-carboxamide : 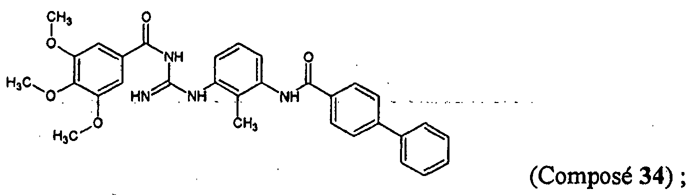
- le N-(4-méthyl-3-(3-(3,4,5-triméthoxybenzoyl)guanidino)phényl)biphényl-4-carboxamide : 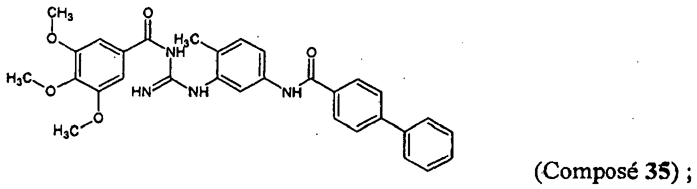
- le N-(N-(3-benzamidophényl)carbamimidoyl)benzamide : 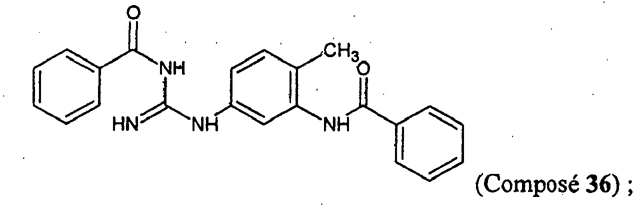
- le N-(N-(3-(3-biphényl-4-ylureido)phényl)carbamimidoyl)-3,4,5-triméthoxybenzamide: 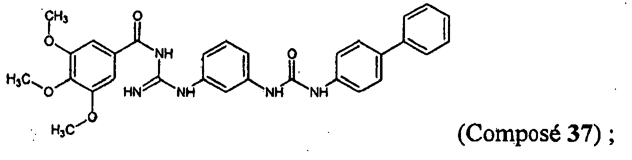
- le N-(N-(4-chloro-3-(3-phénylureido)phényl)carbamimidoyl)-3,4,5-triméthoxybenzamide :

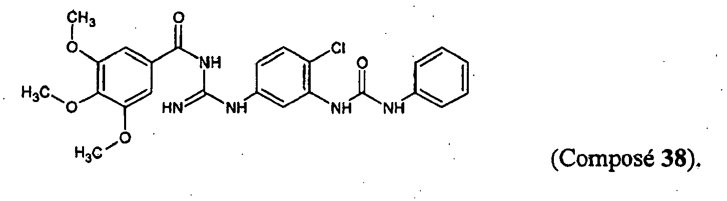

19. Composés selon la revendication 18, **caractérisés par le fait qu'**ils sont choisis parmi :
- le 3,4,5-triméthoxy-N-(N-(3-(4-méthoxybenzamido)phényl)carbamimidoyl)benzamide (Composé **1**) ;
- le N-(N-(3-(2-chlorobenzamido)phényl)carbamimidoyl)-3,4,5-triméthoxybenzamide (Composé **2**) ;
- le 3,4,5-triméthoxy-N-(N-(3-(3-méthoxybenzamido)phényl)carbamimidoyl)benzamide (Composé **3**) ;
- le N-(3-(3-(3,4,5-triméthoxybenzoyl)guanidino)phényl)-2-naphthamide (Composé **6**) ;
- le N-(3-(3-(3,4,5-triméthoxybenzoyl)guanidino)phényl)furan-2-carboxamide (Composé **8**) ;
- le N-(N-(3-benzamidophényl)carbamimidoyl)-3,5-diméthoxybenzamide (Composé **9**) ;
- le N-(N-(3-benzamidophényl)carbamimidoyl)-7-méthoxybenzo[*d*][1,3]dioxole-5-carboxamide (Composé **12**) ;
- le N-(N-(3-benzamidophényl)carbamimidoyl)-2,4-diméthoxybenzamide (Composé **13**) ;
- le N-(N-(3-benzamidophényl)carbamimidoyl)-3,4,5-triméthoxybenzamide (Composé **15**) ;
- le N-(2-chloro-5-(3-(3,4,5-triméthoxybenzoyl)guanidino)phényl)biphényl-4-carboxamide (Composé **17**) ;
- le N-(N-(3-(*1H*-indol-2-yl)phényl)carbamimidoyl)-3,4,5-triméthoxybenzamide (Composé **19**) ;
- le N-(N-(4-chloro-3-(phénylcarbamoyl)phényl)carbamimidoyl)-3,4,5-triméthoxybenzamide (Composé **21**) ;
- le 4'-fluoro-N-(3-(3-(3,4,5-triméthoxybenzoyl)guanidino)phényl)biphényl-4-carboxamide (Composé **23**) ;
- le N-(2-méthyl-5-(3-(3,4,5-triméthoxybenzoyl)guanidino)phényl)biphényl-4-carboxamide (Composé **24**) ;
- le N-(N-(3-(*1H*-indol-1-yl)phényl)carbamimidoyl)-3,4,5-triméthoxybenzamide (Composé **29**) ;
- le 4'-fluoro-N-(2-méthyl-5-(3-(3,4,5-triméthoxybenzoyl)guanidino)phényl)biphényl-4-carboxamide (Composé **30**) ;
- le N-(N-(4-chloro-3-(4-phénoxybenzamido)phényl)carbamimidoyl)-3,4,5-triméthoxybenzamide (Composé **32**) ;
- le N-(N-(4-chloro-3-(3-phénylureido)phényl)carbamimidoyl)-3,4,5- triméthoxybenzamide (Composé **38**).

20. Composés de formule **(I)** selon l'une des revendications 16 à 19, **caractérisés en ce qu'**ils se présentent sous forme de sels, de préférence de sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques, tels, que l'acide méthane sulfonique et l'acide éthane sulfonique, arylsulfoniques, tels que le benzène sulfonique et le paratoluène sulfonique, ou arylcarboxyliques, plus préférablement de sels formés avec l'acide chlorhydrique.

## Patentansprüche

1. Verbindungen, **dadurch gekennzeichnet, dass** sie der folgenden Formel (I) entsprechen: 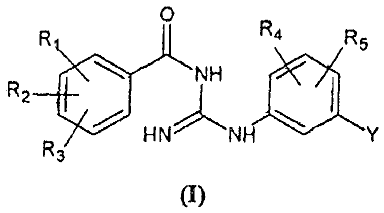 in der:
- R₁, R₂ und R₃, die gleich oder verschieden sind und unabhängig voneinander sind, ein Wasserstoffatom oder Halogenatom, einen Hydroxylrest, eine Alkyl-, Perfluoralkyl-, Alkoxy-Gruppe, gegebenenfalls substituiert, eine Alkylthio-, Nitril-Gruppe oder einen Heterocyclus, der ausgehend von zwei der R₁, R₂ und R₃, die mit zwei benachbarten Kohlenstoffatomen des Phenylrings, an die sie gebunden sind, kondensiert sind, erhalten wird, darstellen;
- Y eine mono- oder polycyclische Heteroarylgruppe, -NH-(C=O)-R₆, -(C=O)-NH-R₆ oder -NH-(C=O)-NH-R₆ darstellt, worin R₆ eine nichtsubstituierte mono- oder polycyclische Arylgruppe; eine Arylgruppe, die einen oder mehrere Substituenten, ausgewählt aus einem Halogenatom, einem Alkyl-, Alkoxy- oder Alkoxyaryl-, Mono- oder Dialkylaminorest, einer Aryl- oder Heteroarylgruppe, einem Heterocyclyl, trägt; eine mono- oder polycyclische Heteroarylgruppe; einen linearen oder verzweigten Alkylrest; eine gesättigte oder ungesättigte mono- oder polycyclische Kohlenwasserstoffgruppe darstellt;
- R₄ und R₅, die gleich oder verschieden und unabhängig voneinander sind, ein Wasserstoffatom oder Halogenatom, eine Alkoxy-, Alkylthio-, Alkyl-, Perfluoralkyl-, Nitril- oder Nitrogruppe darstellen,
als Medikamente.

2. Verbindungen der Formel (I) gemäß Anspruch 1, **dadurch gekennzeichnet, dass** sie ausgewählt sind aus denen, in denen:
- R₁, R₂ und R₃, die gleich oder verschieden sind, ein Wasserstoffatom, einen Methyloxy- oder Ethyloxyrest oder ein Dioxolan, kondensiert mit zwei benachbarten Kohlenstoffatomen des Phenylrings, an das sie gebunden sind, darstellen;
- R₄ und R₅, die gleich oder verschieden sind, ein Wasserstoffatom, Chlor-, Brom-, Fluoratom oder einen Methyl- oder Methoxyrest darstellen und
- Y darstellt:
- eine mono- oder polycyclische Heteroarylgruppe, die vorzugsweise aus Indol oder Imidazol, kondensiert an eine Thiazolgruppe, ausgewählt ist, oder
- eine Gruppe -NH-(C=O)-R₆, -(C=O)-NH-R₆ oder -NH-(C=O)-NH-R₆, in der R₆ eine Phenylgruppe, gegebenenfalls substituiert mit einem Halogenatom, einem Alkyl-, Diaminoalkyl- oder Alkoxyrest, einer Cycloalkylgruppe oder einer Arylgruppe; eine Cycloalkylgruppe; eine Pyridinylgruppe; eine Naphthylgruppe; eine Furylgruppe; eine Thiophenylgruppe; einen Isopropylrest darstellt, R₆ vorzugsweise eine Phenylgruppe, gegebenenfalls substituiert mit einem Chloratom, einem Methoxyrest, einem Morpholin, einer Phenyl- oder Phenoxygruppe, eine Cyclohexylgruppe; eine Pyridinylgruppe; eine Naphthylgruppe; eine Furylgruppe darstellt.

3. Verbindungen der Formel (I) gemäß Anspruch 1 oder Anspruch 2, **dadurch gekennzeichnet, dass** sie ausgewählt sind aus:
- 3,4,5-Trimethoxy-N-(N-(3-(4-methoxybenzamido)phenylcarbamimidoyl)benzamid: 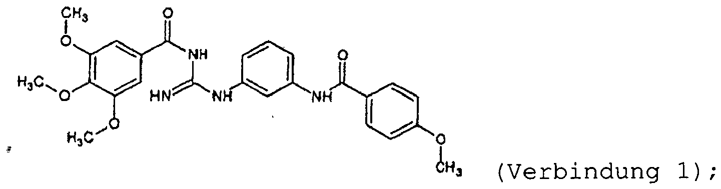
- N-(N-(3-(2-Chlorbenzamido)phenyl)carbamimidoyl)-3,4,5-trimethoxybenzamid: 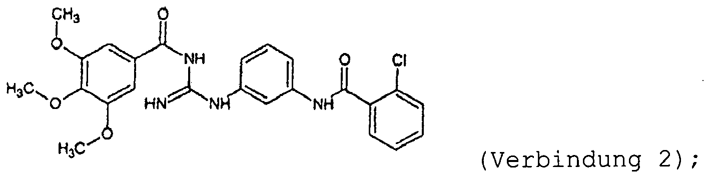
- 3,4,5-Trimethoxy-N-(N-(3-(3-methoxybenzamido)phenyl)carbamimidoyl)benzamid: 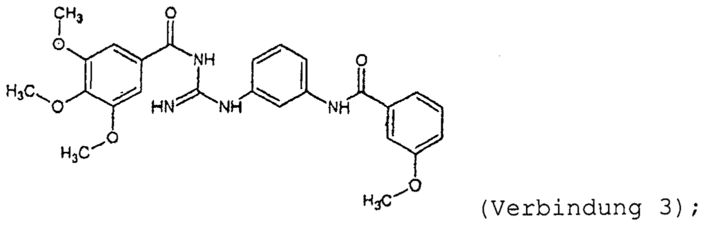
- N-(3-(3-(3,4,5-Trimethoxybenzoyl)guanidino)phenyl)biphenyl-4-carboxamid: 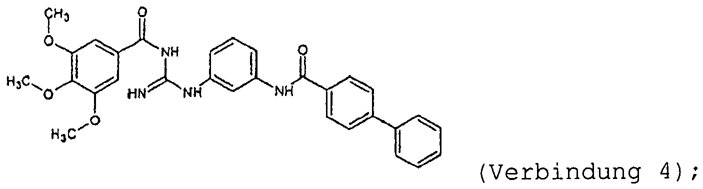
- N-(N-(3-(Cyclohexancarboxamido)phenyl)carbamimidoyl)-3,4,5-trimethoxybenzamid: 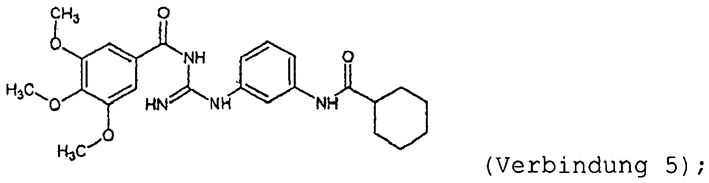
- N-(3-(3-(3,4,5-Trimethoxybenzoyl)guanidino)phenyl)-2-naphthamid: 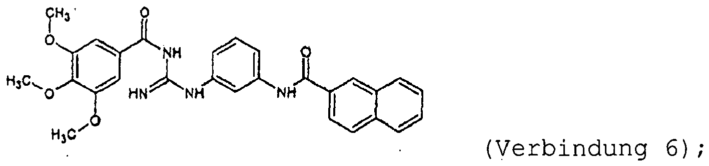
- N-(3-(3-(3,4,5-Trimethoxybenzoyl)guanidino)phenyl)isonicotinamid: 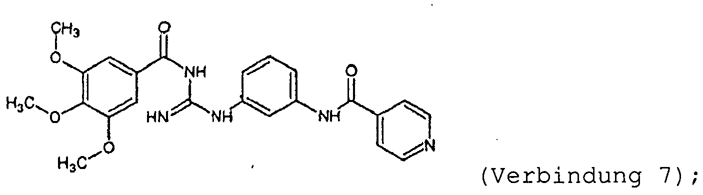
- N-(3-(3-(3,4,5-Trimethoxybenzoyl)guanidino)phenyl)furan-2-carboxamid: 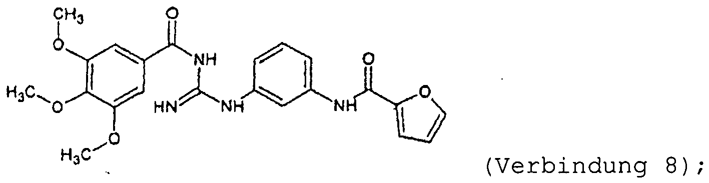
- N-(N-(3-Benzamidophenyl)carbamimidoyl)-3,5-dimethoxybenzamid: 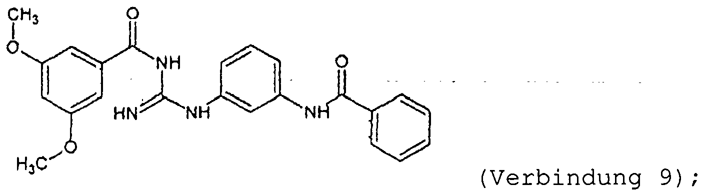
- N-(N-(3-Benzamidophenyl)carbamimidoyl)-4-ethoxy-3,5-dimethoxybenzamid: 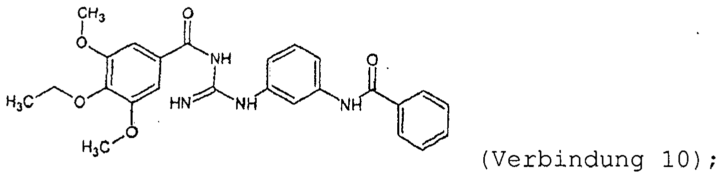
- N-(N-(3-Benzamidophenyl)carbamimidoyl)-3,4-diethoxy-5-methoxybenzamid: 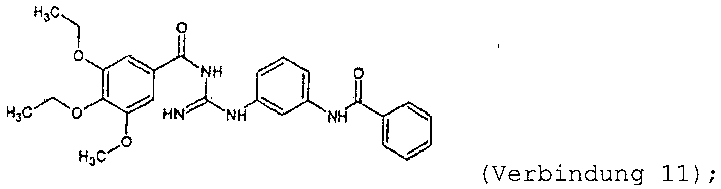
- N-(N-(3-Benzamidophenyl)carbamimidoyl)-7-methoxybenzo[d][1,3]dioxol-5-carboxamid: 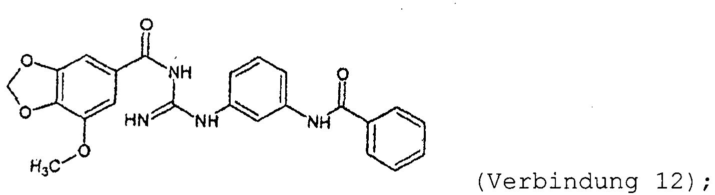
- N-(N-(3-Benzamidophenyl)carbamimidoyl)-2,4-dimethoxybenzamid: 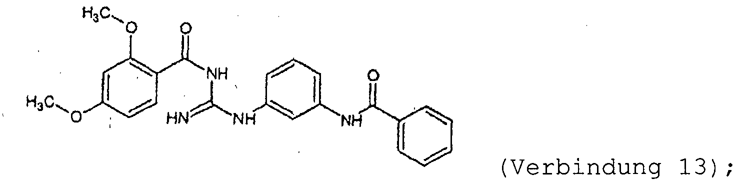
- N-(N-(3-Benzamido-4-fluorphenyl)carbamimidoyl)-3,4,5-trimethoxybenzamid: 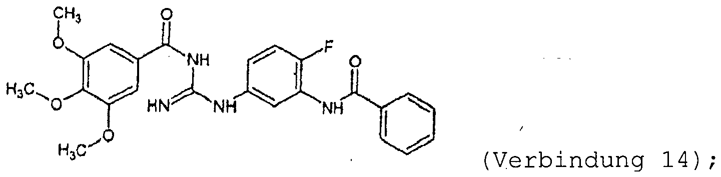
- N-(N-(3-Benzamidophenyl)carbamimidoyl)-3,4,5-trimethoxybenzamid: 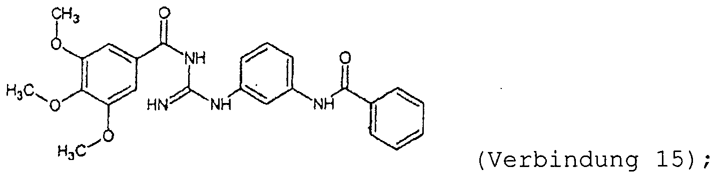
- N-(N-(3-Benzamido-4-chlorphenyl)carbamimidoyl)-3,4,5-trimethoxybenzamid: 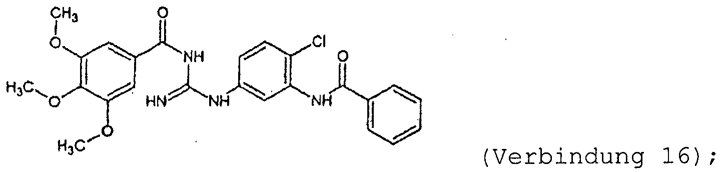
- N-(2-Chlor-5-(3-(3,4,5-trimethoxybenzoyl)guanidino)phenyl)biphenyl-4-carboxamid: 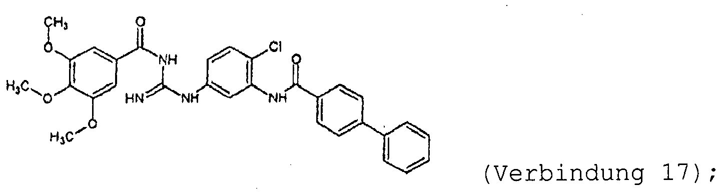
- 3,4,5-Trimethoxy-N-(N-(3-(4-morpholinobenzamido)phenyl)carbamimidoyl)benzamid: 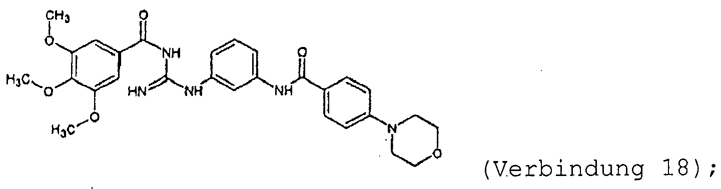
- N-(N-(3-(1H-Indol-2-yl)phenyl)carbamimidoyl)-3,4,5-trimethoxybenzamid: 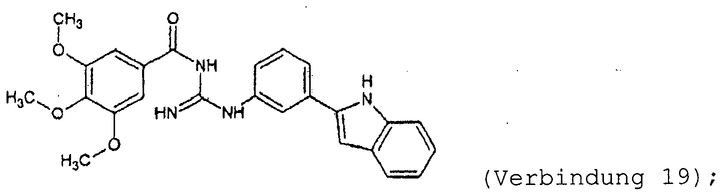
- N-(N-(4-Chlor-3-(4-methoxyphenylcarbamoyl)phenyl)carbamimidoyl)-3,4,5-trimethoxybenzamid: 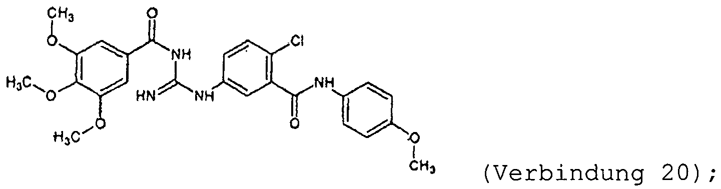
- N-(N-4-Chlor-3-(phenylcarbamoyl)phenyl)carbamimidoyl)-3,4,5-trimethoxybenzamid: 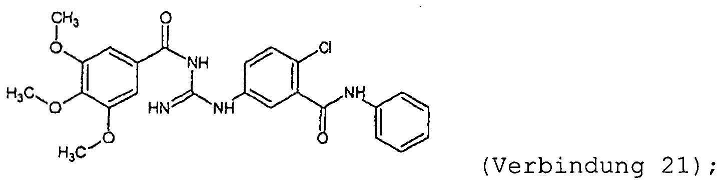
- 3,4,5-Trimethoxy-N-(N-(4-methyl-3-(phenylcarbamoyl)phenyl)carbamimidoyl)benzamid: 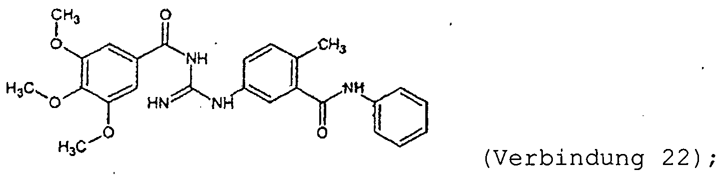
- 4'-Fluor-N-(3-(3-(3,4,5-trimethoxybenzoyl)guanidino)phenyl)biphenyl-4-carboxamid: 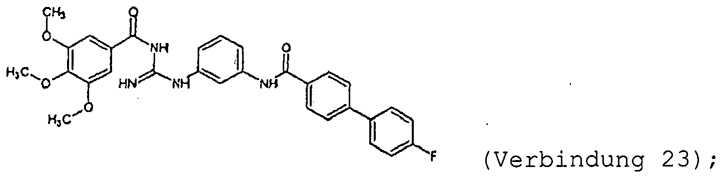
- N-(2-Methyl-5-(3-(3,4,5-trimethoxybenzoyl)guanidino)phenyl)biphenyl-4-carboxamid: 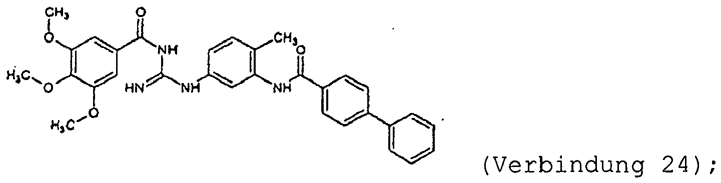
- N-(2-Methyl-5-(3-(3,4,5-trimethoxybenzoyl)guanidino)phenyl)biphenyl-3-carboxamid: 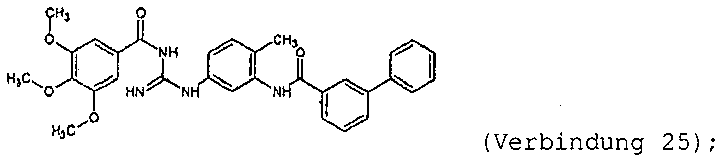
- N-(2-Chlor-5-(3-(3,4,5-trimethoxybenzoyl)guanidino)phenyl)biphenyl-3-carboxamid: 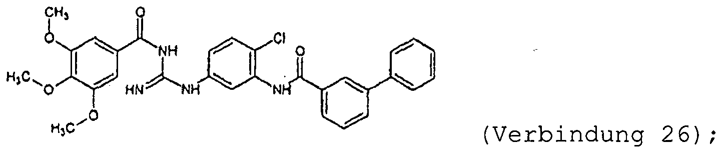
- N-N-(3-Benzamido-4-methylphenyl)carbamimidoyl)-3,4,5-trimethoxybenzamid: 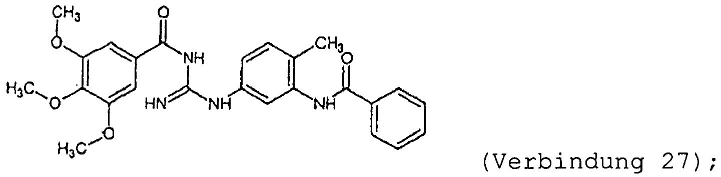
- N-N-(3-(Imidazo[2,1-b]thiazol-6-yl)phenyl)carbamimidoyl)-3,4,5-trimethoxybenzamid: 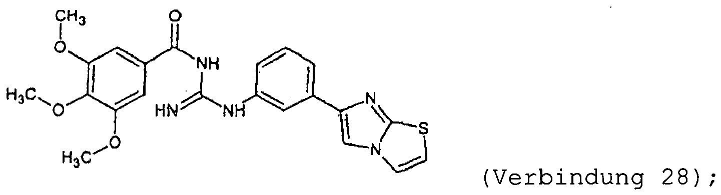
- N-(N-(3-(1H-Indol-1-yl)phenyl)carbamimidoyl)-3,4,5-trimethoxybenzamid: 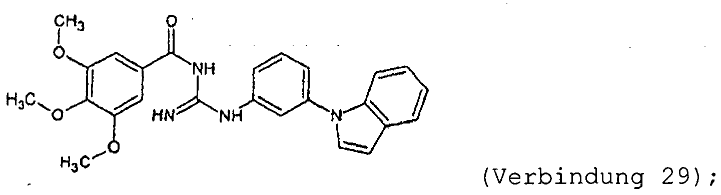
- 4'-Fluor-N-(2-methyl-5-(3-(3,4,5-trimethoxybenzoyl)guanidino)phenyl)biphenyl-4-carboxamid: 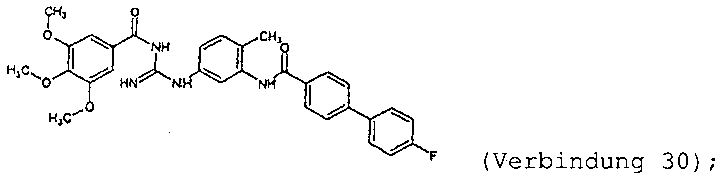
- 3,4,5-Trimethoxy-N-(N-(4-methyl-3-(4-(pyridin-4-yl)benzamido)phenyl)carbamimidoyl)benzamid: 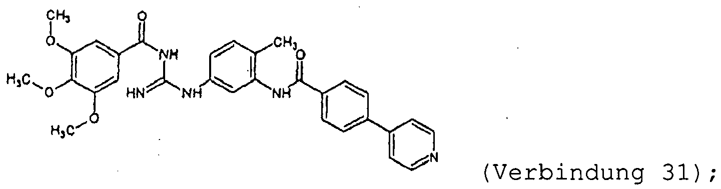
- N-(N-(4-Chlor-3-(4-phenoxybenzamido)phenyl)carbamimidoyl)-3,4,5-trimethoxybenzamid: 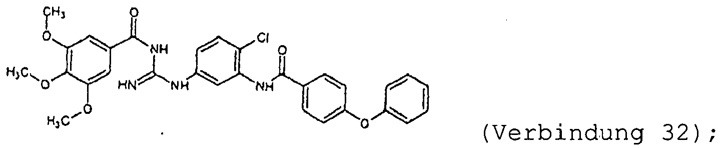
- N-(3-(3-Benzoylguanidino)phenyl)biphenyl-4-carboxamid: 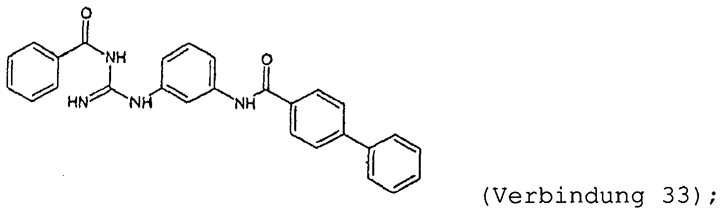
- N-(2-Methyl-3-(3-(3,4,5-trimethoxybenzoyl)guanidino)phenyl)biphenyl-4-carboxamid: 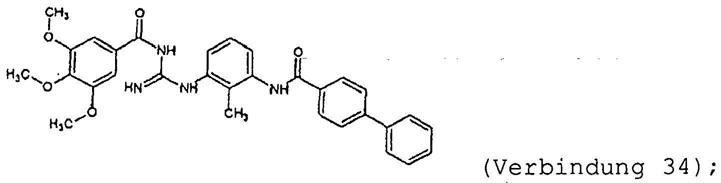
- N-(4-Methyl-3-(3-(3,4,5-trimethoxybenzoyl)guanidino)phenyl)biphenyl-4-carboxamid: 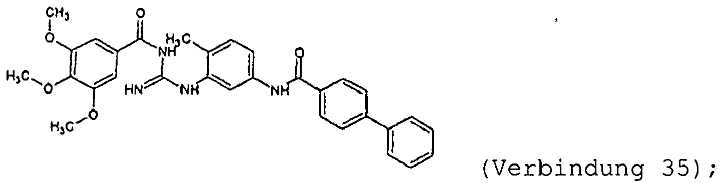
- N-(N-(3-Benzamidophenyl)carbamimidoyl)benzamid: 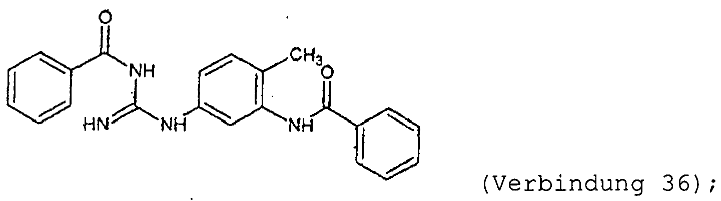
- N-(N-(3-(3-Biphenyl-4-ylureido)phenyl)carbamimidoyl)-3,4,5-trimethoxybenzamid: 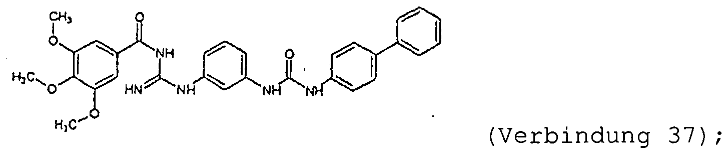
- N-(N-(4-Chlor-3-(3-phenylureido)phenyl)carbamimidoyl)-3,4,5-trimethoxybenzamid:

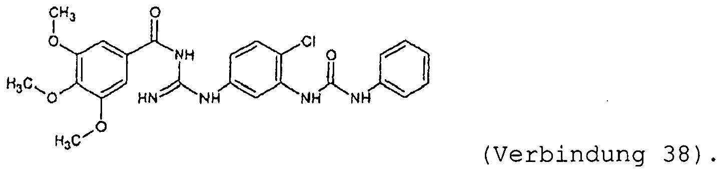

4. Verbindungen gemäß Anspruch 3, **dadurch gekennzeichnet, dass** sie ausgewählt sind aus:
- 3,4,5-Trimethoxy-N-(N-(3-(4-methoxybenzamido)phenyl)carbamimidoyl)benzamid (Verbindung 1);
- N-(N-(3-(2-Chlorbenzamido)phenyl)carbamimidoyl)-3,4,5-trimethoxybenzamid (Verbindung 2);
- 3,4,5-Trimethoxy-N-(N-(3-(3-methoxybenzamido)phenyl)carbamimidoyl)benzamid (Verbindung 3);
- N-(3-(3-(3,4,5-Trimethoxybenzoyl)guanidino)phenyl)-2-naphthamid (Verbindung 6);
- N-(3-(3-(3,4,5-Trimethoxybenzoyl)guanidino)phenyl)furan-2-carboxamid (Verbindung 8);
- N-(N-(3-Benzamidophenyl)carbamimidoyl)-3,5-dimethoxybenzamid (Verbindung 9);
- N-(N-(3-Benzamidophenyl)carbamimidoyl)-7-methoxybenzo[d][1,3]dioxol-5-carboxamid (Verbindung 12);
- N-(N-(3-Benzamidophenyl)carbamimidoyl)-2,4-dimethoxybenzamid (Verbindung 13);
- N-(N-(3-Benzamidophenyl)carbamimidoyl)-3,4,5-trimethoxybenzamid (Verbindung 15);
- N-(2-Chlor-5-(3-(3,4,5-trimethoxybenzoyl)guanidino)phenyl)biphenyl-4-carboxamid (Verbindung 17);
- N-(N-(3-(1H-Indol-2-yl)phenyl)carbamimidoyl)-3,4,5-trimethoxybenzamid (Verbindung 19);
- N-(N-(4-Chlor-3-(phenylcarbamoyl)phenyl)carbamimidoyl)-3,4,5-trimethoxybenzamid (Verbindung 21);
- 4'-Fluor-N-(3-(3-(3,4,5-trimethoxybenzoyl)guanidino)phenyl)biphenyl-4-carboxamid (Verbindung 23);
- N-(2-Methyl-5-(3-(3,4,5-trimethoxybenzoyl)guanidino)phenyl)biphenyl-4-carboxamid (Verbindung 24);
- N-(N-(3-(1H-Indol-1-yl)phenyl)carbamimidoyl)-3,4,5-trimethoxybenzamid (Verbindung 29);
- 4'-Fluor-N-(2-methyl-5-(3-(3,4,5-trimethoxybenzoyl)guanidino)phenyl)biphenyl-4-carboxamid (Verbindung 30);
- N-(N-(4-Chlor-3-(4-phenoxybenzamido)phenyl)carbamimidoyl)-3,4,5-trimethoxybenzamid (Verbindung 32);
- N-(N-(4-Chlor-3-(3-phenylureido)phenyl)carbamimidoyl)-3,4,5-trimethoxybenzamid (Verbindung 38).

5. Verbindungen der Formel (I) gemäß einem der Ansprüche 1 bis 4, **dadurch gekennzeichnet dass** sie in Form von Salzen, vorzugsweise von Salzen, die mit Chlorwasserstoffsäure, Bromwasserstoffsäure, Salpetersäure, Schwefelsäure, Phosphorsäure, Essigsäure, Ameisensäure, Propionsäure, Benzoesäure, Maleinsäure, Fumarsäure, Bernsteinsäure, Weinsäure, Citronensäure, Oxalsäure, Glyoxylsäure, Asparaginsäure, Alkansulfonsäuren, wie Methansulfonsäure und Ethansulfonsäure, Arylsulfonsäuren, wie Benzolsulfonsäure und para-Toluolsulfonsäure, oder Arylcarbonsäuren gebildet sind, und bevorzugter Salzen, die mit Chlorwasserstoffsäure gebildet sind, vorliegen.

6. Verbindungen der Formel (I) gemäß einem der Ansprüche 1 bis 5 zur Verwendung als Medikament, das zur Behandlung von Tumoren, die mit einer Hyperaktivierung des Signalübertragungswegs der Hedgehog-Proteine assoziiert sind, bestimmt ist.

7. Verbindungen der Formel (I) gemäß Anspruch 6 zur Verwendung als Medikament für die Behandlung von Tumoren des Nervengewebes (Medulloblastome, neuroektodermale primitive Tumore, Glioblastome, Meningiome und Oligodendrogliome), Hauttumoren (Basalzellkarzinome, Trichoepitheliome), Tumoren der Muskel- und Knochengewebe (Rhabdomyosarkome, Osteosarkome) und Tumoren anderer Gewebe (Niere, Gefäße, Prostata, Lunge, Magen, Pankreas, Brust, Leber).

8. Verbindungen der Formel (I) gemäß einem der Ansprüche 1 bis 5 für eine Verwendung als Medikament, das für die Behandlung von Pathologien des neurodegenerativen Typs bestimmt ist.

9. Verbindungen der Formel (I) gemäß Anspruch 8 zur Verwendung als Medikament für die Behandlung der Parkinson'schen Krankheit, von Huntington-Chorea, der Alzheimer'schen Krankheit, von multipler Sklerose und der Motoneuronenerkrankung.

10. Verbindungen der Formel (I) gemäß einem der Ansprüche 1 bis 5 zur Verwendung als Medikament, das für die Behandlung von Krankheiten, die mit zerebraler Entwicklung verbunden sind (Holoprosenzephalie), für die Behandlung von zerebrovaskulären und kardiovaskulären Ereignissen sowie für Krankheiten der Oligodendrozyten und der Schwannsche Zellen bestimmt ist.

11. Verbindungen der Formel (I) gemäß Anspruch 10 zur *In-vitro*-Verwendung, um die Erneuerung der humanen oder titerischen Stammzellen zu regulieren und zu modulieren.

12. Verbindungen der Formel (I) gemäß einem der Ansprüche 1 bis 5 zur Verwendung als Medikament, das für die Behandlung von Diabetes bestimmt ist.

13. Verbindungen der Formel (I) gemäß einem der Ansprüche 1 bis 5 als Marker, um das Vorliegen von Proteinen, wie zum Beispiel den Proteinen *Smoothened, Patched (Patched 1* und *Patched 2)*, *Dispatched (Dispatched 1* und *Dispatched 2)* und HIP, in Geweben oder Zelllinien zu detektieren.

14. Verbindungen der Formel (I) gemäß einem der Ansprüche 1 bis 5 als Diagnosewerkzeuge, um Proteine, wie zum Beispiel die Proteine *Smoothened, Patched (Patched 1* und *Patched 2)*, *Dispatched (Dispatched 1* und *Dispatched 2)* und HIP, in Geweben oder Zelllinien durchzumustern.

15. Pharmazeutische Zusammensetzung, **dadurch gekennzeichnet, dass** sie als Wirkstoff wenigstens eine Verbindung der Formel (I), wie in einem der Ansprüche 1 bis 5 definiert, und wenigstens ein pharmazeutisch annehmbares Exzipiens umfasst.

16. Verbindungen, **dadurch gekennzeichnet, dass** sie der folgenden Formel (I) entsprechen: 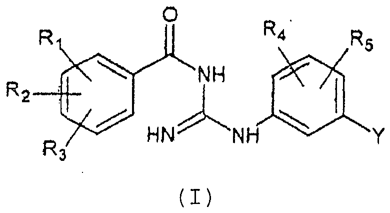 in der:
- R₁, R₂ und R₃, die gleich oder verschieden und voneinander unabhängig sind, ein Wasserstoffatom oder Halogenatom, einen Hydroxylrest, eine Alkyl-, Perfluoralkyl-, Alkoxygruppe, gegebenenfalls substituiert, eine Alkylthio-, Nitrilgruppe oder ein Heterocyclyl, erhalten ausgehend von zwei der R₁, R₂ und R₃, die mit zwei benachbarten Kohlenstoffatomen des Phenylrings, an die sie gebunden sind, kondensiert sind, darstellen;
- Y eine mono- oder polycyclische Heteroarylgruppe, ausgewählt aus Indol oder Imidazol, kondensiert an eine Thiazolgruppe, darstellt oder Y eine Gruppe -NH-(C=O)-R₆,
- (C=O)-NH-R₆ oder -NH-(C=O)-NH-R₆ darstellt, in der R₆ eine nichtsubstituierte mono- oder polycyclische Arylgruppe; eine Arylgruppe, die einen oder mehrere Substituenten, ausgewählt aus einem Halogenatom, einem Alkyl-, Alkoxy- oder Alkoxyaryl-, Mono- oder Dialkylaminorest, einer Aryl- oder Heteroarylgruppe, einem Heterocyclyl, trägt; eine mono- oder polycyclische Heteroarylgruppe; einen linearen oder verzweigten Alkylrest; eine gesättigte oder ungesättigte mono- oder polycyclische Kohlenwasserstoffgruppe darstellt;
- R₄ und R₅, die gleich oder verschieden und voneinander unabhängig sind, ein Wasserstoffatom oder Halogenatom, eine Alkoxy-, Alkylthio-, Alkyl-, Perfluoralkyl-, Nitril- oder Nitrogruppe darstellen.

17. Verbindungen der Formel (I) gemäß Anspruch 16, **dadurch gekennzeichnet, dass** sie aus denen ausgewählt sind, in denen:
- R₁, R₂ und R₃, die gleich oder verschieden sind, ein Wasserstoffatom, einen Methyloxy- oder Ethyloxyrest oder ein Dioxolan, kondensiert mit zwei benachbarten Kohlenstoffatomen des Phenylrings, an die sie gebunden sind, darstellen;
- R₄ und R₅, die gleich oder verschieden sind, ein Wasserstoffatom, ein Chlor-, Brom-, Fluoratom oder einen Methyl- oder Methoxyrest darstellen und
- Y darstellt:
- eine mono- oder polycyclische Heteroarylgruppe, ausgewählt aus Indol oder Imidazol, kondensiert an eine Thiazolgruppe, oder
- Y eine Gruppe -NH-(C=O)-R₆, -(C=O)-NH-R₆ oder -NH-(C=O)-NH-R₆ darstellt, in der R₆ eine Phenylgruppe, gegebenenfalls substituiert mit einem Halogenatom, einem Alkyl-, Diaminoalkyl- oder Alkoxyrest, einer Cycloalkylgruppe oder einer Arylgruppe; eine Cycloalkylgruppe; eine Pyridinylgruppe; eine Naphthylgruppe; eine Furylgruppe; eine Thiophenylgruppe; einen Isopropylrest darstellt, R₆ vorzugsweise eine Phenylgruppe, gegebenenfalls substituiert mit einem Chloratom, einem Methoxyrest, einem Morpholin, einer Phenyl- oder Phenoxygruppe; eine Cyclohexylgruppe; eine Pyridinylgruppe; eine Naphthylgruppe; eine Furylgruppe darstellt, wenn die Gruppe. Y eine Gruppe -NH-(C=O)-R₆,-(C=O)-NH-R₆ oder -NH-(C=O)-NH-R₆ darstellt.

18. Verbindungen der Formel (I) gemäß Anspruch 16 oder Anspruch 17, **dadurch gekennzeichnet , dass** sie ausgewählt sind aus:
- 3,4,5-Trimethoxy-N-(N-(3-(4-methoxybenzamido)phenylcarbamimidoyl)benzamid: 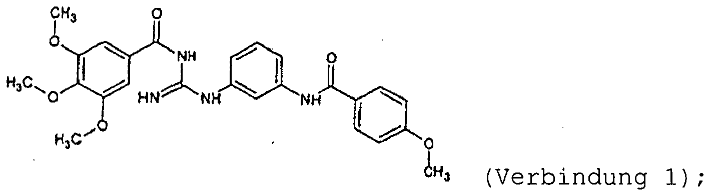
- N-(N-(3-(2-Chlorbenzamido)phenyl)carbamimidoyl)-3,4,5-trimethoxybenzamid: 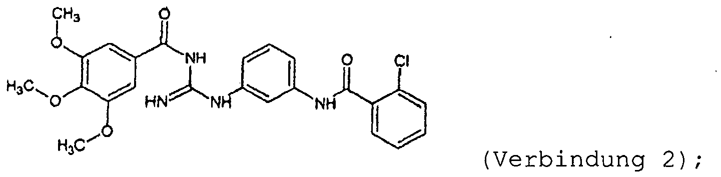
- 3,4,5-Trimethoxy-N-(N-(3-(3-methoxybenzamido)phenyl)carbamimidoyl)benzamid: 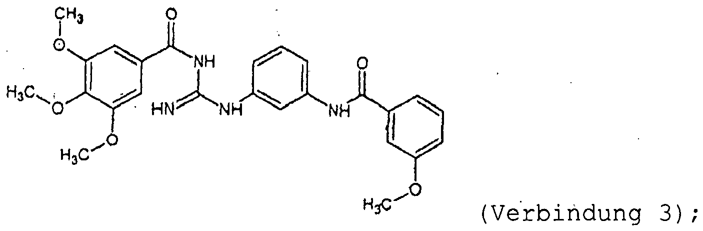
- N-(3-(3-(3,4,5-Trimethoxybenzoyl)guanidino)phenyl)biphenyl-4-carboxamid: 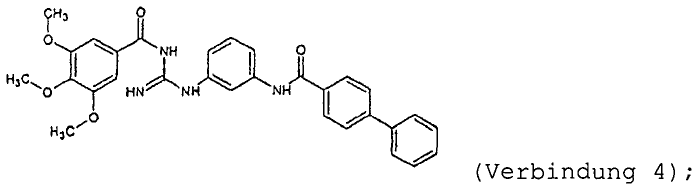
- N-(N-(3-(Cyclohexancarboxamido)phenyl)carbamimidoyl)-3,4,5-trimethoxybenzamid: 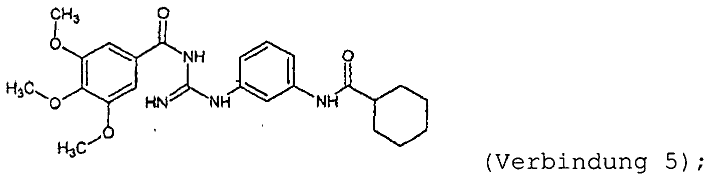
- N-(3-(3-(3,4,5-Trimethoxybenzoyl)guanidino)phenyl)-2-naphthamid: 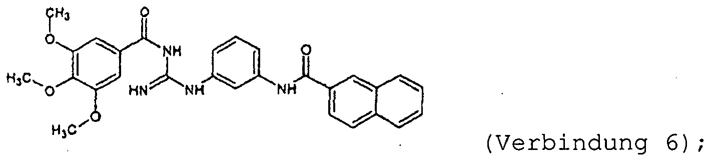
- N-(3-(3-(3,4,5-Trimethoxybenzoyl)guanidino)phenyl)isonicotinamid: 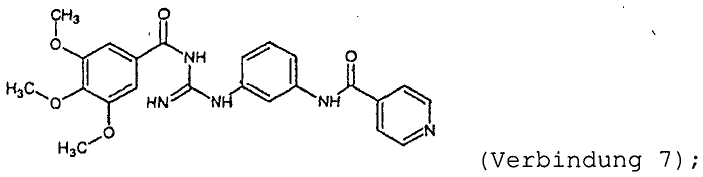
- N-(3-(3-(3,4,5-Trimethoxybenzoyl)guanidino)phenyl)furan-2-carboxamid: 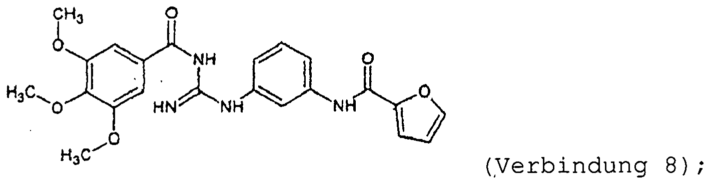
- N-(N-(3-Benzamidophenyl)carbamimidoyl)-3,5-dimethoxybenzamid: 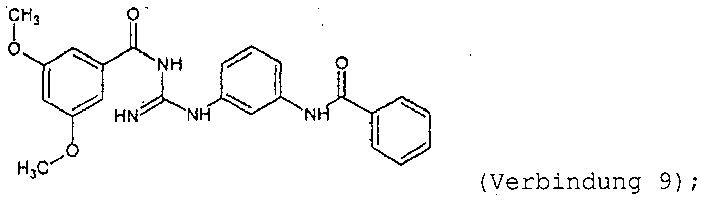
- N-(N-(3-Benzamidophenyl)carbamimidoyl)-4-ethoxy-3,5-dimethoxybenzamid: 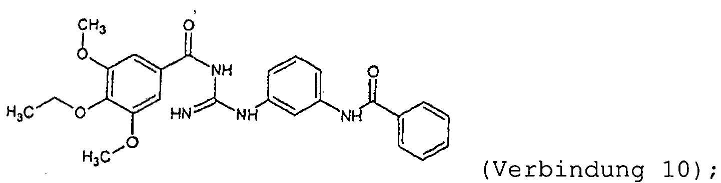
- N-(N-(3-Benzamidophenyl)carbamimidoyl)-3,4-diethoxy-5-methoxybenzamid: 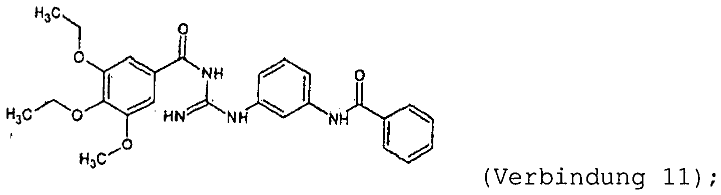
- N-(N-(3-Benzamidophenyl)carbamimidoyl)-7-methoxybenzo[d][1,3]dioxol-5-carboxamid: 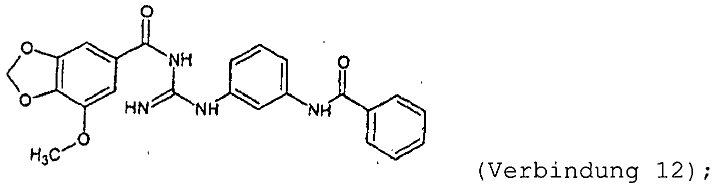
- N-(N-(3-Benzamidophenyl)carbamimidoyl)-2,4-dimethoxybenzamid: 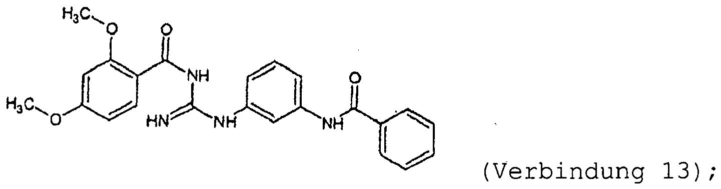
- N-(N-(3-Benzamido-4-fluorphenyl)carbamimidoyl)-3,4,5-trimethoxybenzamid: 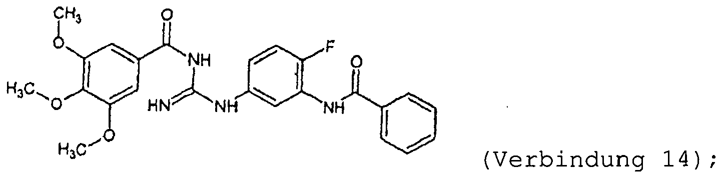
- N-(N-(3-Benzamidophenyl)carbamimidoyl)-3,4,5-trimethoxybenzamid: 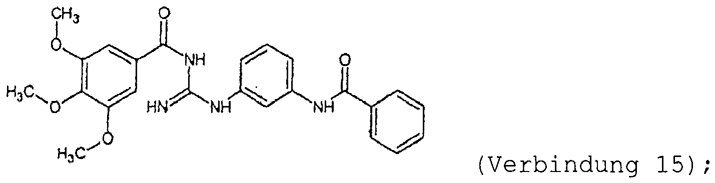
- N-(N-(3-Benzamido-4-chlorphenyl)carbamimidoyl)-3,4,5-trimethoxybenzamid: 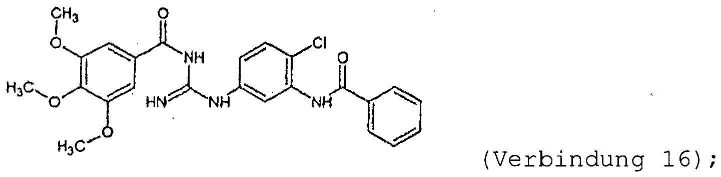
- N-(2-Chlor-5-(3-(3,4,5-trimethoxybenzoyl)guanidino)phenyl)biphenyl-4-carboxamid: 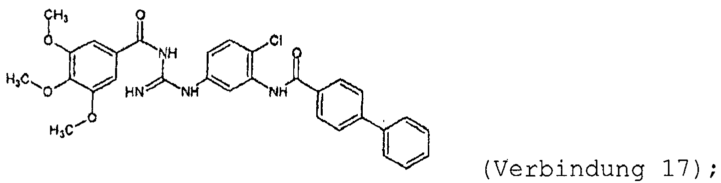
- 3,4,5-Trimethoxy-N-(N-(3-(4-morpholinobenzamido)phenyl)carbamimidoyl)benzamid: 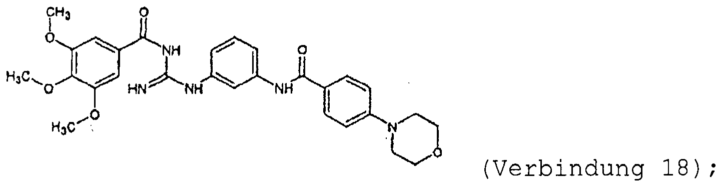
- N-(N-(3-(1H-Indol-2-yl)phenyl)carbamimidoyl)-3,4,5-trimethoxybenzamid: 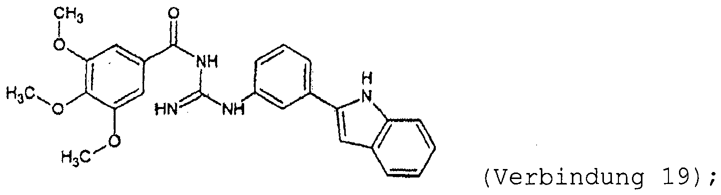
- N-(N-(4-Chlor-3-(4-methoxyphenylcarbamoyl)phenyl)carbamimidoyl)-3,4,5-trimethoxybenzamid: 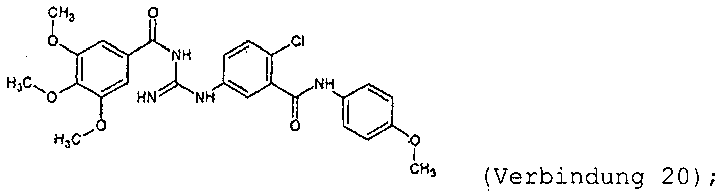
- N-(N-4-Chlor-3-(phenylcarbamoyl)phenyl)carbamimidoyl)-3,4,5-trimethoxybenzamid: 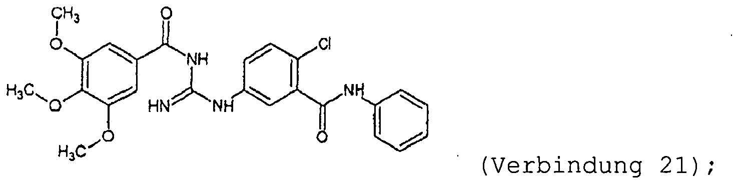
- 3,4,5-Trimethoxy-N-(N-(4-methyl-3-(phenylcarbamoyl)phenyl)carbamimidoyl)benzamid: 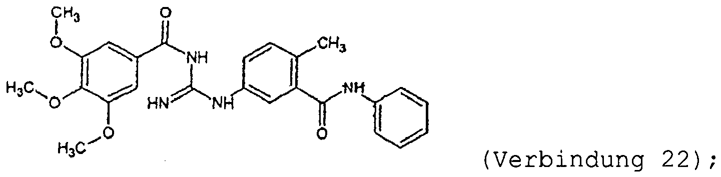
- 4'-Fluor-N-(3-(3-(3,4,5-trimethoxybenzoyl)guanidino)phenyl)biphenyl-4-carboxamid: 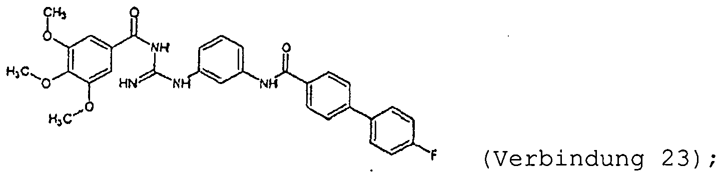
- N-(2-Methyl-5-(3-(3,4,5-trimethoxybenzoyl)guanidino)phenyl)biphenyl-4-carboxamid: 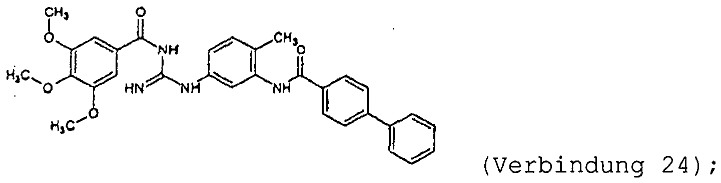
- N-(2-Methyl-5-(3-(3,4,5-trimethoxybenzoyl)guanidino)phenyl)biphenyl-3-carboxamid: 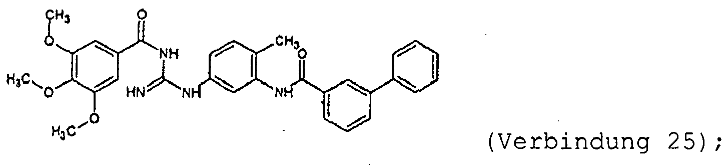
- N-(2-Chlor-5-(3-(3,4,5-trimethoxybenzoyl)guanidino)phenyl)biphenyl-3-carboxamid: 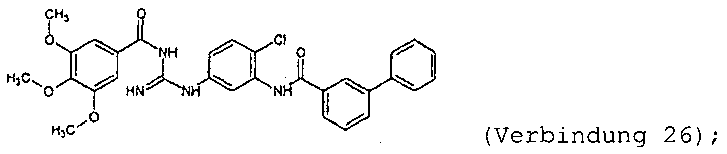
- N-N-(3-Benzamido-4-methylphenyl)carbamimidoyl)-3,4,5-trimethoxybenzamid: 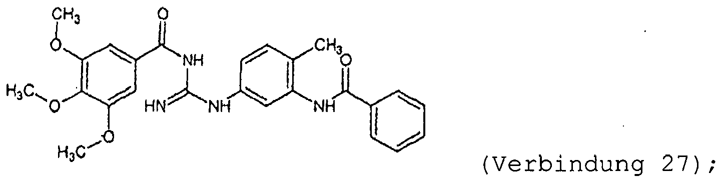
- N-N-(3-(Imidazo[2,1-b]thiazol-6-yl)phenyl)carbamimidoyl)-3,4,5-trimethoxybenzamid: 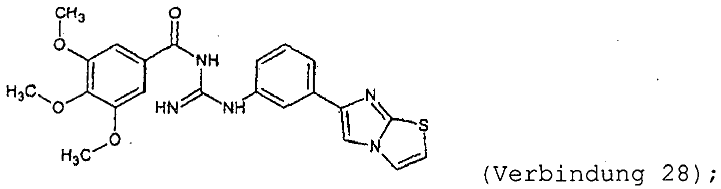
- N-(N-(3-(1H-Indol-1-yl)phenyl)carbamimidoyl)-3,4,5-trimethoxybenzamid: 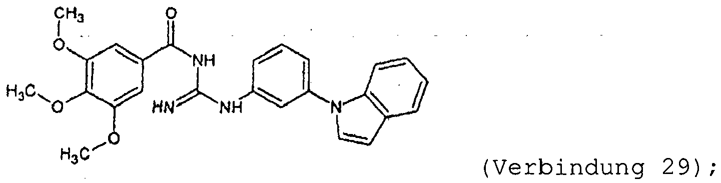
- 4'-Fluor-N-(2-methyl-5-(3-(3,4,5-trimethoxybenzoyl)guanidino)phenyl)biphenyl-4-carboxamid: 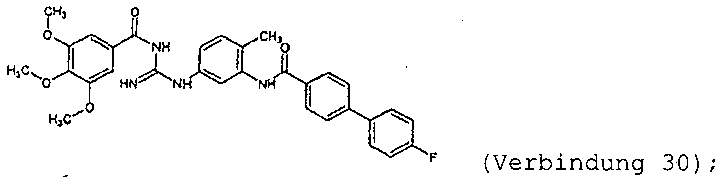
- 3,4,5-Trimethoxy-N-(N-(4-methyl-3-(4-(pyridin-4-yl)benzamido)phenyl)carbamimidoyl)benzamid: 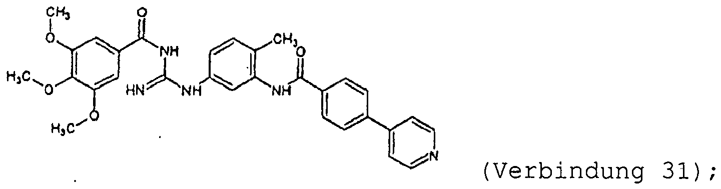
- N-(N-(4-Chlor-3-(4-phenoxybenzamido)phenyl)carbamimidoyl)-3,4,5-trimethoxybenzamid: 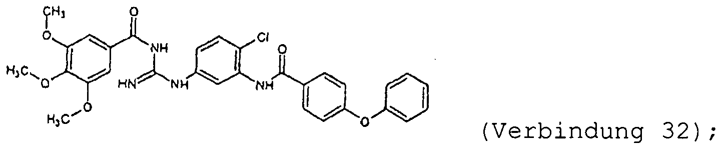
- N-(3-(3-Benzoylguanidino)phenyl)biphenyl-4-carboxamid: 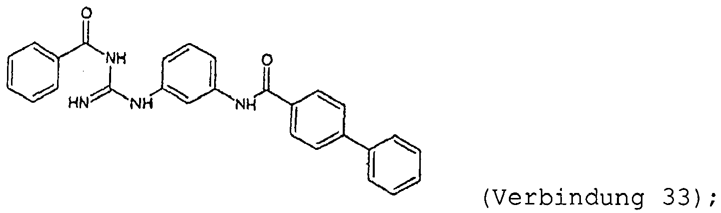
- N-(2-Methyl-3-(3-(3,4,5-trimethoxybenzoyl)guanidino)phenyl)biphenyl-4-carboxamid: 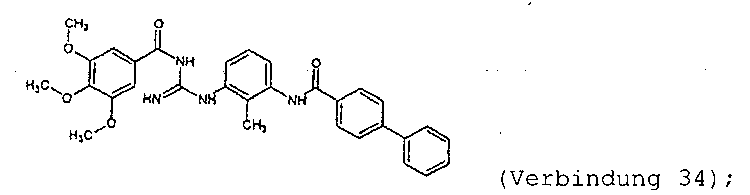
- N-(4-Methyl-3-(3-(3,4,5-trimethoxybenzoyl)guanidino)phenyl)biphenyl-4-carboxamid: 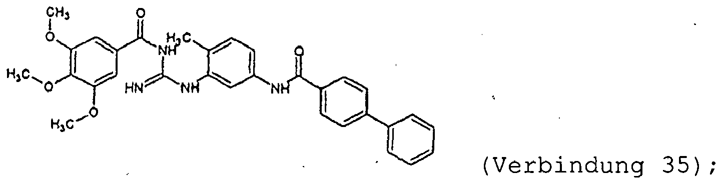
- N-(N-(3-Benzamidophenyl)carbamimidoyl)benzamid: 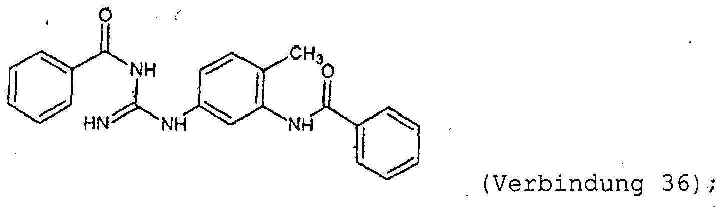
- N-(N-(3-(3-Biphenyl-4-ylureido)phenyl)carbamimidoyl)-3,4,5-trimethoxybenzamid: 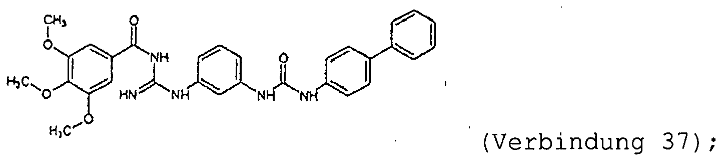
- N-(N-(4-Chlor-3-(3-phenylureido)phenyl)carbamimidoyl)-3,4,5-trimethoxybenzamid:

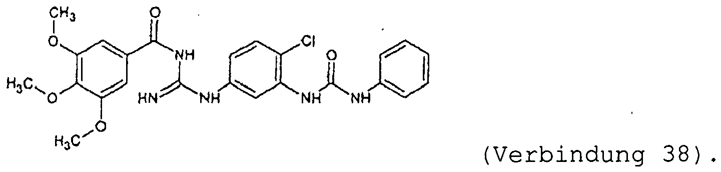

19. Verbindungen gemäß Anspruch 18 , **dadurch gekennzeichnet, dass** sie ausgewählt sind aus:
- 3,4,5-Trimethoxy-N-(N-(3-(4-methoxybenzamido)phenyl)carbamimidoyl)benzamid (Verbindung 1);
- N-(N-(3-(2-Chlorbenzamido)phenyl)carbamimidoyl)-3,4,5-trimethoxybenzamid (Verbindung 2);
- 3,4,5-Trimethoxy-N-(N-(3-(3-methoxybenzamido)phenyl)carbamimidoyl)benzamid (Verbindung 3);
- N-(3-(3-(3,4,5-Trimethoxybenzoyl)guanidino)phenyl)-2-naphthamid (Verbindung 6);
- N-(3-(3-(3,4,5-Trimethoxybenzoyl)guanidino)phenyl)furan-2-carboxamid (Verbindung 8);
- N-(N-(3-Benzamidophenyl)carbamimidoyl)-3,5-dimethoxybenzamid (Verbindung 9);
- N-(N-(3-Benzamidophenyl)carbamimidoyl)-7-methoxybenzo[d][1,3]dioxol-5-carboxamid (Verbindung 12);
- N-(N-(3-Benzamidophenyl)carbamimidoyl)-2,4-dimethoxybenzamid (Verbindung 13);
- N-(N-(3-Benzamidophenyl)carbamimidoyl)-3,4,5-trimethoxybenzamid (Verbindung 15);
- N-(2-Chlor-5-(3-(3,4,5-trimethoxybenzoyl)guanidino)phenyl)biphenyl-4-carboxamid (Verbindung 17);
- N-(N-(3-(1H-Indol-2-yl)phenyl)carbamimidoyl)-3,4,5-trimethoxybenzamid (Verbindung 19);
- N-(N-(4-Chlor-3-(phenylcarbamoyl)phenyl)carbamimidoyl)-3,4,5-trimethoxybenzamid (Verbindung 21);
- 4'-Fluor-N-(3-(3-(3,4,5-trimethoxybenzoyl)guanidino)phenyl)biphenyl-4-carboxamid (Verbindung 23);
- N-(2-Methyl-5-(3-(3,4,5-trimethoxybenzoyl)guanidino)phenyl)biphenyl-4-carboxamid (Verbindung 24);
- N-(N-(3-(1H-Indol-1-yl)phenyl)carbamimidoyl)-3,4,5-trimethoxybenzamid (Verbindung 29);
- 4'-Fluor-N-(2-methyl-5-(3-(3,4,5-trimethoxybenzoyl)guanidino)phenyl)biphenyl-4-carboxamid (Verbindung 30);
- N-(N-(4-Chlor-3-(4-phenoxybenzamido)phenyl)carbamimidoyl)-3,4,5-trimethoxybenzamid (Verbindung 32);
- N-(N-(4-Chlor-3-(3-phenylureido)phenyl)carbamimidoyl)-3,4,5-trimethoxybenzamid (Verbindung 38).

20. Verbindungen der Formel (I) gemäß einem der Ansprüche 16 bis 19, **dadurch gekennzeichnet , dass** sie in Form von Salzen, vorzugsweise von Salzen, die mit Chlorwasserstoffsäure, Bromwasserstoffsäure, Salpetersäure, Schwefelsäure, Phosphorsäure, Essigsäure, Ameisensäure, Propionsäure, Benzoesäure, Maleinsäure, Fumarsäure, Bernsteinsäure, Weinsäure, Citronensäure, Oxalsäure, Glyoxylsäure, Asparaginsäure, Alkansulfonsäuren, wie Methansulfonsäure und Ethansulfonsäure, Arylsulfonsäuren, wie Benzolsulfonsäure und para-Toluolsulfonsäure, oder Arylcarbonsäuren gebildet sind, und bevorzugter Salzen, die mit Chlorwasserstoffsäure gebildet sind, vorliegen.

## Claims

1. Compounds **characterised in that** they have the following general formula (I): 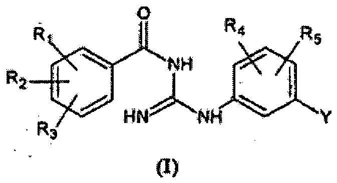 in which:
- R₁, R₂ and R₃, identical or different, and independently of one another, are a hydrogen or halogen atom, a hydroxyl radical, an alkyl, perfluoroalkyl, alkoxy possibly substituted, alkylthio or nitrile group, or a heterocycle obtained from two of R₁, R₂ and R₃ combined with two adjacent carbon atoms of the phenyl ring, to which they are bonded;
- Y is a mono- or polycyclic heteroaryl group, -NH-(C=O)-R_{6,} -(C=O)-NH-R₆ or -NH-(C=O)-NH-R₆ in which R₆ is an unsubstituted mono- or polycyclic aryl group; an aryl group comprising one or more substituents selected from a halogen atom, an alkyl, alkoxy or alkoxyaryl, mono- or dialkylamino radical, an aryl or heteroaryl group, a heterocycle; a mono- or polycyclic heteroaryl group; a straight-chain or branching alkyl radical; or a saturated or unsaturated, mono- or polycyclic hydrocarbonated group;
- R₄ and R₅, identical or different, and independently of one another, are a hydrogen or halogen atom, or an alkoxy, alkylthio, alkyl, perfluoroalkyl, nitrile or nitro group,
as medicaments.

2. Compounds having the general formula (I), according to claim 1, **characterised in that** they are selected from those in which:
- R₁, R₂ and R₃, identical or different, are a hydrogen atom, a methyloxy or ethyloxy radical, or a dioxolane combined with two adjacent carbon atoms of the phenyl ring, to which they are bonded;
- R₄ and R₅, are identical or different, are a hydrogen, chlorine, bromine, fluorine atom, or a methyl or methoxy radical, and
- Y is:
- a mono- or polycyclic heteroaryl group, preferably selected from indole or imidazole combined with a thiazole group, or
- a group -NH-(C=O)-R₆, -(C=O)-NH-R₆ or -NH-(C=O)-NH-R₆ in which R₆ is a phenyl group which is possibly substituted by a halogen atom, an alkyl, dialkylamino or alkoxy radical, a cycloalkyl group, or an aryl group; a cycloalkyl group; a pyridinyl group; a naphthyl group; a furyl group; a thiophenyl group; or an isopropyl radical, and in which R₆ is preferably a phenyl group which is possibly substituted by a chlorine atom, a methoxy radical, a morpholine, or a phenyl or phenoxy group; a cyclohexyl group; a pyridinyl group; a naphthyl group; or a furyl group.

3. Compounds having the general formula (I), according to claim 1 or claim 2, **characterised by** the fact that they are selected from:
- 3,4,5-trimethoxy-N-(N-(3-(4-methoxybenzamido)phenyl)-carbamimidoyl)benzamide: 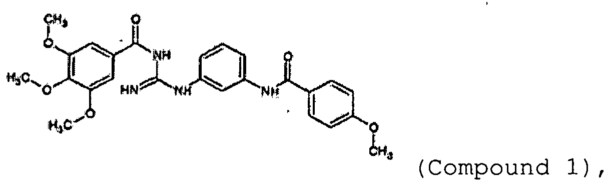
- N-(N-(3-(2-chlorobenzamido)phenyl)carbamimidoyl)-3,4,5-trimethoxybenzamide: 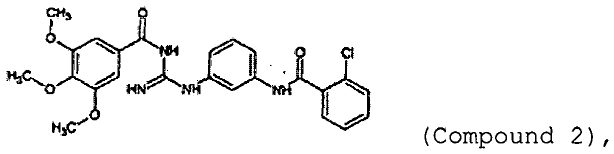
- 3,4,5-trimethoxy-N-(N-(3-(3-methoxybenzamido)phenyl)-carbamimidoyl)benzamide: 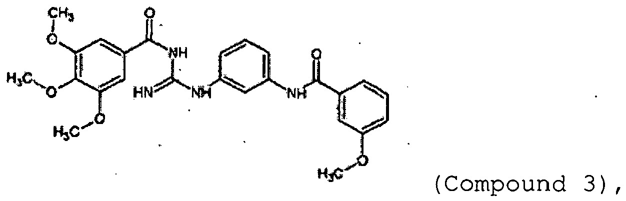
- N-(3-(3-(3,4,5-trimethoxybenzoyl)guanidino)phenyl)-biphenyl-4-carboxamide: 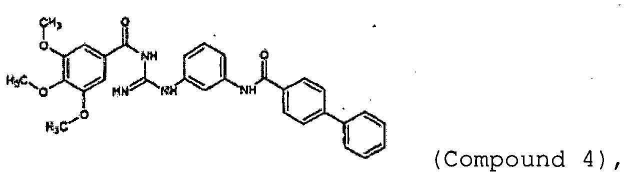
- N-(N-(3-(cyclohexanecarboxamido)phenyl)carbamimidoyl)-3,4,5-trimethoxybenzamide: 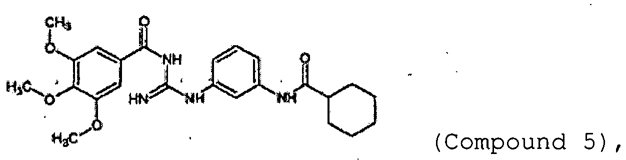
- N-(3-(3-(3,4,5-trimethoxybenzoyl)guanidino)phenyl)-2-naphthamide: 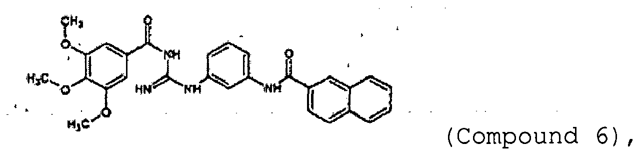
- N-(3-(3-(3,4,5-trimethoxybenzoyl)guanidino)phenyl)-isonicotinamide: 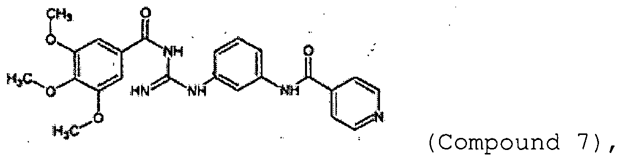
- N-(3-(3-(3,4,5-trimethoxybenzoyl)guanidino)phenyl)furan-2-carboxamide: 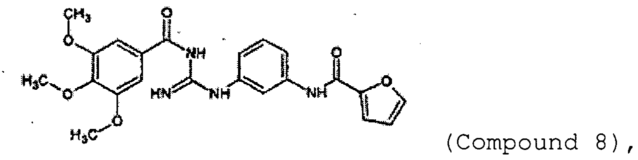
- N-(N-(3-benzamidophenyl)carbamimidoyl)-3,5-dimethoxy-benzamide: 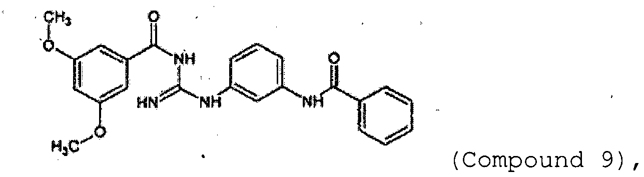
- N-(N-(3-benzamidophenyl)carbamimidoyl)-4-ethoxy-3,5-dimethoxybenzamide: 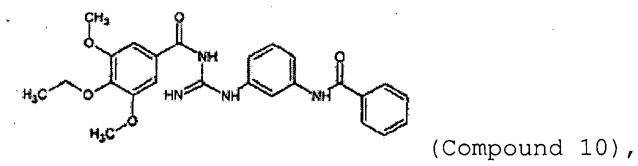
- N-(N-(3-benzamidophenyl)carbamimidoyl)-3,4-diethoxy-5-methoxybenzamide: 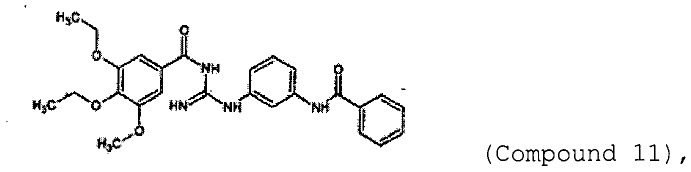
- N-(N-(3-benzamidophenyl)carbamimidoyl)-7-methoxybenzo[d][1,3]dioxole-5-carboxamide: 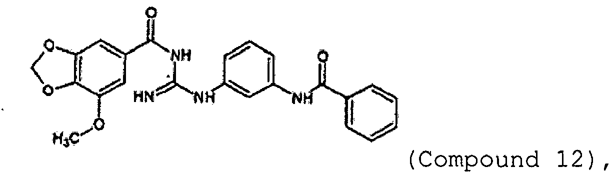
- N-(N-(3-benzamidophenyl)carbamimidoyl)-2,4-dimethoxy-benzamide: 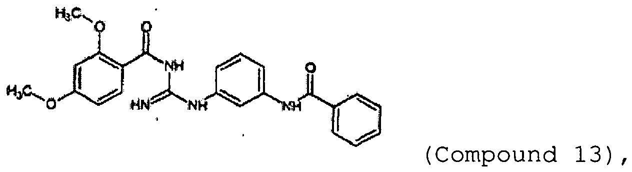
- N-(N-(3-benzamido-4-fluorophenyl)carbamimidoyl)-3,4,5-trimethoxybenzamide: 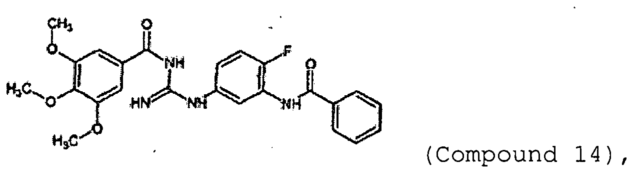
- N-(N-(3-benzamidophenyl)carbamimidoyl)-3,4,5-trimethoxy-benzamide: 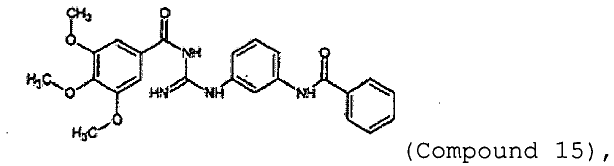
- N-(N-(3-benzamido-4-chlorophenyl)carbamimidoyl)-3,4,5-trimethoxybenzamide: 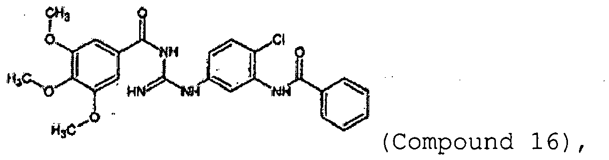
- N-(2-chloro-5-(3-(3,4,5-trimethoxybenzoyl)guanidino)-phenyl)biphenyl-4-carboxamide: 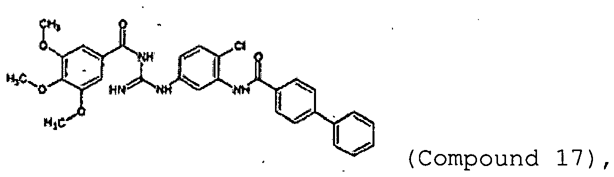
- 3,4,5-trimethoxy-N-(N-(3-(4-morpholinobenzamido)-phenyl)carbamimidoyl)benzamide: 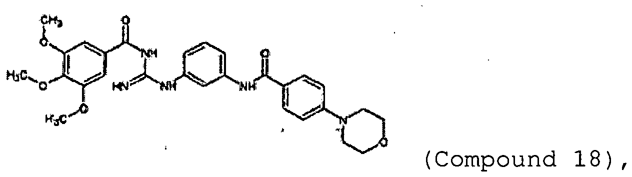
- N-(N-(3-(1H-indol-2-yl)phenyl)carbamimidoyl)-3,4,5-trimethoxybenzamide: 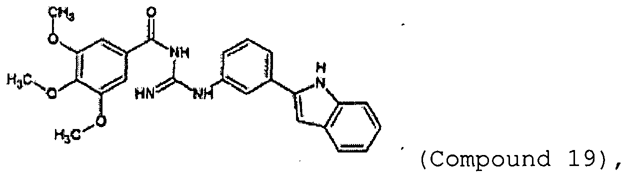
- N-(N-(4-chloro-3-(4-methoxyphenylcarbamoyl)-phenyl)carbamimidoyl)-3,4,5-trimethoxybenzamide: 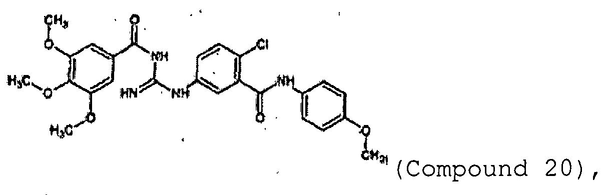
- N-(N-(4-chloro-3-(phenylcarbamoyl)phenyl)-carbamimidoyl)-3,4,5-trimethoxybenzamide: 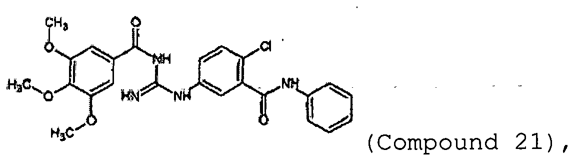
- 3,4,5-trimethoxy-N-(N-(4-methyl-3-(phenylcarbamoyl)-phenyl)carbamimidoyl)benzamide: 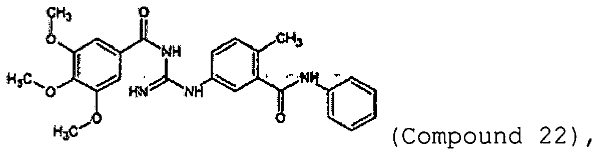
- 4'-fluoro-N-(3-(3-(3,4,5-trimethoxybenzoyl)guanidino)-phenyl)biphenyl-4-carboxamide: 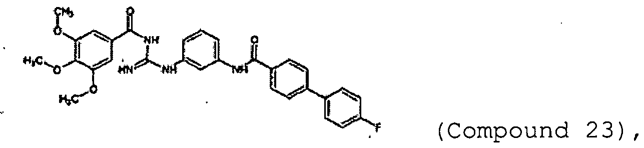
- N-(2-methyl-5-(3-(3,4,5-trimethoxybenzoyl)guanidino)-phenyl)biphenyl-4-carboxamide: 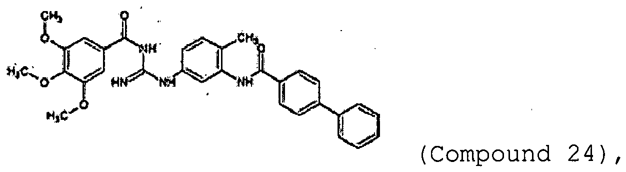
- N-(2-methyl-5-(3-(3,4,5-trimethoxybenzoyl)guanidine)-phenyl)biphenyl-3-carboxamide: 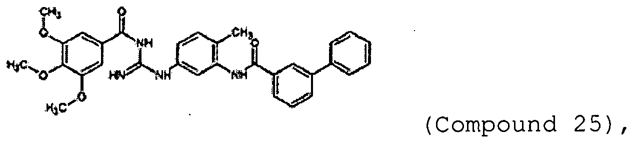
- N-(2-chloro-5-(3-(3,4,5-trimethoxybenzoyl)guanidino)-phenyl)biphenyl-3-carboxamide: 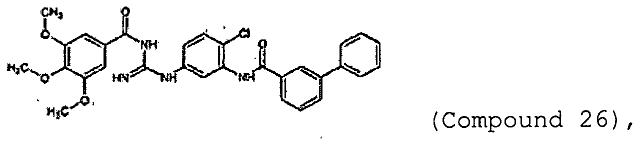
- N-(N-(3-benzamido-4-methylphenyl)carbamimidoyl)-3,4,5-trimethoxybenzamide: 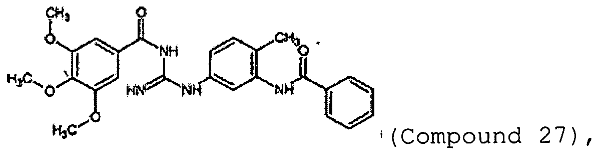
- N-(N-(3-(imidazo[2,1-b]thiazol-6-yl)phenyl)-carbamimidoyl)-3,4,5-trimethoxybenzamide: 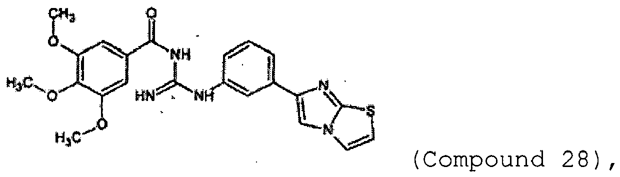
- N-(N-(3-(1H-indol-1-yl)phenyl)carbamimidoyl)-3,4,5-trimethoxybenzamide: 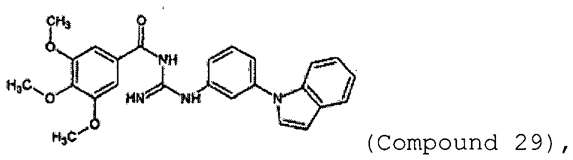
- 4'-fluoro-N-(2-methyl-5-(3-(3,4,5-trimethoxybenzoyl)-guanidino)phenyl)biphenyl-4-carboxamide: 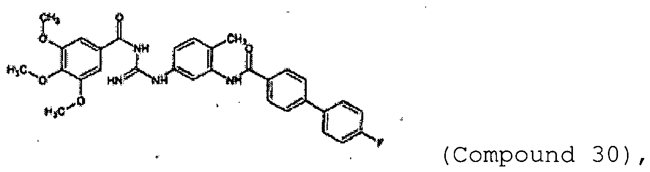
- 3,4,5-trimethoxy-N-(N-(4-methyl-3-(4-(pyridin-4-yl)benzamido)-phenyl)carbamimidoyl)benzamide: 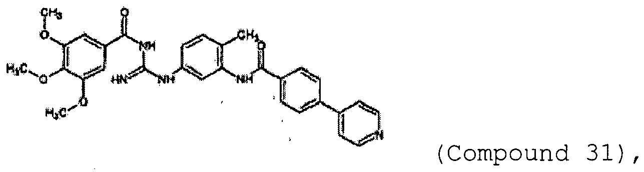
- N-(N-(4-chloro-3-(4-phenoxybenzamido)phenyl)-carbamimidoyl)-3,4,5-trimethoxybenzamide: 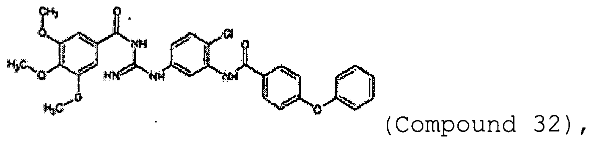
- N-(3-(3-benzoylguanidino)phenyl)biphenyl-4-carboxamide: 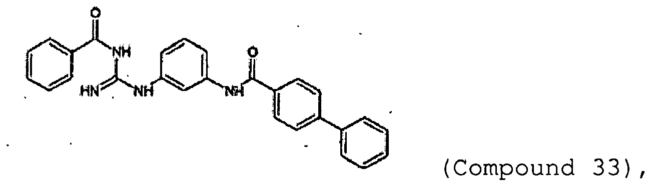
- N-(2-methyl-3-(3-(3,4,5-trimethoxybenzoyl)guanidino)-phenyl)biphenyl-4-carboxamide: 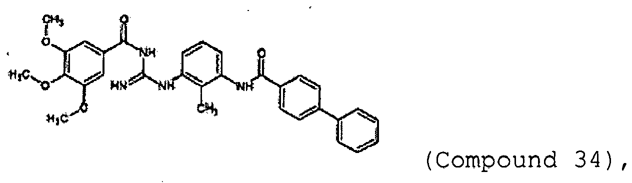
- N-(4-methyl-3-(3-(3,4,5-trimethoxybenzoyl)guanidino)-phenyl)biphenyl-4-carboxamide: 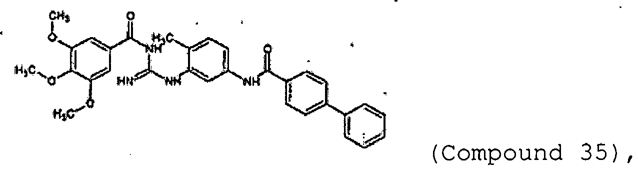
- N-(N-(3-benzamidophenyl)carbamimidoyl)benzamide: 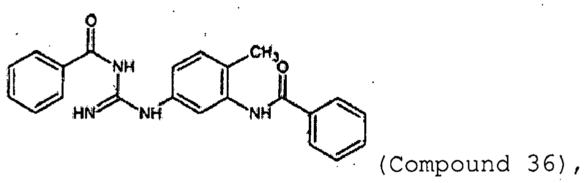
- N-(N-(3-(3-biphenyl-4-ylureido)phenyl)carbamimidoyl)-3,4,5-trimethoxybenzamide: 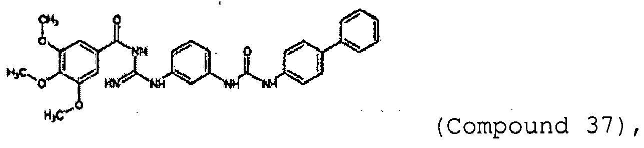
- N-(N-(4-chloro-3-(3-phenylureido)phenyl)carbamimidoyl)-3,4,5-trimethoxybenzamide:

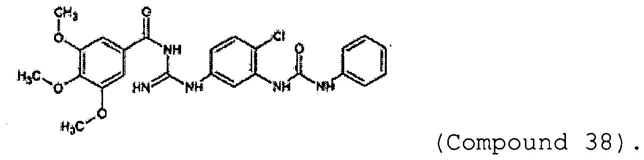

4. Compounds according to claim 3, **characterised by** the fact that they are selected from:
- 3,4,5-trimethoxy-N-(N-(3-(4-methoxybenzamido)phenyl)-carbamimidoyl)benzamide (compound 1),
- N-(N-(3-(2-chlorobenzamido)phenyl)carbamimidoyl)-3,4,5-trimethoxybenzamide (compound 2),
- 3,4,5-trimethoxy-N-(N-(3-(3-methoxybenzamido)phenyl)-carbamimidoyl)benzamide (compound 3),
- N-(3-(3-(3,4,5-trimethoxybenzoyl)guanidino)phenyl)-2-naphthamide (compound 6),
- N-(3-(3-(3,4,5-trimethoxybenzoyl)guanidino)phenyl)furan-2-carboxamide (compound 8),
- N-(N-(3-benzamidophenyl)carbamimidoyl)-3,5-dimethoxybenzamide (compound 9),
- N-(N-(3-benzamidophenyl)carbamimidoyl)-7-methoxybenzo[d][1,3]dioxole-5-carboxamide (compound 12),
- N-(N-(3-benzamidophenyl)carbamimidoyl)-2,4-dimethoxybenzamide (compound 13),
- N-(N-(3-benzamidophenyl)carbamimidoyl)-3,4,5-trimethoxybenzamide (compound 15),
- N-(2-chloro-5-(3-(3,4,5-trimethoxybenzoyl)guanidino)-phenyl)biphenyl-4-carboxamide (compound 17),
- N-(N-(3-(1H-indol-2-yl)phenyl)carbamimidoyl)-3,4,5-trimethoxybenzamide (compound 19),
- N-(N-(4-chloro-3-(phenylcarbamoyl)phenyl)-carbamimidoyl)-3,4,5-trimethoxybenzamide (compound 21),
- 4'-fluoro-N-(3-(3-(3,4,5-trimethoxybenzoyl)guanidino)-phenyl)biphenyl-4-carboxamide (compound 23),
- N-(2-methyl-5-(3-(3,4,5-trimethoxybenzoyl)guanidino)-phenyl)biphenyl-4-carboxamide (compound 24),
- N-(N-(3-(1H-indol-1-yl)phenyl)carbamimidoyl)-3,4,5-trimethoxybenzamide (compound 29),
- 4'-fluoro-N-(2-methyl-5-(3-(3,4,5-trimethoxybenzoyl)-guanidino)phenyl)biphenyl-4-carboxamide (compound 30),
- N-(N-(4-chloro-3-(4-phenoxybenzamido)phenyl)-carbamimidoyl)-3,4,5-trimethoxybenzamide (compound 32),
- N-(N-(4-chloro-3-(3-phenylureido)phenyl)carbamimidoyl)-3,4,5-trimethoxybenzamide (compound 38).

5. Compounds having the general formula (I), according to one of claims 1 to 4, **characterised in that** they are in the form of salts, and preferably salts formed with hydrochloric, hydrobromic, nitric, sulphuric, phosphoric, acetic, formic, propionic, benzoic, maleic, fumaric, succinic, tartric, citric, oxalic, glyoxalic, and aspartic acids, alkane sulphonic acids such as methane sulphonic acid and ethane sulphonic acid, aryl sulphonic acids such as benzene sulphonic acid and paratoluene sulphonic acid, or aryl carboxylic acids, and more preferably, salts formed with hydrochloric acid.

6. Compounds having the general formula (I), according to claims 1 to 5, for use as medicaments intended for treating tumours associated with hyperactivation of the signalling pathway of Hedgehog proteins.

7. Compounds having the general formula (I), according to claim 6, for use as medicaments for treating tumours of nerve tissue (medulloblastomas, primitive neurodectermal tumours, glioblastomas, meningiomas and oligodendrogliomas), cutaneous tumours (basal cell carcinomas, trichoepitheliomas), tumours of muscle and bone tissue (rhabdomyosarcomas, osteosarcomas) and tumours of other tissues (kidney, bladder, prostate, lung, stomach, pancreas, breast, liver).

8. Compounds having the general formula (I), according to one of claims 1 to 5, for use as medicaments intended for treating pathologies of the neurodegenerative type.

9. Compounds having the general formula (I), according to claim 8, for use as medicaments for treating Parkinson's disease, Huntington's chorea, Alzheimer's disease, multiple sclerosis and motor neuron disease.

10. Compounds having the general formula (I), according to one of claims 1 to 5, for use as medicaments intended for treating diseases related to cerebral development (holoprosencephaly), for treating strokes and cardiovascular events, and for treating diseases of the oligodendrocyte and the Schwann cells.

11. Compounds having the general formula (I), according to claim 10, for in vitro use for monitoring and modulating the renewal of human or animal stem cells.

12. Compounds having the general formula (I), according to one of claims 1 to 5, for use as medicaments intended for treating diabetes.

13. Compounds having the general formula (I), according to one of claims 1 to 5, as labels for detecting the presence of proteins such as *Smoothened, Patched* (*Patched I* and *Patched 2*), *Dispatched* (*Dispatched 1* and *Dispatched 2*) and HIP proteins in tissues or cell lines.

14. Compounds having the general formula (I), according to one of claims 1 to 5, as diagnostic tools for screening proteins such as *Smoothened, Patched* (*Patched I* and *Patched 2*), *Dispatched* (*Dispatched 1* and *Dispatched 2*) and HIP proteins in tissues or cell lines.

15. Pharmaceutical composition, **characterised by** the fact that it comprises, as an active principle, at least one compound having the general formula (I), as defined in any one of claims 1 to 5, and at least one pharmaceutically acceptable excipient.

16. Compounds **characterised in that** they have the following general formula (I): 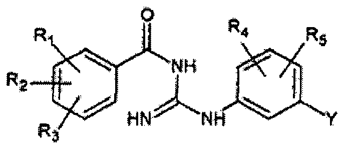 in which:
- R₁, R₂ and R₃, identical or different, and independently of one another, are a hydrogen or halogen atom, a hydroxyl radical, an alkyl, perfluoroalkyl, alkoxy possibly substituted, alkylthio or nitrile group, or a heterocycle obtained from two of R₁, R₂ and R₃ combined with two adjacent carbon atoms of the phenyl ring, to which they are bonded;
- Y is a mono- or polycyclic heteroaryl group selected from indole or imidazole combined with a thiazole group, or Y is a group -NH-(C=O)-R₆, -(C=O)-NH-R₆ or -NH-(C=O)-NH-R₆ in which R₆ is a unsubstituted mono- or polycyclic aryl group; an aryl group comprising one or more substituents selected from a halogen atom, an alkyl, alkoxy or alkoxyaryl, or mono- or dialkylamino radical, an aryl or heteroaryl group, or a heterocycle; a mono- or polycyclic heteroaryl group; a straight-chain or branching alkyl radical; or a saturated or unsaturated, mono- or polycyclic hydrocarbonated group;
- R₄ and R₅, identical or different, and independently of one another, are a hydrogen or halogen atom, or an alkoxy, alkylthio, alkyl, perfluoroalkyl, nitrile or nitro group.

17. Compounds having the general formula (I), according to claim 16, **characterised in that** they are selected from those in which:
- R₁, R₂ and R₃, identical or different, are a hydrogen or halogen atom, a methyloxy or ethyloxy radical, or a dioxolane combined with two adjacent carbon atoms of the phenyl ring, to which they are bonded;
- R₄ and R₅, identical or different, are a hydrogen, chlorine, bromine, or fluorine atom, or a methyl or methoxy radical, and
- Y is:
- a mono- or polycyclic heteroaryl group selected from indole or imidazole combined with a thiazole group, or
- Y is a group -NH-(C=O)-R₆, -(C=O)-NH-R₆ or -NH-(C=O)-NH-R₆ in which R₆ is a phenyl group possibly substituted by a halogen atom, an alkyl, diaminoalkyl or alkoxy radical, a cycloalkyl group or an aryl group; a cycloalkyl group; a pyridinyl group; a naphthyl group; a furyl group; a thiophenyl group; an isopropyl radical, and R₆ is preferably a phenyl group possibly substituted by a chlorine atom, a methoxy radical, a morpholine, or a phenyl or phenoxy group; a cyclohexyl group; a pyridinyl group; a naphthyl group; a furyl group; when the said group Y is a group -NH-(C=O)-R₆, -(C=O)-NH-R₆ or -NH-(C=O)-NH-R₆.

18. Compounds having the general formula (I), according to claim 16 or claim 17, **characterised by** the fact that they are selected from:
- 3,4,5-trimethoxy-N-(N-(3-(4-methoxybenzamido)phenyl)carbamimidoyl)benzamide: 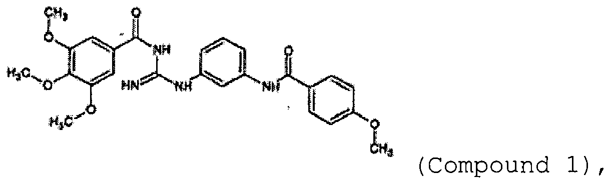
- N-(N-(3-(2-chlorobenzamido)phenyl)carbamimidoyl)-3,4,5-trimethoxybenzamide: 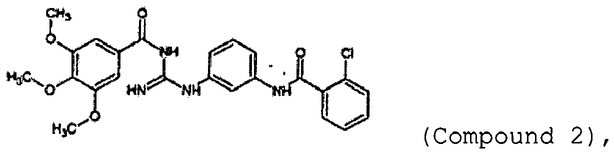
- 3,4,5-trimethoxy-N-(N-(3-(3-methoxybenzamido)phenyl)-carbamimidoyl)benzamide: 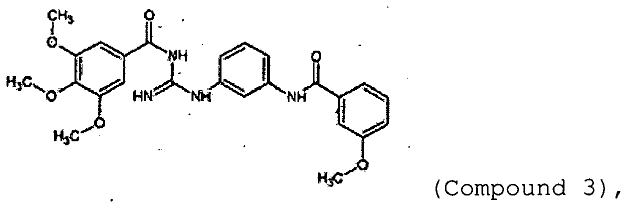
- N-(3-(3-(3,4,5-trimethoxybenzoyl)guanidino)phenyl)-biphenyl-4-carboxamide: 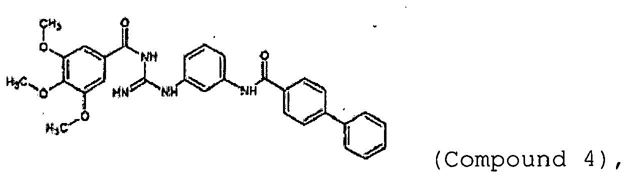
- N-(N-(3-(cyclohexanecarboxamido)phenyl)carbamimidoyl)-3,4,5-trimethoxybenzamide: 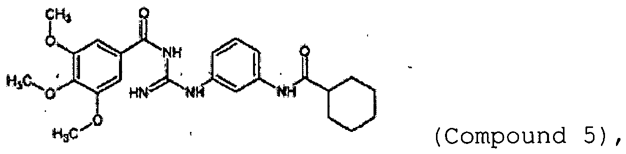
- N-(3-(3-(3,4,5-trimethoxybenzoyl)guanidino)phenyl)-2-naphthamide: 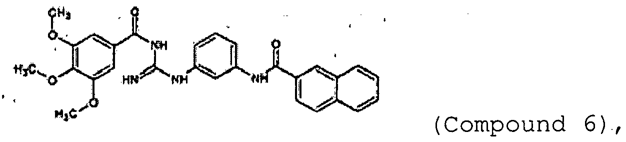
- N-(3-(3-(3,4,5-trimethoxybenzoyl)guanidino)phenyl)-isonicotinamide: 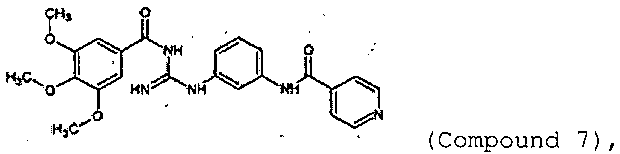
- N-(3-(3-(3,4,5-trimethoxybenzoyl)guanidino)phenyl)-furan-2-carboxamide: 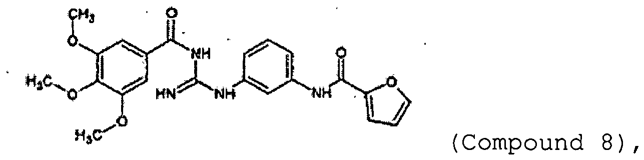
- N-(N-(3-benzamidophenyl)carbamimidoyl)-3,5-dimethoxy-benzamide: 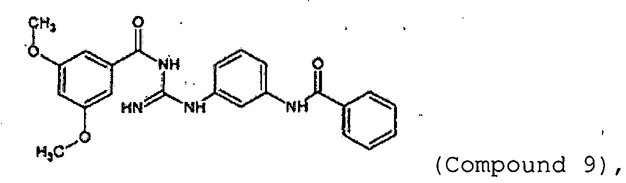
- N-(N-(3-benzamidophenyl)carbamimidoyl)-4-ethoxy-3,5-dimethoxybenzamide: 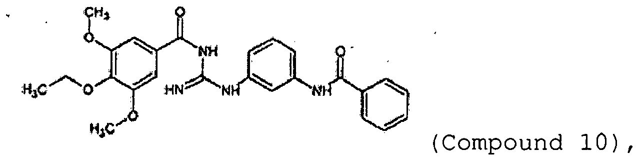
- N-(N-(3-benzamidophenyl)carbamimidoyl)-3,4-diethoxy-5-methoxybenzamide: 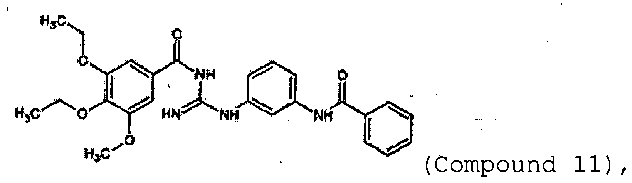
- N-(N-(3-benzamidophenyl)carbamimidoyl)-7-methoxybenzo[d][1,3]dioxole-5-carboxamide: 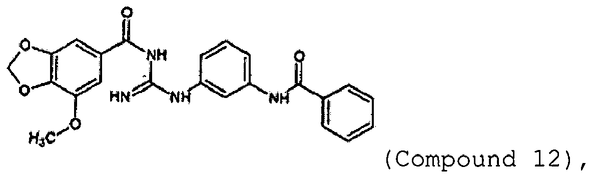
- N-(N-(3-benzamidophenyl)carbamimidoyl)-2,4-dimethoxy-benzamide: 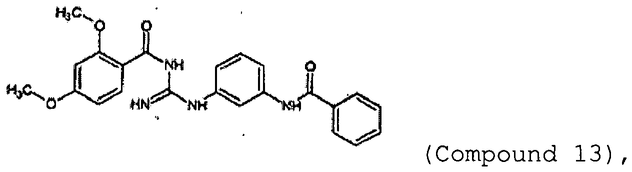
- N-(N-(3-benzamido-4-fluorophenyl)carbamimidoyl)-3,4,5-trimethoxybenzamide: 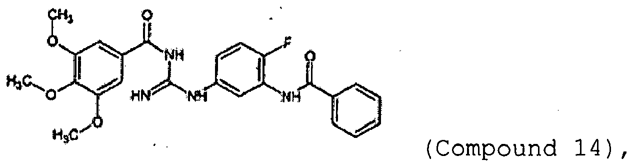
- N-(N-(3-benzamidophenyl)carbamimidoyl)-3,4,5-trimethoxy-benzamide: 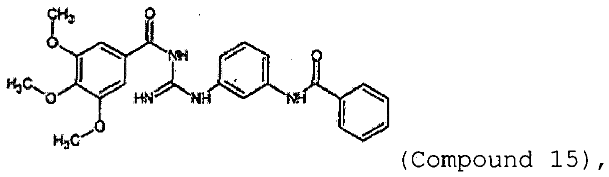
- N-(N-(3-benzamido-4-chlorophenyl)carbamimidoyl)-3,4,5-trimethoxybenzamide: 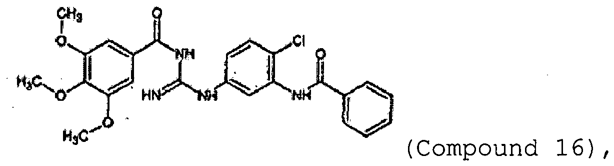
- N-(2-chloro-5-(3-(3,4,5-trimethoxybenzoyl)guanidino)-phenyl)biphenyl-4-carboxamide: 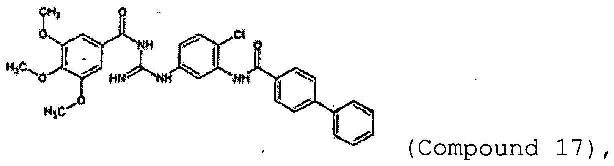
- 3,4,5-trimethoxy-N-(N-(3-(4-morpholinobenzamido)-phenyl)carbamimidoyl)benzamide: 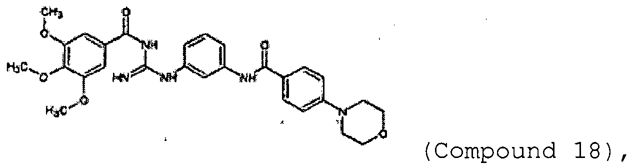
- N-(N-(3-(1H-indol-2-yl)phenyl)carbamimidoyl)-3,4,5-trimethoxybenzamide: 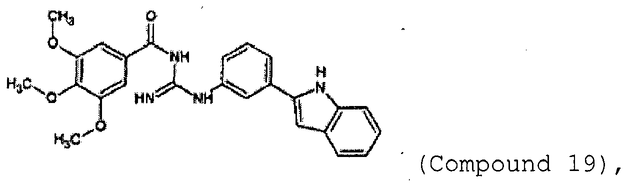
- N-(N-(4-chloro-3-(4-methoxyphenylcarbamoyl)-phenyl)carbamimidoyl)-3,4,5-trimethoxybenzamide: 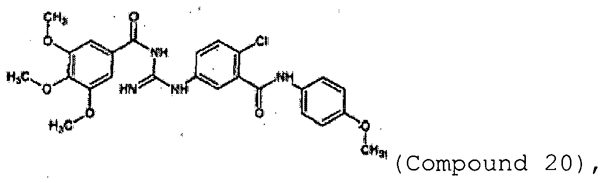
- N-(N-(4-chloro-3-(phenylcarbamoyl)phenyl)-carbamimidoyl)-3,4,5-trimethoxybenzamide: 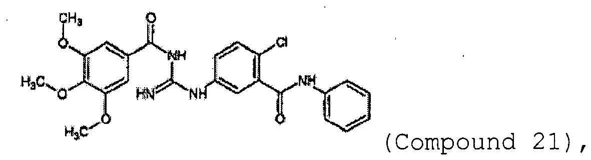
- 3,4,5-trimethoxy-N-(N-(4-methyl-3-(phenylcarbamoyl)-phenyl)carbamimidoyl)benzamide: 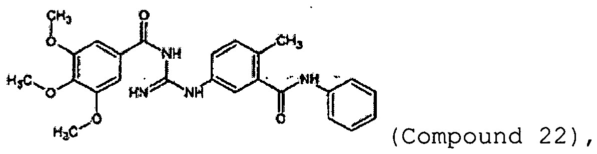
- 4'-fluoro-N-(3-(3-(3,4,5-trimethoxybenzoyl)guanidino)-phenyl)biphenyl-4-carboxamide: 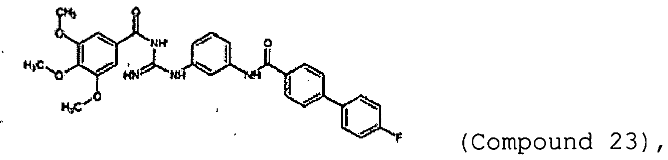
- N-(2-methyl-5-(3-(3,4,5-trimethoxybenzoyl)guanidino)-phenyl)biphenyl-4-carboxamide: 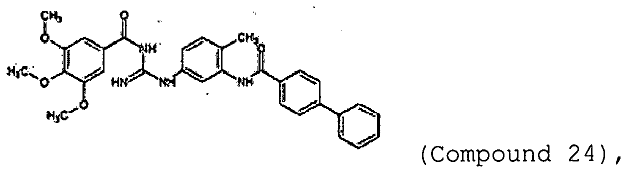
- N-(2-methyl-5-(3-(3,4,5-trimethoxybenzoyl)guanidino)-phenyl)biphenyl-3-carboxamide: 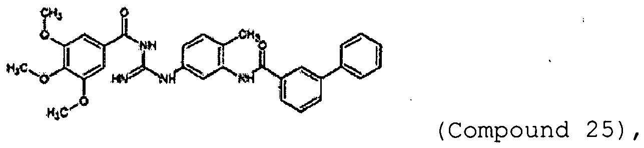
- N-(2-chloro-5-(3-(3,4,5-trimethoxybenzoyl)guanidino)-phenyl)biphenyl-3-carboxamide: 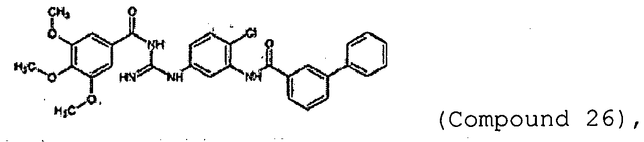
- N-(N-(3-benzamido-4-methylphenyl)carbamimidoyl)-3,4,5-trimethoxybenzamide: 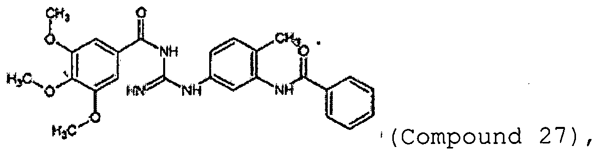
- N-N-(3-(imidazo[2,1-b]thiazol-6-yl)phenyl)-carbamimidoyl)-3,4,5-trimethoxybenzamide: 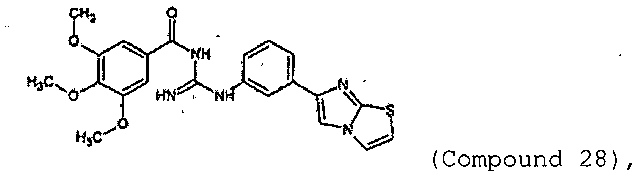
- N-(N-(3-(1*H*-indol-1-yl)phenyl)carbamimidoyl)-3,4,5-trimethoxybenzamide: 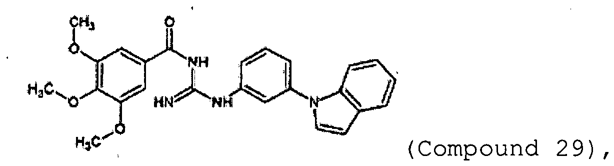
- 4'-fluoro-N-(2-methyl-5-(3-(3,4,5-trimethoxybenzoyl)-guanidino)phenyl)biphenyl-4-carboxamide: 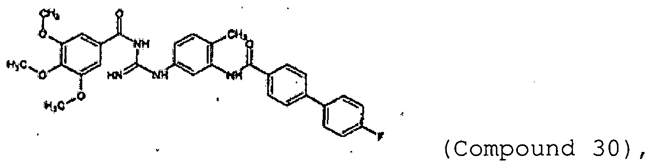
- 3,4,5-trimethoxy-N-(N-(4-methyl-3-(4-(pyridin-4-yl)-benzamido)phenyl)carbamimidoyl)benzamide: 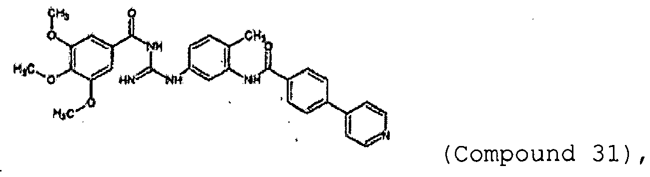
- N-(N-(4-chloro-3-(4-phenoxybenzamido)phenyl)-carbamimidoyl)-3,4,5-trimethoxybenzamide: 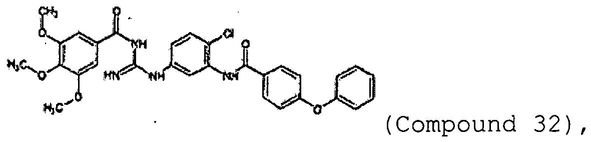
- N-(3-(3-benzoylguanidino)phenyl)biphenyl-4-carboxamide: 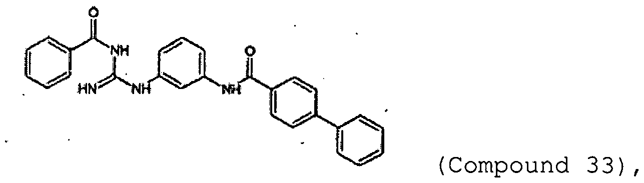
- N-(2-methyl-3-(3-(3,4,5-trimethoxybenzoyl)guanidino)-phenyl)biphenyl-4-carboxamide: 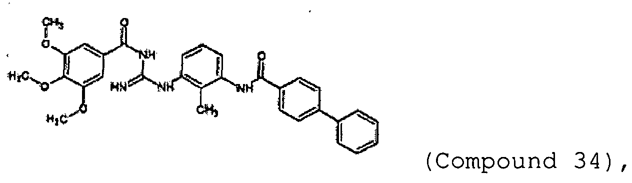
- N-(4-methyl-3-(3-(3,4,5-trimethoxybenzoyl)guanidino)-phenyl)biphenyl-4-carboxamide: 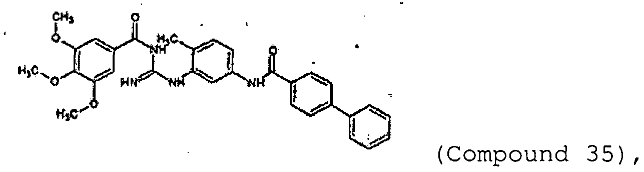
- N-(N-(3-benzamidophenyl)carbamimidoyl)benzamide: 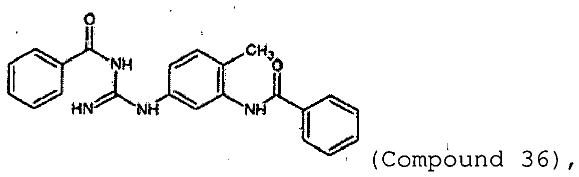
- N-(N-(3-(3-biphenyl-4-ylureido)phenyl)carbamimidoyl)-3,4,5-trimethoxybenzamide: 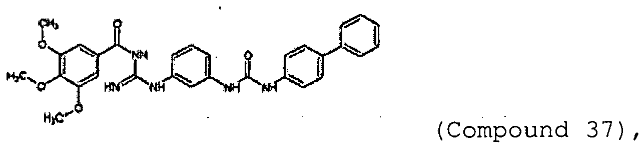
- N-(N-(4-chloro-3-(3-phenylureido)phenyl)carbamimidoyl)-3,4,5-trimethoxybenzamide:

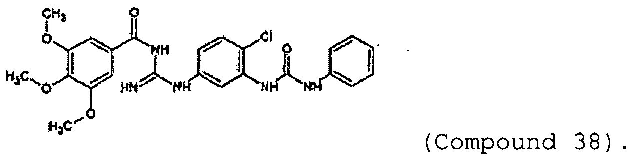

19. Compounds according to claim 18, **characterised by** the fact that they are selected from:
- 3,4,5-trimethoxy-N-(,N-(3-(4-methoxybenzamido)phenyl)-carbamimidoyl)benzamide (compound 1),
- N-(N-(3-(2-chlorobenzamido)phenyl)carbamimidoyl)-3,4,5-methoxybenzamide (compound 2),
- 3,4,5-trimethoxy-N-(N-(3-(3-methoxybenzamido)phenyl)-carbamimidoyl)benzamide (compound 3),
- N-(3-(3-(3,4,5-trimethoxybenzoyl)guanidino)phenyl)-2-naphthamide (compound 6),
- N-(3-(3-(3,4,5-trimethoxybenzoyl)guanidino)phenyl)-furan-2-carboxamide (compound 8),
- N-(N-(3-benzamidophenyl)carbamimidoyl)-3,5-dimethoxy-benzamide (compound 9),
- N-(N-(3-benzamidophenyl)carbamimidoyl)-7-methoxybenzo[d][1,3]dioxole-5-carboxamide (compound 12),
- N-(N-(3-benzamidophenyl)carbamimidoyl)-2,4-dimethoxy-benzamide (compound 13),
- N-(N-(3-benzamidophenyl)carbamimidoyl)-3,4,5-trimethoxybenzamide (compound 15),
- N-(2-chloro-5-(3-(3,4,5-trimethoxybenzoyl)guanidino)-phenyl)biphenyl-4-carboxamide (compound 17),
- N-(N-(3-(1*H*-indol-2-yl)phenyl)carbamimidoyl)-3,4,5-trimethoxybenzamide (compound 19),
- N-(N-(4-chloro-3-(phenylcarbamoyl)phenyl)-carbamimidoyl)-3,4,5-trimethoxybenzamide (compound 21),
- 4'-fluoro-N-(3-(3-(3,4,5-trimethoxybenzoyl)guanidino)-phenyl)biphenyl-4-carboxamide (compound 23),
- N-(2-methyl-5-(3-(3,4,5-trimethoxybenzoyl)guanidino)-phenyl)biphenyl-4-carboxamide (compound 24),
- N-(N-(3-(1*H*-indol-1-yl)phenyl)carbamimidoyl)-3,4,5-trimethoxybenzamide (compound 29),
- 4'-fluoro-N-(2-methyl-5-(3-(3,4,5-trimethoxybenzoyl)-guanidino)phenyl)biphenyl-4-carboxamide (compound 30),
- N-(N-(4-chloro-3-(4-phenoxybenzamido)phenyl)-carbamimidoyl)-3,4,5-trimethoxybenzamide (compound 32),
- N-(N-(4-chloro-3-(3-phenylureido)phenyl)carbamimidoyl)-3,4,5-trimethoxybenzamide (compound 38).

20. Compounds having the general formula (I), according to one of claims 16 to 19, **characterised in that** they are in the form of salts, and preferably salts formed with hydrochloric, hydrobromic, nitric, sulphuric, phosphoric, acetic, formic, propionic, benzoic, maleic, fumaric, succinic, tartric, citric, oxalic, glyoxalic, and aspartic acids, alkane sulphonic acids such as methane sulphonic acid and ethane sulphonic acid, aryl sulphonic acids such as benzene sulphonic acid and paratoluene sulphonic acid, or aryl carboxylic acids, and more preferably, salts formed with hydrochloric acid.
